(19) Europäisches Patentamt
European Patent Office
Office européen des brevets

(11) EP 0 933 351 A1

(12) **EUROPEAN PATENT APPLICATION**

(43) Date of publication:
**04.08.1999 Bulletin 1999/31**

(21) Application number: **99105682.1**

(22) Date of filing: **21.12.1995**

(51) Int. Cl.$^6$: **C07C 57/50**, C07D 311/92, C07C 205/56, C07C 69/618, C07C 233/54, C07D 471/04, C07C 251/84, A61K 31/19, A61K 31/215, A61K 31/35, G01N 33/53
// C07D223/14, A61K31/55, C07D221/08, A61K31/435, C07D209/60, A61K31/40, C07D221/10, C07D265/34, A61K31/535

(84) Designated Contracting States:
**AT BE CH DE ES FR GB IT LI NL**

(30) Priority: **30.12.1994 US 366630**
**07.06.1995 US 475397**
**07.06.1995 US 472127**
**07.06.1995 US 475514**

(62) Document number(s) of the earlier application(s) in accordance with Art. 76 EPC:
**95944221.1 / 0 800 504**

(71) Applicant:
**LIGAND PHARMACEUTICALS INCORPORATED**
**San Diego, California 92121 (US)**

(72) Inventors:
- **Hwang, Chan Kou**
  **Boulder, CO 80304 (US)**
- **White, Steven K.**
  **San Diego, CA 92129 (US)**
- **Bennani, Youssef L.**
  **La Jolla, CA 92037 (US)**
- **Canan Koch, Stacie S.**
  **San Diego, CA 92116 (US)**
- **Badea, Beth Ann**
  **San Diego, CA 92127 (US)**
- **Hebert, Jonathan J.**
  **Mission Viejo, CA 92692 (US)**
- **Farmer, Luc J.**
  **La Jolla, CA 92037 (US)**
- **Nadzan, Alex M.**
  **San Diego, CA 92130 (US)**

(74) Representative:
**Winter, Brandl & Partner**
**Patent- und Rechtsanwaltskanzlei**
**Alois-Steinecker-Strasse 22**
**85354 Freising (DE)**

Remarks:
This application was filed on 19 - 03 - 1999 as a divisional application to the application mentioned under INID code 62.

(54) **Tricyclic retinoids, methods for their production and use**

(57) Tricyclic retinoids having activity for retinoic acid receptors and/or retinoid X receptors are provided. Also provided are pharmaceutical compositions incorporating such tricyclic retinoid compounds and methods for their therapeutic use.



Printed by Xerox (UK) Business Services
2.16.7/3.6

## Description

### Field of the Invention

The present invention relates to retinoid compounds having activity for retinoic acid receptors and retinoid X receptors, and to methods for the production and therapeutic use of such compounds.

### Background of the Invention

The vitamin A metabolite, retinoic acid, has long been recognized to induce a broad spectrum of biological effects. In addition, a variety of structural analogues of retinoic acid have been synthesized that also have been found to be bioactive. Some, such as Retin-A® and Accutane®, have found utility as therapeutic agents for the treatment of various pathological conditions. In addition, synthetic retinoids have been found to mimic many of the pharmacological actions of retinoic acid.

Medical professionals have become very interested in the therapeutic applications of retinoids. Among their uses approved by the FDA is the treatment of severe forms of acne and psoriasis. A large body of evidence also exists that these compounds can be used to arrest and, to an extent, reverse the effects of skin damage arising from prolonged exposure to the sun. Other evidence exists that these compounds may be useful in the treatment and prevention of a variety of cancerous and pre-cancerous conditions, such as melanoma, cervical cancer, some forms of leukemia, oral leukoplakia and basal and squamous cell carcinomas. Retinoids have also shown an ability to be efficacious in treating and preventing diseases of the eye, cardiovascular system, immune system, skin, respiratory and digestive tracts, and as agents to facilitate wound healing and modulate programmed cell death (apoptosis).

Major insight into the molecular mechanism of retinoic acid signal transduction was gained in 1988, when a member of the steroid/thyroid hormone intracellular receptor superfamily was shown to transduce a retinoic acid signal. Evans, *Science*, 240:889-95 (1988); Giguere et al., *Nature*, 330:624-29 (1987); Petkovich *et al*., *Nature*, 330: 444-50 (1987). It is now known that retinoids regulate the activity of two distinct intracellular receptor subfamilies; the Retinoic Acid Receptors (RARs) and the Retinoid X Receptors (RXRs), including their isoforms, RARα, β, γ and RXRα, β, γ. In this regard, an endogenous low-molecular-weight ligand which modulates the transcriptional activity of the RARs is all-*trans*-retinoic acid (ATRA), while an endogenous ligand for the RXRs is 9-*cis* retinoic acid (9-*cis*). Heyman *et al*., *Cell*, 68:397-406 (1992) and Levin *et al. Nature*, 355:359-61 (1992).

Although both the RARs and RXRs respond to ATRA *in vivo,* due to the *in vivo* conversion of some of the ATRA to 9-*cis,* the receptors differ in several important aspects. First, the RARs and RXRs are significantly divergent in primary structure (e.g., the ligand binding domains of RARα and RXRα have only 27% amino acid identity). These structural differences are reflected in the different relative degrees of responsiveness of RARs and RXRs to various vitamin A metabolites and synthetic retinoids. In addition, distinctly different patterns of tissue distribution are seen for RARs and RXRs. For example, in contrast to the RARs, which are not expressed at high levels in the visceral tissues, RXRα mRNA has been shown to be most abundant in the liver, Kidney, lung, muscle and intestine. Finally, the RARs and RXRs have different target gene specificity. For example, response elements have recently been identified in the cellular retinal binding protein type II (CRBPII) and Apolipoprotein AI genes which confer responsiveness to RXR, but not RAR. Furthermore, RAR has also been recently shown to repress RXR-mediated activation through the CRBPII RXR response element (Manglesdorf *et al*., *Cell*, 66:555-61 (1991)). These data indicate that two retinoic acid responsive pathways are not simply redundant, but instead manifest a complex interplay.

In view of the related, but clearly distinct, nature of these receptors, retinoids which are more selective for the RAR subfamily or the RXR subfamily would be of great value for selectively controlling processes mediated by one or more of the RAR or RXR isoforms, and would provide the capacity for independent control of the physiologic processes mediated by the RARs or RXRs. In addition, pan-agonist retinoids that activate one or more isoforms of both the RARs and RXRs would also be valuable for controlling processes mediated by both of these subfamilies of retinoid receptors. Furthermore, retinoids which preferentially affect one or more but not all of the receptor isoforms also offer the possibility of increased therapeutic efficacy and reduced side effect profiles when used for therapeutic applications.

Various tricyclic compounds, the majority of which are beta-substituted on the C ring, have been disclosed to have retinoid activity, including those compounds disclosed in H. Kagechika et al., "Retinobenzoic Acids. 2. Structure-Activity Relationship of Chalcone-4-carboxylic Acids and Flavone-4'-carboxylic Acids", 32 *J. Med Chem*., 834 (1989); H. Kagechika et al. , "Retinobenzoic Acids. 3. Structure-Activity Relationships of Retinoidal Azobenzene-4-carboxylic Acids and Stilbene-4-carboxylic Acids", 32 *J. Med. Chem*., 1098 (1989); H. Kagechika et al., "Retinobenzoic Acids. 4. Conformation of Aromatic Amides with Retinoidal Activity. Importance of *trans*-Amide Structure for the Activity", 32 *J. Med Chem*., 2292 (1989); M.I. Dawson et al., "Effect of Structural Modifications in the C7-C11 Region of the Retinoid Skeleton on Biological Activity in a Series of Aromatic Retinoids", 32 *J. Med Chem*., 1504 (1989) and U.S. Patent Nos. 4,831,052, 4,874,747 ,4,925,979, 5,004,730, 5,124,473 and Re 33,533.

## Summary of the Invention

The present invention provides novel tricyclic retinoid compounds that have selective activity on RARs and RXRs or pan-agonist activity on one or more of each of the RAR and RXR isoforms. The present invention also provides pharmaceutical compositions incorporating these novel tricyclic compounds and methods for the therapeutic use of such compounds and pharmaceutical compositions.

These and various other advantages and features of novelty which characterize the invention are pointed out with particularity in the claims annexed hereto and forming a part hereof However, for a better understanding of the invention, its advantages, and objects obtained by its use, reference should be had to the accompanying drawings and descriptive matter, in which there is illustrated and described preferred embodiments of the invention.

## Definitions

In accordance with the present invention and as used herein, the following terms are defined with the following meanings, unless explicitly stated otherwise.

The term alkyl refers to straight-chain, branched-chain, cyclic structures, and combinations thereof.

The term aryl refers to an optionally substituted six-membered aromatic ring.

The term heteroaryl refers to an optionally substituted five-membered or six-membered heterocyclic ring containing one or more heteroatoms selected from the group consisting of oxygen, nitrogen and sulfur.

The terms retinoid or retinoids refer to compound(s) that bind and/or activate one or more retinoid receptors, thereby affecting the transcriptional activity of a target gene to which the activated receptor and compound complex binds.

The term pan-agonist refers to a retinoid that activates at least one member of both the RARs subfamily (i.e., RARα, RARβ, or RARγ) and the RXR subfamily (i.e., RXRα, RXRβ or RXRγ). Preferably such pan-agonist retinoids activate all members of both the RAR and RXR subfamilies of retinoid receptors.

## Detailed Description of Embodiments of the Invention

In accordance with a first aspect of the present invention, we have developed tricyclic retinoid compounds of the formulae:

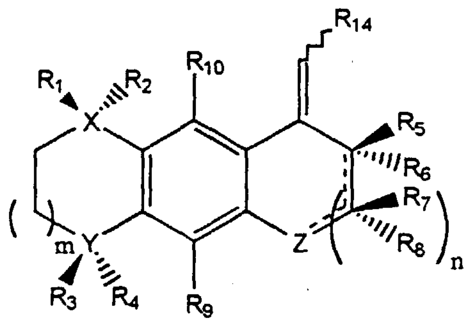

(I)

OR

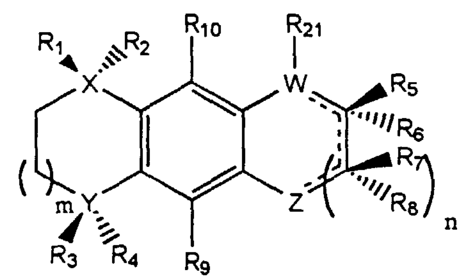

(II)

OR

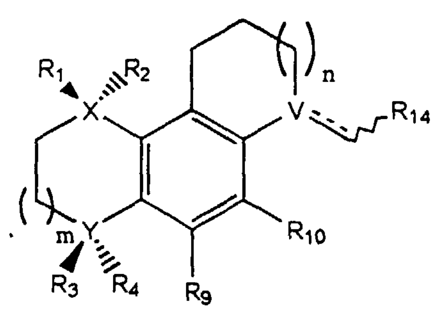

(III)

OR

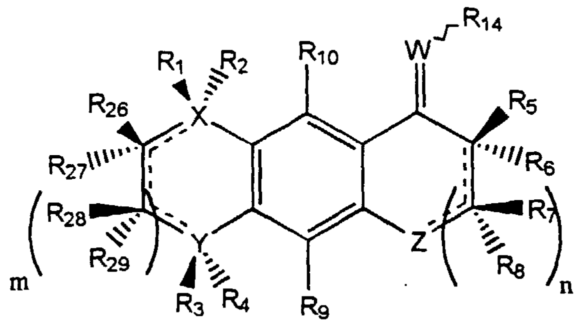

(IV)

OR

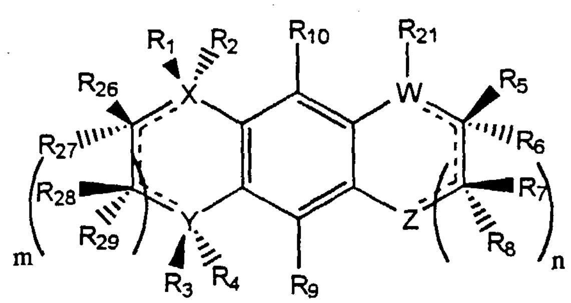

(V)

OR

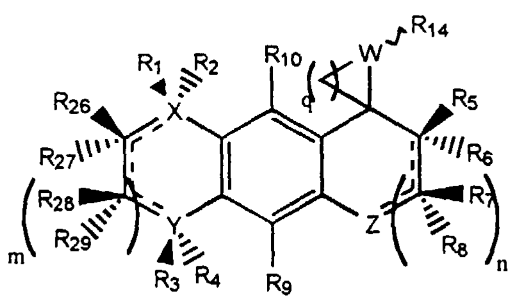

(VI)

wherein,

$R_1$ through $R_4$ each independently are hydrogen, a $C_1$ - $C_6$ alkyl, or a $C_7$ - $C_{15}$ arylalkyl;

$R_5$ through $R_8$ each independently are hydrogen, a $C_1$ - $C_6$ alkyl, or at least two of $R_5$ through $R_8$ taken together are a $C_3$ - $C_6$ cycloalkyl;

$R_9$ and $R_{10}$ each independently are hydrogen, a $C_1$ - $C_6$ alkyl, F, Cl, Br, $NR_{11}R_{12}$, $NO_2$ or $OR_{13}$, where $R_{11}$ and $R_{12}$ each independently are hydrogen, a $C_1$ - $C_8$ alkyl, a $C_7$ - $C_{15}$ arylalkyl, a $C_1$ - $C_8$ acyl, provided that only one of $R_{11}$ or $R_{12}$ can be acyl, or $R_{11}$ and $R_{12}$ taken together are a $C_3$ - $C_6$ cycloalkyl, and where $R_{13}$ is hydrogen or a $C_1$ - $C_8$ alkyl or a $C_7$-$C_{15}$ arylalkyl;

$R_{14}$ represents:

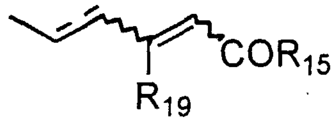

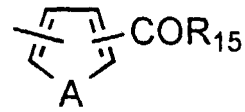

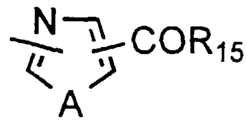

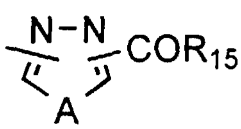

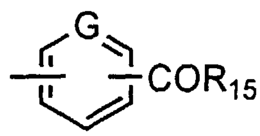

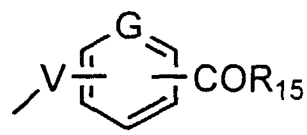

where $R_{15}$ is $OR_{16}$ or $NR_{17}R_{18}$, with $R_{16}$ being hydrogen, a $C_1$ - $C_6$ alkyl or a $C_7$ - $C_{15}$ arylalkyl, and with $R_{17}$ and $R_{18}$ each independently being hydrogen, a $C_1$ - $C_6$ alkyl, a $C_7$ - $C_{15}$ arylalkyl, aryl, ortho-, meta-, or para-substituted hydroxyaryl, or taken together are a $C_3$ - $c_6$ cycloalkyl, provided that $R_{18}$ must be hydrogen when $R_{17}$ is aryl or hydroxyaryl, $R_{19}$ is a $C_1$ - $C_5$ alkyl, and A is O, S or $NR_{20}$, where $R_{20}$ is a hydrogen, $C_1$ - $C_6$ alkyl or a $C_7$ - $C_{15}$ arylalkyl;

$R_{21}$ represents:

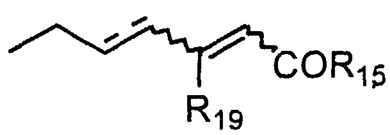

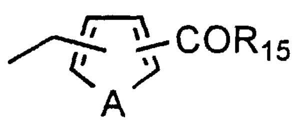

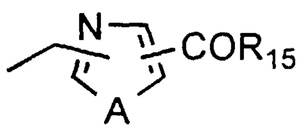

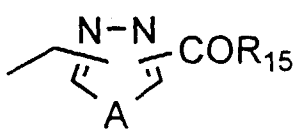

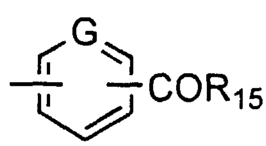

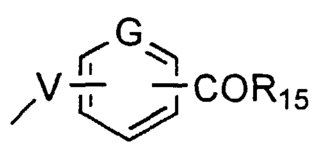

where $R_{15}$ $R_{19}$, and A have the same definitions given above;

$R_{26}$ through $R_{29}$ each independently are hydrogen or a $C_1$ - $C_6$ alkyl, or taken together then one each of $R_{26}$ and $R_{27}$ or $R_{28}$ and $R_{29}$ respectively, form a carbonyl group;

X and Y each independently represent C, O, S, N, SO or $SO_2$, provided, however, that when X or Y are O, S, SO or $SO_2$, then either $R_1$ and $R_2$ or $R_3$ and $R_4$ respectively do not exist, and further provided, that when X or Y is N, then one each of $R_1$ and $R_2$ or $R_3$ and $R_4$ respectively, do not exist;

Z is O, S, $CR_{22}R_{23}$ or $NR_{24}$, where $R_{22}$ through $R_{24}$ each independently are hydrogen or a $C_1$ - $C_6$ alkyl or $R_{22}$ and $R_{23}$ taken together are a $C_3$ - $C_6$ cycloalkyl;

W is N or $CR_{25}$, where $R_{25}$ is hydrogen or a $C_1$ - $C_6$ alkyl;

V is C or N, provided, however, that when V is N, then no double bond exists adjacent to V;

G is C or N, provided G cannot be C when W is C;

M is C, O, S, $NR_{24}$, where $R_{24}$ is hydrogen, a $C_1$- $C_6$ alkyl, or does not exist if a double bond exists adjacent to N;

m is 0 or 1 carbon atoms;

n is 0, 1 or 2 carbon atoms;

q is 1 or 2 carbon atoms;

the dashed lines in the structures represent optional double bonds, provided, however, that the double bonds cannot be contiguous, and further provided that when such optional double bonds exist then one each of $R_5$ and $R_6$ or $R_7$ and $R_8$ respectively do not exist; and

the wavy lines represent olefin bonds that are either in the *cis* (Z) or *trans* (E) configuration.

The compounds of the present invention also include all pharmaceutically acceptable salts, as well as esters and amides. Preferably, such salts, esters and amides, will be formed at the $R_{15}$ and $R_{16}$ positions. As used in this disclosure, pharmaceutically acceptable salts include, but are not limited to: pyridine, ammonium, piperazine, diethylamine, nicotinamide, formic, urea, sodium, potassium, calcium, magnesium, zinc, lithium, cinnamic, methylamino, methanesulfonic, picric, tartaric, triethylamino, dimethylamino, and tris(hydoxymethyl)aminomethane. Additional pharmaceutically acceptable salts are known to those skilled in the art.

The compounds of the present invention are particularly useful in the treatment of skin-related diseases, including, without limitation, actinic keratoses, arsenic keratoses, inflammatory and non-inflammatory acne, psoriasis, ichthyoses and other keratinization and hyperproliferative disorders of the skin, eczema, atopic dermatitis, Darriers disease, lichen planus, prevention and reversal of glucocorticoid damage (steroid atrophy), as a topical anti-microbial, as skin pigmentation agents and to treat and reverse the effects of age and photo damage to the skin. The compounds are also useful for the prevention and treatment of cancerous and precancerous conditions, including, premalignant and malignant hyperproliferative diseases such as cancers of the breast, skin, prostate, cervix, uterus, colon, bladder, esophagus, stomach, lung, larynx, oral cavity, blood and lymphatic system metaplasias, dysplasias, neoplasias, leukoplalcias and papillomas of the mucous membranes and in the treatment of Kaposis sarcoma. In addition, the present compounds can be used as agents to treat diseases of the eye, including, without limitation, proliferative vitreoretinopathy (PVR), retinal detachment, dry eye and other corneopathies, as well as in the treatment and prevention of various cardiovascular diseases, including, without limitation, diseases associated with lipid metabolism such as dyslipidemias, prevention of restenosis and as an agent to increase the level of circulating tissue plasminogen activator (TPA). Other uses for the compounds of the present invention include the prevention and treatment of conditions and diseases associated with human papilloma virus (HPV), including warts and genital warts, various inflammatory diseases such as pulmonary fibrosis, ileitis, colitis and Krohn's disease, neurodegenerative diseases such as Alzheimer's disease, Parkinson's disease and Amyotrophic Lateral Sclerosis (ALS), improper pituitary function, including insufficient production of growth hormone, modulation of apoptosis, including both the induction of apoptosis and inhibition of T-Cell activated apoptosis, restoration of hair growth, including combination therapies with the present compounds and other agents such as Minoxidil®, diseases associated with the immune system, including use of the present compounds as immunosuppressants and immunostimulants, modulation of organ transplant rejection and facilitation of wound healing, including modulation of chelosis. It will also be understood by those skilled in the art that the retinoid compounds of the present invention will prove useful in any therapy in which retinoids, including RAR selective retinoids, RXR selective retinoids, and pan-agonist retinoids will find application.

Furthermore, it will be understood by those skilled in the art that the compounds of the present invention, including pharmaceutical compositions and formulations containing these compounds, can be used in a wide variety of combination therapies to treat the conditions and diseases described above. Thus, the compounds of the present invention can be used in combination with other therapies, including, without limitation, chemotherapeutic agents such as cytostatic and cytotoxic agents, immunological modifiers such as interferons, interleukins, growth hormones and other cytokines, hormone therapies, surgery and radiation therapy.

Representative retinoids of the present invention include, without limitation, ethyl (2E,4E)-3-methyl-6-[(Z)-3,4,5,6,7,8-hexahydro-5,5,8,8-tetramethyl-2H-anthracen-1-ylidene]hexa-2,4-dienoate; (2E,4E)-3-methyl-6-[(Z)-3,4,5,6,7,8-hexahydro-5,5,8,8-tetramethyl-2H-anthracen-1-ylidene]hexa-2,4-dienoic acid; ethyl (2E,4E)-3-methyl-6-[(Z)-2,3-5,6,7,8-hexahydro-5,5,8,8-tetramethylcyclopenta[b]napthalen-1-ylidene]hexa-2,4-dienoate; (2E,4E)-3-methyl-6-[(Z)-2,3-5,6,7,8-hexahydro-5,5,8,8,-tetramethylcyclopenta[b]naphthalen-1-ylidene]hexa-2,4-dienoic acid; ethyl (2E,4E)-3-methyl-6-[(Z)-1,2,3,6,7,8-hexahydro-1,1,3,3-tetramethylcycopenta[b]naphthalen-5-ylidene]hexa-2,4-dienoate; (2E,4E)-3-methyl-6-[(Z)-1,2,3,6,7,8-hexahydro-1,1,3,3-tetracyclopenta[b]naphthalen-5-ylidene]hexa-2,4-dienoic acid; (2E,4E)-3-methyl-6-(2,3,6,7,8,9-hexahydro-2,2,6,6,9,9-hexamethylbenzo[b]-chromen-4-ylidene)hexa-2,4-dienoic acid; ethyl (2E,4E)-3-methyl-6-[(Z)3,4,5,6,7,8-hexahydro-10-nitro-5,5,8,8-tetramethyl-2H-anthracen-1-ylidene]hexa-2,4-dienoate; (2E,4E)-3-methyl-6-[(Z)-3,4,5,6,7,8-hexahydro-10-nitro-5,5,8,8-tetramethyl-2H-anthracen-1-ylidene]hexa-2,4-dienoic acid; ethyl (2E,4E)-3-methyl-6-[(Z)-3,4,5,6,7,8-hexahydro-4,4,5,5,8,8-hexamethyl-2H-anthracen-1-ylidene]hexa-2,4-dienoate; (2E,4E)-3-methyl-6-[(Z)-3,4,5,6,7,8-hexahydro-4,4,5,5,8,8-hexamethyl-2H-anthracen-1-ylidene]hexa-2,4-dienoic acid; (2E,4E)-3-methyl-6-[(Z)-2,3,5,6,7,8-hexahydro-3,5,5,8,8-pentamethylcyclopenta[b]naphthalen-1-ylidene]hexa-2,4,dienoic acid; (2E,4E)-3-methyl-6-[(Z)-3,5,6,7-tetrahydro-5,5,7,7-tetramethyl-2H-5-indacen-1-ylidene]hexa-2,4-dienoic acid; ethyl (2E,4E)-3-methyl-6-[(E)-3,4,5,6,7,8-hexahydro-5,5,8,8-tetramethyl-2H-anthracen-1-ylidene]hexa-2,4-dienoate; (2E,4E)-3-methyl-6-[(E)-3,4,5,6,7,8-hexahydro-5,5,8,8-tetramethyl-2H-anthracen-1-ylidene]hexa-2,4-dienoic acid; ethyl (2E,4E)-3-methyl-6-[(E)-2,3,5,6,7,8-hexahydro-5,5,8,8-tetramethylcyclopenta[b]naphthalen-1-ylidene]hexa-2,4-dienoate; (2E,4E)-3-methyl-6-[(E)-2,3,5,6,7,8-hexahydro-5,5,8,8-tetramethylcyclopenta[b]naphthalen-1-ylidene]hexa-2,4-dienoic acid; ethyl (2E,4E)-3-methyl-6-[(E)-1,2,3,6,7,8-hexahydro-1,1,3,3-tetramethylcyclopenta[b]naphthalen-5-ylidene]hexa-2,4-dienoate; (2E,4E)-3-methyl-6-[(E)-1,2,3,6,7,8-hexahydro-1,1,3,3-tetramethylcyclopenta[b]-5-ylidene]hexa-2,4-dienoic acid; ethyl (2Z,4E)-3-methyl-6-[(E)-1,2,3,6,7,8-hexahydro-1,1,3,3-tetramethylcyclopenta[b]naphthalen-5-ylidene]hexa-2,4-dienoate; (2Z,4E)-3-methyl-6-[(E)-1,2,3,6,7,8-hexahydro-1,1,3,3-tetramethylcyclopenta[b]naphthalen-5-ylidene]hexa-2,4-dienoic acid; (2E,4E)-3-methyl-6-(E)-[4,4,6,6,9,9-hexamethyl-1,2,3,4,5,6,7,8-octahydroanthracen-1-ylidene]hexa-2,4-dienoic acid; ethyl

(2E,4E,6E)-3-methyl-6-[1,2,3,4,7,8,9,10-octahydro-1,1,4,4-tetramethyl-6H-naphthocycloheptene-10-yl]hexa-2,4,6-trienoate; ethyl (2E,4E,6E)-3-methyl-6-[1,2,3,4,7,8,9,10-octahydro-1,1,4,4-tetramethyl-6H-naphthocycloheptene-10-yl]hexa-2,4,6-trienoate; ethyl (2Z,4E,6E)-3-methyl-6-[1,2,3,4,7,8,9,10-octahydro-1,1,4,4-tetramethyl-6H-naphthocycloheptene-10-yl]hexa-2,4,6-trienoate; (2Z, 4E, 6E)-3-methyl-6-[1,2,3,4,7,8,9,10-octahydro-1,1,4,4-tetramethyl-6H-naphthocycloheptene-10-yl]hexa-2,4,6-trienoic acid; (2E,4E)-3-methyl-6-[(E)-3,5,6,7-tetrahydro-5,5,7,7-tetramethyl-2H-5-indacen-1-ylidene]hexa-2,4-dienoic acid; ethyl (2E,4E)-3-methyl-6-[(E)-3,4,5,6,7,8-hexahydro-10-nitro-5,5,8,8-tetramethyl-2H-anthracen-1-ylidene]hexa-2,4-dienoate; (2E,4E)-3-methyl-6-[(E)-53,4,5,6,7,8-hexahydro-10-nitro-5,5,8,8-tetramethyl-2H-anthracen-1-ylidene]hexa-2,4-dienoic acid; (2Z,4E)-3-methyl-6-[(E)-3,4,5,6,7,8-hexahydro-10-nitro-5,5,8,8-tetramethyl-2H-anthracen-1-ylidene]hexa-2,4-dienoic acid; (2E,4E)-3-methyl-6-[(E) 2,3,6,7,8,9-hexahydro-6,6,9,9-tetramethylbenzo[g]chromen-4-ylidene]hexa-2,4-dienoic acid; (2Z,4E)-3-methyl-6-[(E)-2,3,6,7,8,9-hexahydro-6,6,9,9-tetramethylbenzo[g]chromen-4-yliden]hexa-2,4-dienoic acid; (2E,4E)-3-methyl-6-[(E)-,3,6,7,8,9-hexahydro-6,6,9,9-tetramethylbenzo[g]chromen-4-ylidene]hexa-2,4-dienoic acid; ethyl (2E,4E)-3-methyl-6-(3,4,5,6,7,8-hexahydro-5,5,8,8-tetramethylanthracen-1-yl)hexa-2,4-dienoate; (2E,4E)-3-methyl-6-(3,4,5,6,7,8-hexahydro-5,5,8,8-tetramethylanthracen-1-yl)hexa-2,4-dienoic acid; (3E,5E)-3-methyl-6-(3,4,5,6,7,8-hexahydro-5,5,8,8-tetramethylanthracen-1-yl)hexa-3,5-dienoic acid; ethyl (2E,4E)-3-methyl-6-(1,2,3,4,5,6,7,8-octahydro-5,5,8,8-tetramethylanthracen-1-yl)hexa-2,4-dienoate; (2E,4E)-3-methyl-6-(1,2,3,4,5,6,7,8-octahydro-5,5,8,8-tetramethyl-anthracen-1-yl)hexa-2,4-dienoic acid; ethyl (2E,4E)-3-methyl-6-(1,2,3,5,6,7,8-heptahydro-5,5,8,8-tetramethylcyclopenta[b]napthalen-1-yl)hexa-2,4-dienoate; (2E,4E)-3-methyl-6-1,2,3(5,6,7,8-heptahydro-5,5,8,8-tetramethylcyclopenta[b]naphthalen-1-yl]hexa-2,4-dienoic acid; ethyl (2E,4E)-3-methyl-6 [1,2,3,4,7,8,9,10-octahydro-1,1,4,4-tetramethyl-6H-naphthocycloheptan-10-yl]hexa-2,4-dienoate; (2E,4E)-3-methyl-6-[1,2,3,4,7,8,9,10-octahydro-1,1,4,4-tetramethyl-6H-naphthocycloheptan-10-yl]hexa-2,4-dienoic acid; ethyl (2Z,4E)-3-methyl-6-[1,2,3,4,7,8,9,10-octahydro-1,1,4,4-tetramethyl-6H-naphthocycloheptan-10-yl]hexa-2,4-dienoate; (2Z,4E)-3-methyl-6-[1,2,3,4,7,8,9,10-octahydro-1,1,4,4-tetramethyl-6H-naphthocycloheptan-10-yl]hexa-2,4-dienoic acid; (2Z,4E)-3-methyl-6-[1,2,3,4,7,8,9,10-octahydro-1,1,4,4-tetramethyl-6H-naphthocycloheptan-10-yl]hexa-2,4-dienoic acid; (2Z,4E)-3-methyl-6-[5,6,7,8-tetrahydro-5,5,8,8-tetramethylnaphthyl-(2,3-b)-2,2-dimethylpyran-4-yl]hexa-2,4-dienoic acid; (2E,4E)-3-methyl-6-[5,6,7,8-tetrahydro-5,5,8,8-tetramethylnaphthyl-(2,3-b)-pyran-4-yl]hexa-2,4-dienoic acid; (2Z,4E)-3-methyl-6-[5,6,7,8-tetrahydro-5,5,8,8-tetramethylnaphthyl-(2,3-b )-pyran-4-yl]hexa-2,4-dienoic acid; (+)-(2E,4E)-3-methyl-6-(1,2,3,4,5,6,7,8-octahydro-5,5,8,8-tetramethylanthracen-1-yl)hexa-2,4-dienoic acid; (-)-(2E,4E)-3-methyl-6-(1,2,3,4,5,6,7,8-octahydro-5,5,8,8-tetramethylanthracen-1-yl)hexa-2,4-dienoic acid; methyl (2E)-3-methyl-6-(1,2,3,4,6,7,8,9-octahydro-6,6,9,9-tetramethylanthracen-1-yl)hex-2-enoate; (2E)-3-methyl-6-(1,2,3,4,6,7,8,9-octahydro-6,6,9,9-tetramethylanthracen-1-yl)hex-2-enoic acid; (2E)-3-methyl-6-(1,2,3,4,6,7,8,9-octahydro-6,6,9,9-tetramethylanthracen-1-yl)hex-2-enoic acid; (2E,4E)-3-methyl-6-[(E)-2,3,6,7,8,9-hexahydro-6,6,9,9-tetramethyl-1H-cyclopenta[a]naphthalen-3-ylidene]hexa-2,4-dienoic acid; ethyl (2E,4E)-3-methyl-6-(2,3,6,7,8,9-hexahydro-6,6,9,9-tetramethyl-1H-cyclopenta[a]naphthalen-3-yl]hexa-2,4-dienoate; (2E,4E)-3-methyl-6-(2,3,6,7,8,9-hexahydro-6,6,9,9-tetramethyl-1H-cyclopenta[a]naphthalen-3-yl)hexa-2,4-dienoic acid; ethyl (2E,4E)-3-methyl-6-[(Z)-1,2,3,4,7,8,9-heptahydro-7,7,9,9-tetramethylcyclopenta[naphthalen-4-ylidene]hexa-2,4-dienoate; (2E,4E)-3-methyl-6-[(Z)-1,2,3,4,7,8,9-heptahydro-7,7,9,9-tetramethylcyclopenta[f]naphthalen-4-ylidene]hexa-2,4-dienoic acid; ethyl (2E,4E)-3-methyl-6-(7,7,10,10-tetramethyl-2,3,4,5,7,8,9,10-octahydronaphtho[2,3-6]-azepinyl)hexa-2,4- dienoate; (2E,4E)-3-methyl-6-[7,7,10,10-tetramethyl-2,3,4,5,7,8,9,10-octahydronaphtho[2,3-6]azepinyl)hexa-2,4-dienoic acid; ethyl 3-methyl-6-(3,4,6,7,8,9-hexahydro-6,6,9,9-tetramethy-2H-benzo[g]quinolin-1-yl)hexa-2,4-dienoate; (2E,4E)-3-methyl-6-[3,4,6,7,8,9-hexahydro-6,6,9,9-tetramethyl-2H-benzo[g]quinolin-1-yl)hexa-2,4-dienoic acid; ethyl (2E,4E)-3-methyl-6-oxo-6-[5,6,7,8-tetrahydro-5,5,8,8-tetramethyl(2,3)naphthyl [b]piperidin-1-yl]hexa-2,4-dienoate; (2E,4E)-3-methyl-6-oxo-6-(3,4,6,7,8,9-hexahydro-6,6,9,9-tetramethyl-2H-benzo[g]quinolin-1-yl)hexa-2,4-dienoic acid; ethyl (2E,4E)-3-methyl-6-oxo-6-[(2,3,5,6,7,8-hexahydro-5,5,8,8-tetramethyl)benzo[f]indol-1-yl]hexa-2,4-dienoate; (2E,4E)-3-methyl-6-oxo-6-[(2,3,5,6,7,8-hexahydro-5,5,8,8-tetramethyl)benzo-[f]-indol-1-yl]hexa-2,4-dienoic acid; ethyl (2E,4E)-3-methyl-6-(2,3,5,6,7,8-hexahydro-5,5,8,8-tetramethylbenzo[f]indol-1-yl]hexa-2,4-dienoate; (2E,4E)-3-methyl-6-(2,3,5,6,7,8-hexahydro-5,5,8,8-tetramethylbenzo[f]indol-1-yl]hexa-2,4-dienoic acid; ethyl (2E,4E)-3-methyl-6-(7,7,10,10-tetrahydro-5,5,8,8-tetramethyl)benzo[f]quinolin-4-yl)hexa-2,4-dienoate; (2E,4E)-3-methyl-6-(1,2,3,4,7,8,9,10-octahydro-7,7,10,10-tetramethylbenzo[f]quinolin-4-yl)hexa-2,4-dienoic acid; (2E, 4E)-3-methyl-6-[(Z)-5,6,7,8-tetrahydro-5,5,8,8-tetramethylnaphthyl(2,3-b-pyran-4-ylidene]hexa-2,4-dienoic acid; (2E,4E)-3-methyl-[(E)-3,4,5,6,7,8-hexahydro-2,2,5,5,8,8-hexamethyl-2H-anthracen-1-ylidene]-hexa-2,4,-dienoic acid; Ethyl (2E,4E)-3-methyl-6-[(Z)-N-acyl-3,4,5,6,7,8-hexahydro-10-amino-5,5,8,8-tetramethyl-2H-anthracen-1-ylidene]hexa-2,4-dienoate; (2E,4E)-3-methyl-6-[(Z)-N-acyl-3,4,5,6,7,8-hexahydro-10-amino-5,5,8,8-tetramethyl-2H-anthracen-1-ylidene]hexa-2,4-dienoic acid; (2E,4E)-3-methyl-6-[(E)1-ethyl-6,6,9,9-tetramethyl-2,3,6,7,8,9-hexahydro-1H-benzo[g]quinolin-4-ylidene]-hexa-2,4,dienoic acid; 4-(5-carboxy-penta-2E-4E-dieneylidene)-6,6,9,9-tetramethyl-3,4,6,7,8,9-hexahydro-2H-benzo[g]quinolin-1-carboxylic acid tert butyl ester; (2E,4E)-3-methyl-(6,6,9,9-tetramethyl-2,3,6,9-tetrahydro-naphtho[2,3-b]-[1,4]oxazin-4-yl)-hexa-2,4-dienoic acid; (2E,4E)-3-methyl-6-oxo-6-(6,6,9,9-tetrahydro-2,3,6,9-tetrahydro-naphtho[2,3-b][1,4]oxazin-4-yl)hexa-2,4-dienoic acid; (2E,4E)-3-methyl-6-(6-ethyl-1,9,9-trimethyl-7-oxo-2,3,6,7,8,9-hexahydro-1H-pyrido[2,3-g]quinolin-4-ylidene)-hexa-2,4-dienoic acid; E-4-(6,6,9,9-Tetramethyl-1,2,6,7,8,9-hexahydro-2H-anthracen-1-yl)hydrazonebenzoic acid; E-4[1,2,3,4-Tetrahydro-6,6,9,9-tetramethylnaphto(2,3)pyran-4-yl(2-hydrazono)benzoic acid; and (2E,4E) 6-(6-ethyl-1,9,9-trimethyl-2,3,6,9-tetrahydro-1H-pyrido[2,3-g]quinolin-4-ylidene)-3-methyl-hexa-2,4-dienoic acid.

The compounds of the present invention can be obtained by modification of the compounds disclosed or by a total synthesis approach, by techniques known to those skilled in the art. In this regard, the synthesis of the compounds of the present invention often follow established retinoid synthesis schemes and techniques as described in M.I. Dawson and W.H. Okamura, "Chemistry and Biology of Synthetic Retinoids", Chapters 3, 8, 14 and 16, CRC Press, Inc., Florida (1990); M.I. Dawson and P.D. Hobbs, *The Synthetic Chemistry of Retinoids, In* Chapter 2: "The Retinoids, Biology, Chemistry and Medicine", M.B. Sporn et al., Eds. (2nd ed.), Raven Press, New York, New York, pp. 5-178 (1994); R.S.H. Liu and A. E. Asato, "Photochemistry and Synthesis of Stereoisomers of Vitamin A," 40 *Tetrahedron,* 1931 (1984); 43 *Cancer Res.*, 5268 (1983); 15 *Eur. J. Med Chem.*, 9 (1980); and U.S. Patent Nos. 4,326,055 and 4,578,498, the disclosures of which are herein incorporated by reference. The sequence of steps of the general schemes of synthesizing the compounds of the present invention are shown below. In addition, more detailed and illustrative synthetic schemes for specific compounds of the present invention will be found in the Examples included herein.

## Scheme I: Synthesis of Compounds of Structure (I):

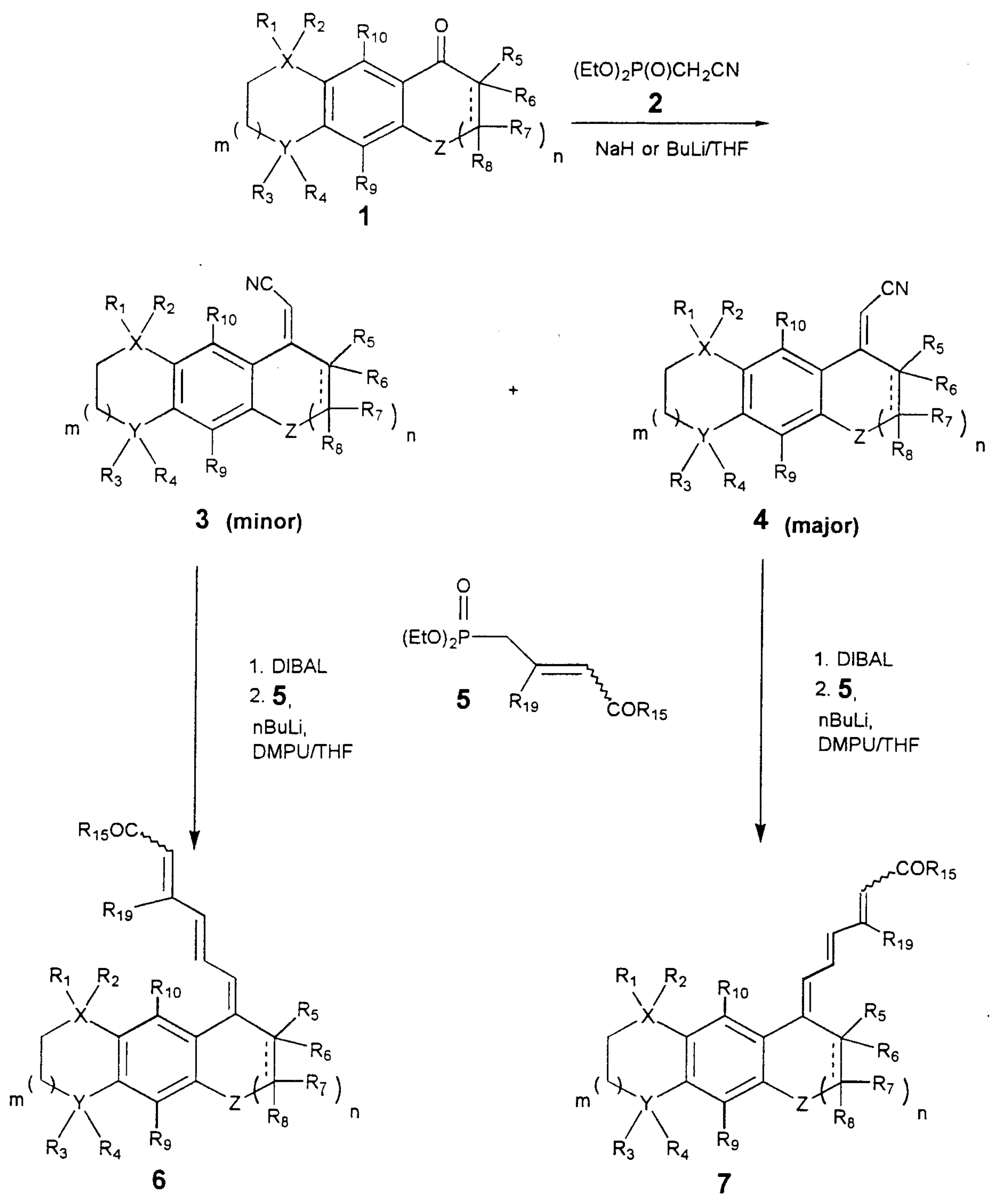


The tricyclic derivatives of the present invention, that is compounds of general structures **6** and **7**, may be prepared in accordance with reaction Scheme I. The starting materials for this sequence, ketones of general structure **1**, may be prepared from the appropriately substituted octahydroanthracene by oxidation with chromium trioxide in acetic acid at ambient temperature or with chromium trioxide in methylene chloride/pyridine at 0°C. Further, in accordance

with this sequence of reactions tricyclic ketones of general structure **1** are condensed with the sodium or lithium salt of diethyl cyanomethylphosphonate in THF at reduced temperatures in a Horner-Wadsworth-Emmons olefination reaction to provide a mixture of the *cis*-cyano olefins **3** as the minor products and the *trans*-cyano olefins **4** as the major products. The olefinic products may be separated by silica gel flash column chromatography.

The *cis*-cyano olefins **3** are reduced with DIBAL at -78°C to give the intermediate enals. The solvent to be used in the reduction includes methylene chloride, hexanes, and THF. The product of the DIBAL reduction, the enal, is reacted with the lithium salt of diethyl 3-ethoxycarbonyl-2-methylprop-2-enylphosphonate (mixture of double bond isomers) in THF at reduced temperatures in a Homer-Wadsworth-Emmons olefination reaction to provide the tricyclic derivatives of general structure **6** where $R_{15}$ is ethyl. The olefination reaction is preferably conducted in the presence of 1,3-dimethyl-3,4,5,6-tetrahydro-2(1H)-pyrimidinone (DMPU). The acids and salts derived from structure **6** are readily obtainable from the corresponding esters. The ethyl esters may be hydrolyzed in an alkanol solvent at ambient temperature with about a three molar excess of base, for example, potassium hydroxide. Alternatively, the ethyl esters may be hydrolyzed in THF/water or acetone/water at ambient temperature with, for example, excess lithium hydroxide. The hydrolysis solution is acidified and the hydrolysate recovered by conventional means to give as the major product the (2E, 4E)-Z-ylidene tricyclic carboxylic acid derivatives of structure **6** where $R_{15}$ is OH. The minor (2Z, 4E)-Z-ylidene geometric isomers of general structure **6**, by-products of the olefination reaction, are readily isolated by silica gel chromatography of the hydrolysate mixture.

In an analogous fashion, in accordance with reaction Scheme I, the *trans*-cyano olefins **4** are transformed into the (2E, 4E)-E-ylidene and (2Z, 4E)-E-ylidene tricyclic derivatives **7** where $R_{15}$ is ethoxy and OH.

## Scheme II: Synthesis of Compounds of Structure (I):

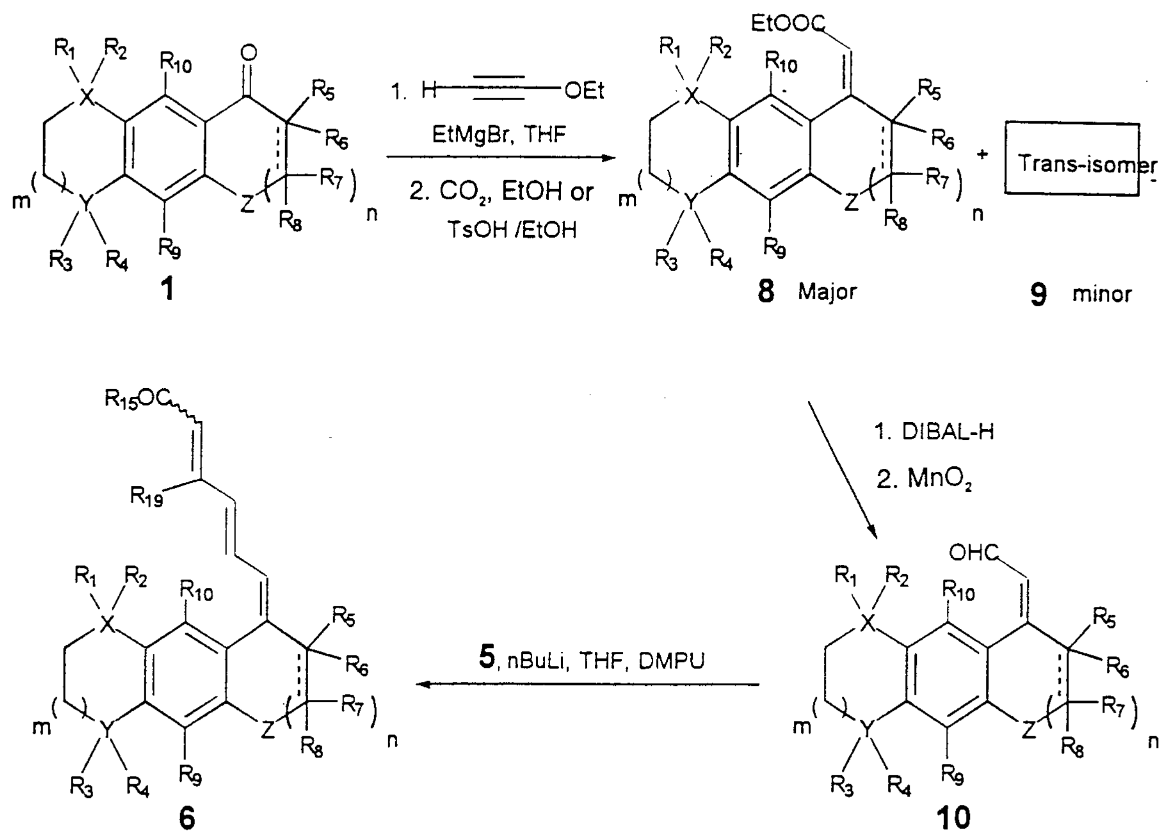


1 — 8 Major — 9 minor — 6 — 10

An alternative means for making the tricyclic derivatives of general structure **6** is in accordance with reaction Scheme II. Tricyclic ketones **1** are treated with ethoxy ethynyl magnesium bromide in THF at 0°C to ambient temperature. The resulting propargylic alcohols are isolated by typical extractive means, dissolved in ethanol and treated with carbon dioxide (gas) or, alternatively, a catalytic amount of *p*-toluensulfonic acid and/or camphor sulfonic acid to provide

a mixture of the *cis*-olefinic esters **8** as the major product and the *trans*-olefinic esters of general structure **9** as the minor product. The olefinic products may be separated by silica gel flash column chromatography.

The *cis*-olefinic esters of general structure **8** are reduced with DIBAL at -78°C to give the intermediate allylic alcohol. The solvent to be used in the reduction includes methylene chloride, hexanes, and THF. The product of the DIBAL reduction, the allylic alcohol, is oxidized with manganese dioxide in methylene chloride at ambient temperature to provide the *cis*-enals of general structure **10**. The enals are converted into tricyclic derivatives of general structure **6** by condensation with the phosphonate **5** by the same processes as those employed in the preparation process of Scheme I to produce tricycles of general structure **6** and **7**.

**Scheme III: Synthesis of Compounds of Structure (I):**

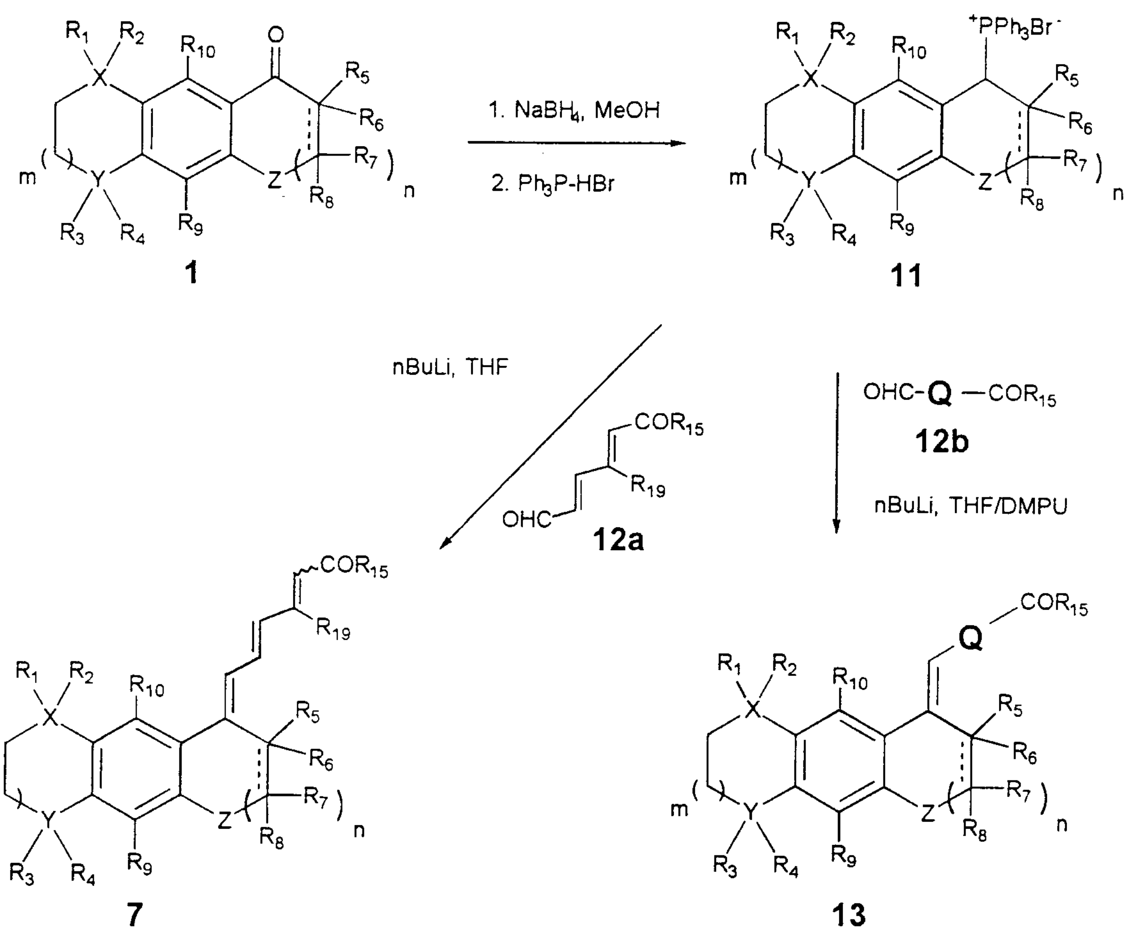


Q = 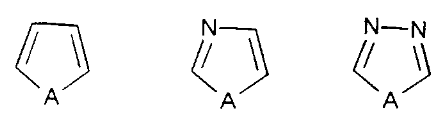

An alternative means for making the tricyclic derivatives of general structure **7** is in accordance with reaction Scheme III. The starting material for this sequence, ketones **1**, may be prepared from the appropriately substituted

octahydroanthracenes by oxidation with chromium trioxide in acetic acid at ambient temperature or with chromium trioxide in methylene chloride/pyridine at low temperature. The tricyclic ketones are reduced with sodium borohydride in methanol at low temperature and the resultant benzylic alcohols are reacted with triphenylphosphine hydrobromide in methanol at elevated temperature to provide phosphonium salts of general structure **11**. Compounds **12a** where $R_{15}$ is ethyl and $R_{19}$ is methyl used for the following olefination reaction are prepared from commercially available (triphenylphosphoranylidene)acetaldehyde and ethyl 3-methyl-4-oxo-crotonate. A Horner-Wadsworth-Emmons olefination reaction in THF at reduced temperatures with the lithium salt of compounds **11** and compounds of general structure **12a** provide the tricyclic derivatives **7** where $R_{15}$ is ethoxy. The olefination reaction is preferably conducted in the presence of 1,3-dimethyl-3,4,5,6-tetrahydro-2(1H)-pyrimidinone (DMPU). The acids and salts derived from general structure **7** are readily obtainable from the corresponding esters by the same processes as those employed in the previously described process for tricycles of general structure **6**.

Compounds of general structure **13** may be prepared from the lithium salt of the phosphonium bromide of general structure **11** and aldehyde of general structure **12b** by the same olefination processes as those employed for the preparation of a compound of general structure **7**. The acids and salts derived from general structure **13** are readily obtainable from the corresponding esters by the same processes as those employed in the preparation process of Scheme I for tricycles of general structures **6** and **7**.

## Scheme IV: Synthesis of Compounds of Structure (I):

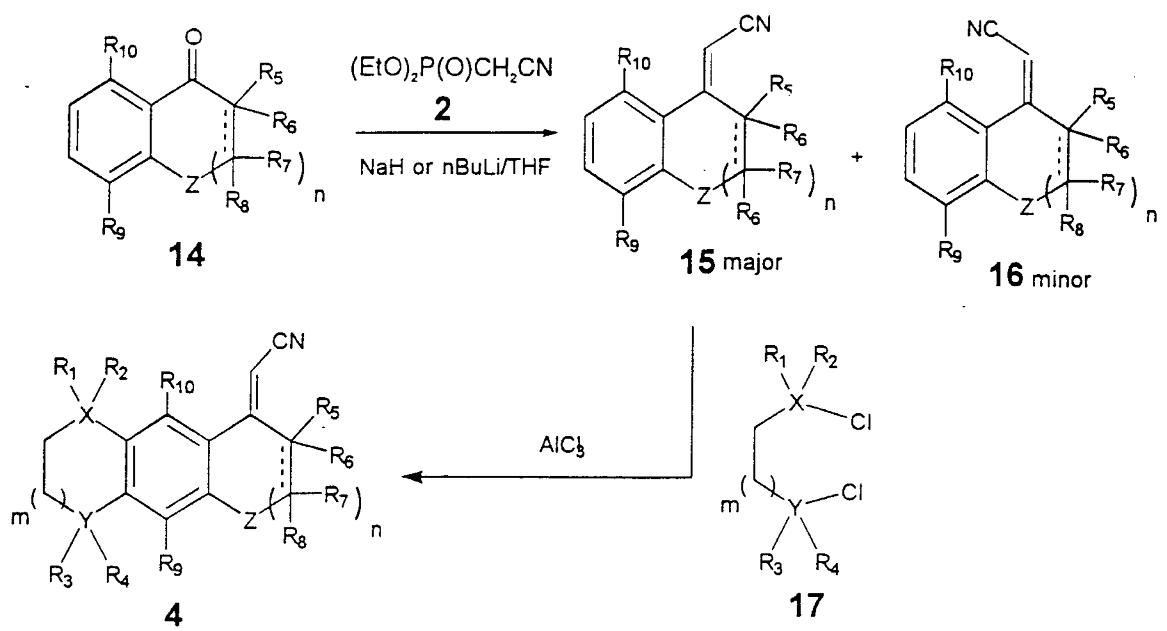


**14** **15** major **16** minor **4** **17**

An alternative means for making the intermediate tricyclic derivatives of general structure **4** is in accordance with reaction Scheme IV. The bicyclic ketones **14** are condensed with the sodium or lithium salt of diethyl cyanomethylphosphonate in THF at reduced temperatures in a Horner-Wadsworth-Emmons olefination reaction to provide a mixture of the *trans*-cyano olefins **15** as the major products and the *cis*-cyano olefins **16** as the minor products. The olefinic products may be separated by silica gel flash column chromatography. The tricyclic derivatives of general structure **4** are prepared by aluminum trichloride catalyzed Friedel-Crafts alkylation/cyclization of 2,5-dichloro-2,5-dialkylhexanes **17** with the bicycles of general structure **15** and **16** in dichloromethane at ambient temperature.

Scheme V: Synthesis of Compounds of Structure (II):

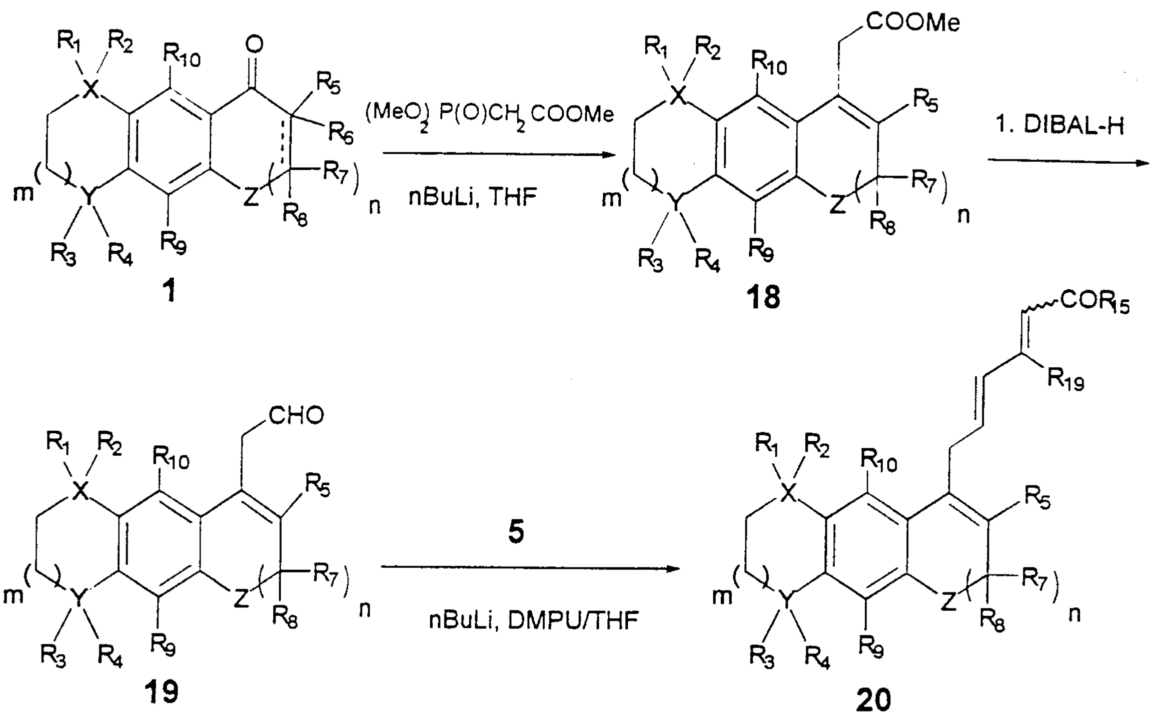


The tricyclic derivatives of general structure **20** can be prepared in accordance with reaction Scheme V. Tricyclic ketones **1** are condensed with the lithium salt of trimethyl phosphonoacetate in THF at elevated temperatures in a Horner-Wadsworth-Emmons olefination reaction to provide the β,γ-unsaturated esters **18**. The esters are reduced with one equivalent of DIBAL in methylene chloride at -78°C to provide aldehydes of general structure **19**. The aldehydes are converted into tricyclic derivatives **20** by condensation with the phosphonates **5** by the same processes as those employed in the process of Scheme I to produce tricycles of general structure **6** and **7**.

Scheme VI: Synthesis of Compounds of Structure (II):

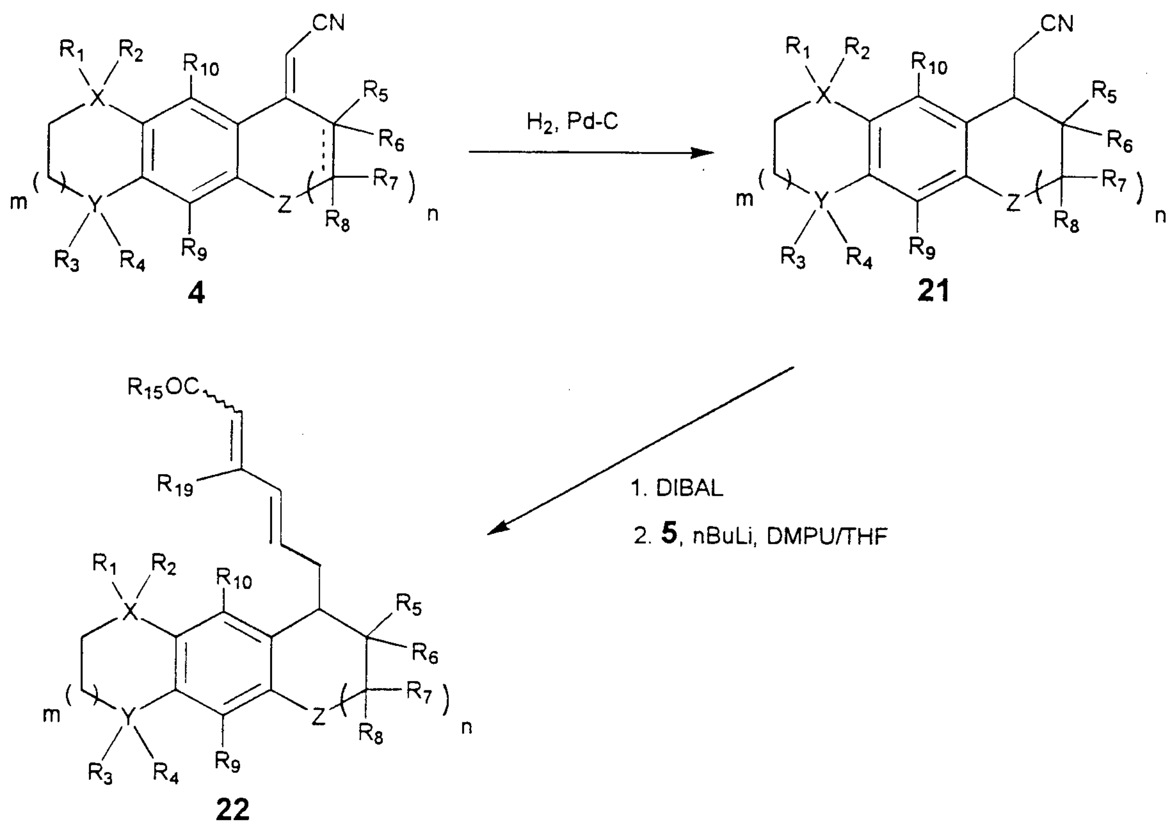


4

21

22

The tricyclic derivatives of structure **22** can be prepared in accordance with reaction Scheme VI. The previously described unsaturated nitriles **4** (see process for reaction Scheme I) are reduced by catalytic hydrogenation over 10% palladium-on-carbon in ethyl acetate to provide the saturated nitriles **21**. In accordance with the processes employed in reaction Scheme I, the nitriles can be transformed into the tricyclic derivatives of general structure **22**.

## Scheme VII: Synthesis of Compounds of Structure (II):

**Scheme VII.**

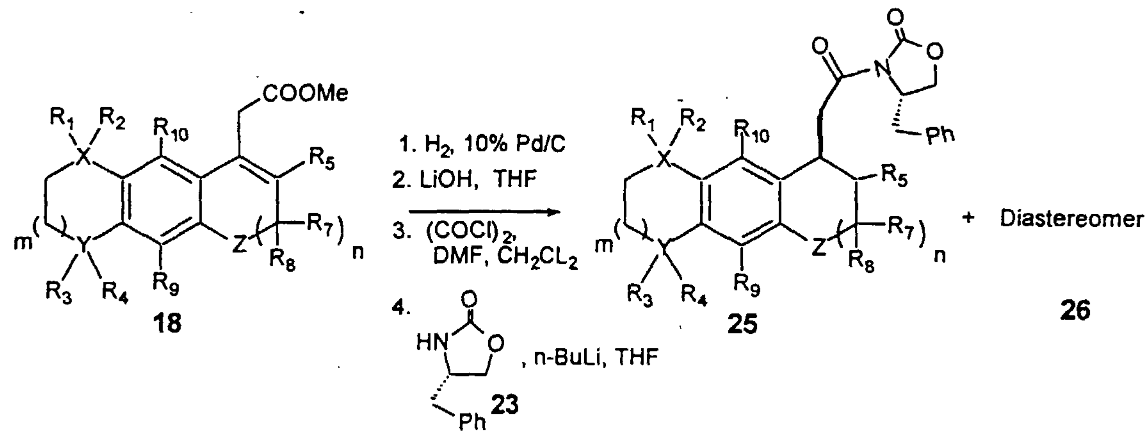


18 25 26 23

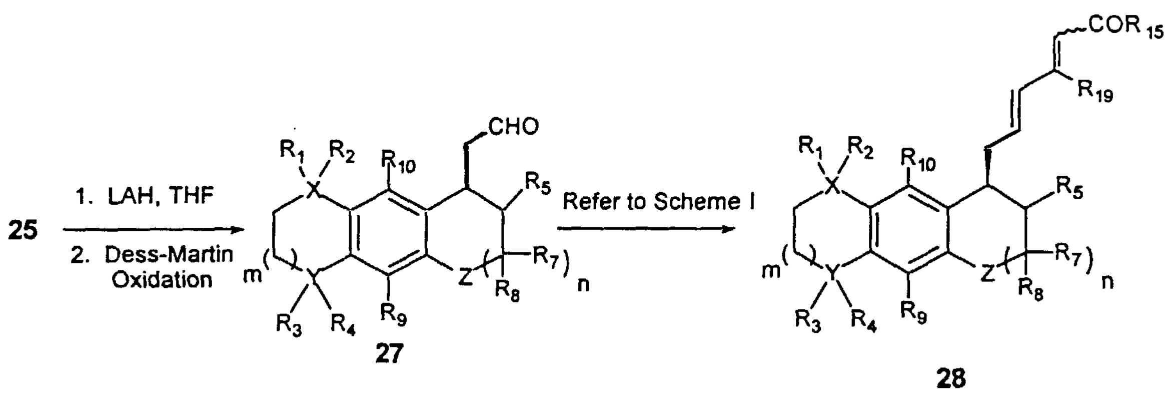


25 27 28

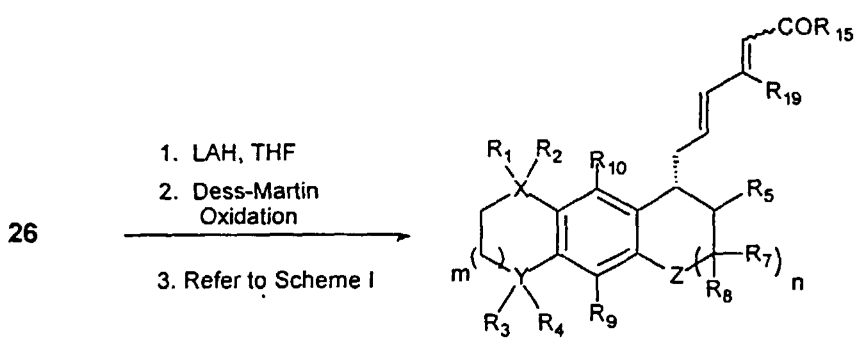


26 29

The chiral tricyclic derivatives of general structure **28** and **29** can be prepared in accordance with reaction Scheme VII. The previously described β,γ-unsaturated esters **18** (see reaction Scheme V) are reduced by catalytic hydrogenation over 10% palladium-on-carbon in ethyl acetate to provide the intermediate saturated esters which are converted to the diastereomeric acyl oxazolidinones of general structure **25** and **26** by coupling of the acid chloride derivative with (S)-4-benzyl-2-oxazolidinone. The mixture of amide diastereomers is separated by silica gel chromatography and each is separately reduced with LAH at reduced temperatures in THF, followed by Dess-Martin oxidation in methylene chloride at ambient temperature to provide the diastereomerically pure tricyclic aldehyde, for example tricyclic aldehydes of general structure **27**. In accordance with the processes employed in reaction Scheme I, the aldehydes are transformed into the tricyclic derivatives of general structure **28** and **29**.

## Scheme VIII: Synthesis of Compounds of Structure (II):

### Scheme VIII.

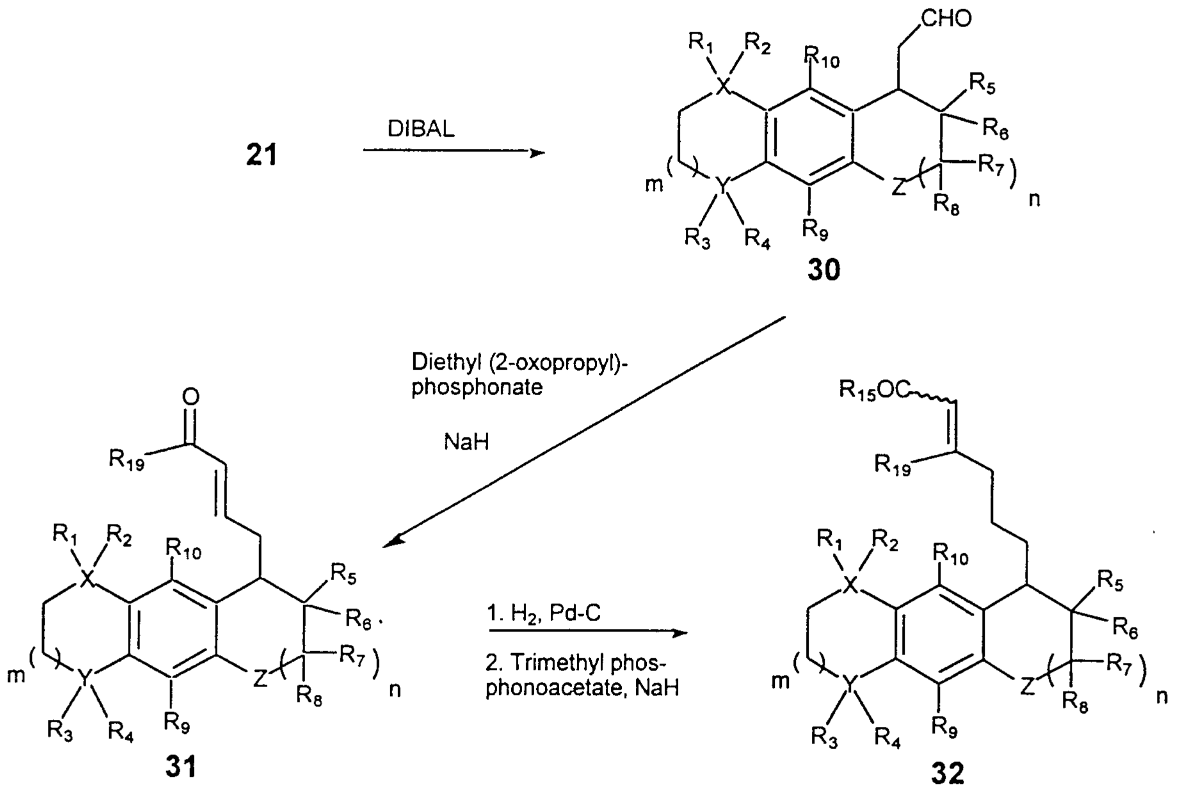

21 → 30 → 31 → 32

The tricyclic derivatives of general structure **32** can be prepared in accordance with reaction Scheme VIII. The previously described saturated nitriles **21** (see Scheme VI) are reduced with DIBAL in methylene chloride at -78°C to provide aldehydes of general structure **30**. The tricyclic aldehydes can be condensed with the sodium salt of diethyl (2-oxopropyl)-phosphonate in THF at reduced temperatures in a Horner-Wadsworth-Emmons olefination reaction to provide the $\alpha,\beta$-unsaturated ketones **31**. The enones **31** can be reduced by catalytic hydrogenation over 10% palladium-on-carbon in ethyl acetate to provide the interrnediate saturated ketones which are condensed with the sodium salt of trimethyl phosphonoacetate in THF at reduced temperatures in a Horner-Wadsworth-Emmons olefnation reaction to provide $\beta,\gamma$-unsaturated esters of general structure **32**. The acids and salts derived from **32** are readily obtainable from the corresponding esters by the same processes as those employed in the preparation process of Scheme I for tricycles of general structure **6** and **7**.

Scheme IX: Synthesis of Compounds of Structure (III):

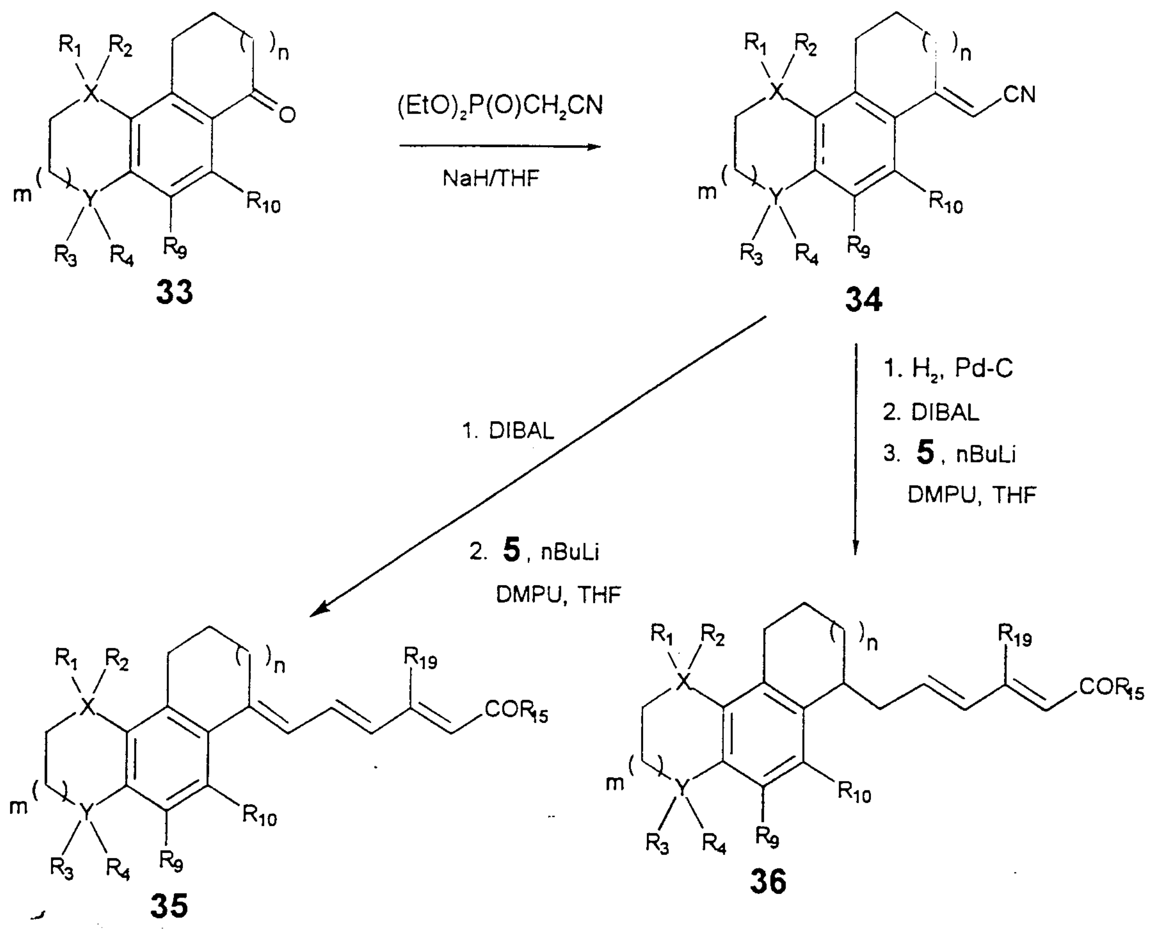


The tricyclic derivatives of general structures **35** and **36** can be prepared in accordance with reaction Scheme IX. The tricyclic trienes **35** can be prepared from the corresponding tricyclic ketones **33** in a manner analogous to that described in the preparation process of Scheme I for tricyclic derivatives of structures **6** and **7**. The tricyclic dienes of general structure **36** are prepared from the tricyclic ketones **33** in a manner analogous to that described in the preparation process of Scheme I and Scheme VI for tricyclic derivatives of general structure **22**.

Scheme X: Synthesis of Compounds of Structure (II):

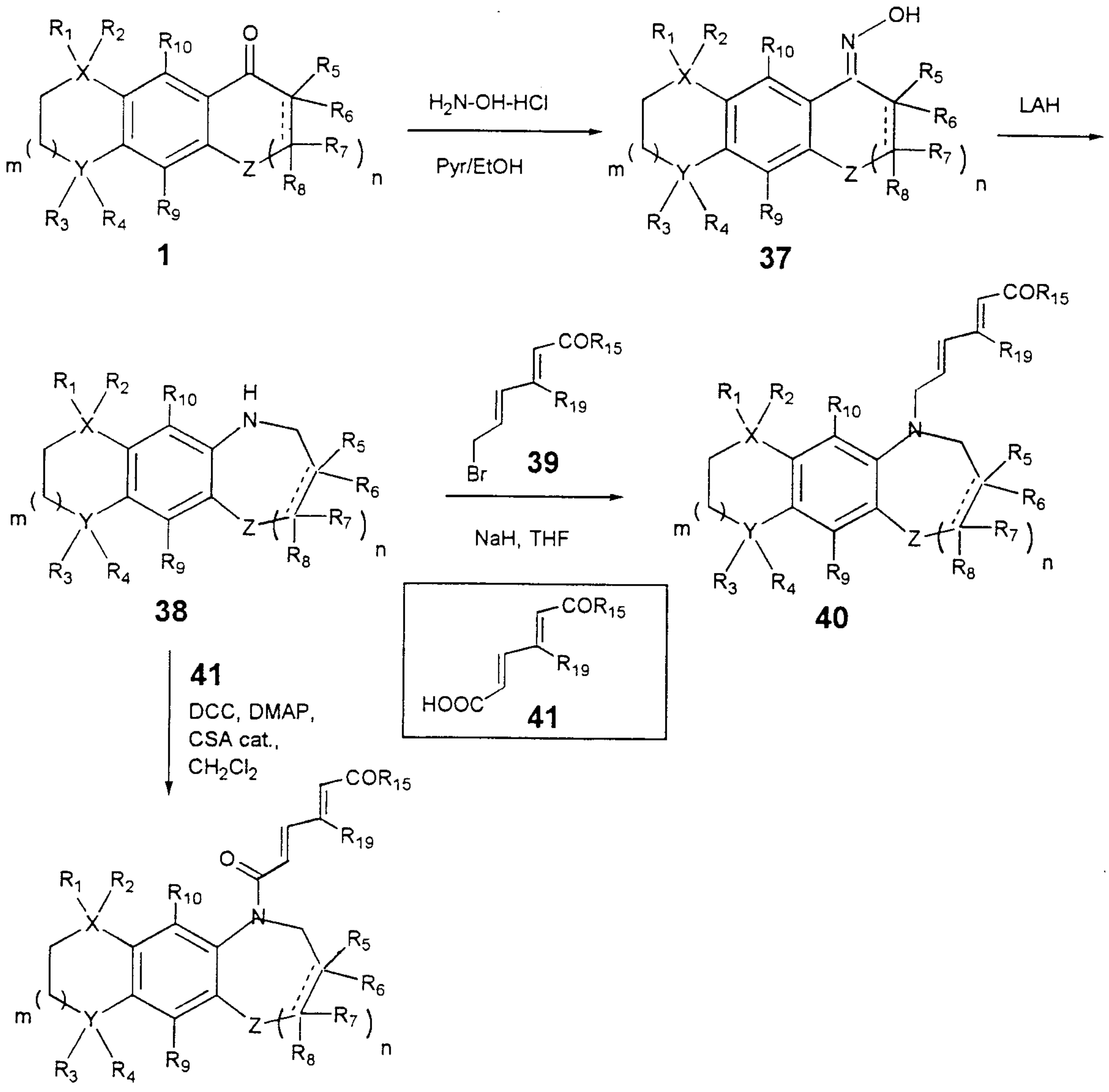


1, 37, 38, 39, 40, 41, 42

The tricyclic derivatives of general structures **40** and **42** can be prepared in accordance with reaction Scheme X. The previously described tricyclic ketones **1** (see preparation process for reaction Scheme I) are heated at reflux with hydroxylamine hydrochloride in pyridine and ethanol to provide oximes **37**. A Beckman rearrangement is effected with LAH in ethanol at 80°C to afford the corresponding tricyclic amines **38**, which are deprotonated with NaH at 0°C in THF and alkylated at ambient temperature with ethyl (2E,4E)-6-bromo-3-methylhexa-2,4-dienoate to give aminodienes of general structure **40**. Alternatively, the tricyclic amines **38** can be acylated by a DCC coupling in methylene chloride with (2E,4E)-3-methyl-5-carboxypenta-2,4-dienoate to give amides of general structure **42**. The acids and salts derived from the esters are readily obtainable by the same processes as those employed in the preparation process of Scheme I for tricycles of general structure **6** and **7**.

**Scheme XI: Synthesis of Compounds of Structure (II):**

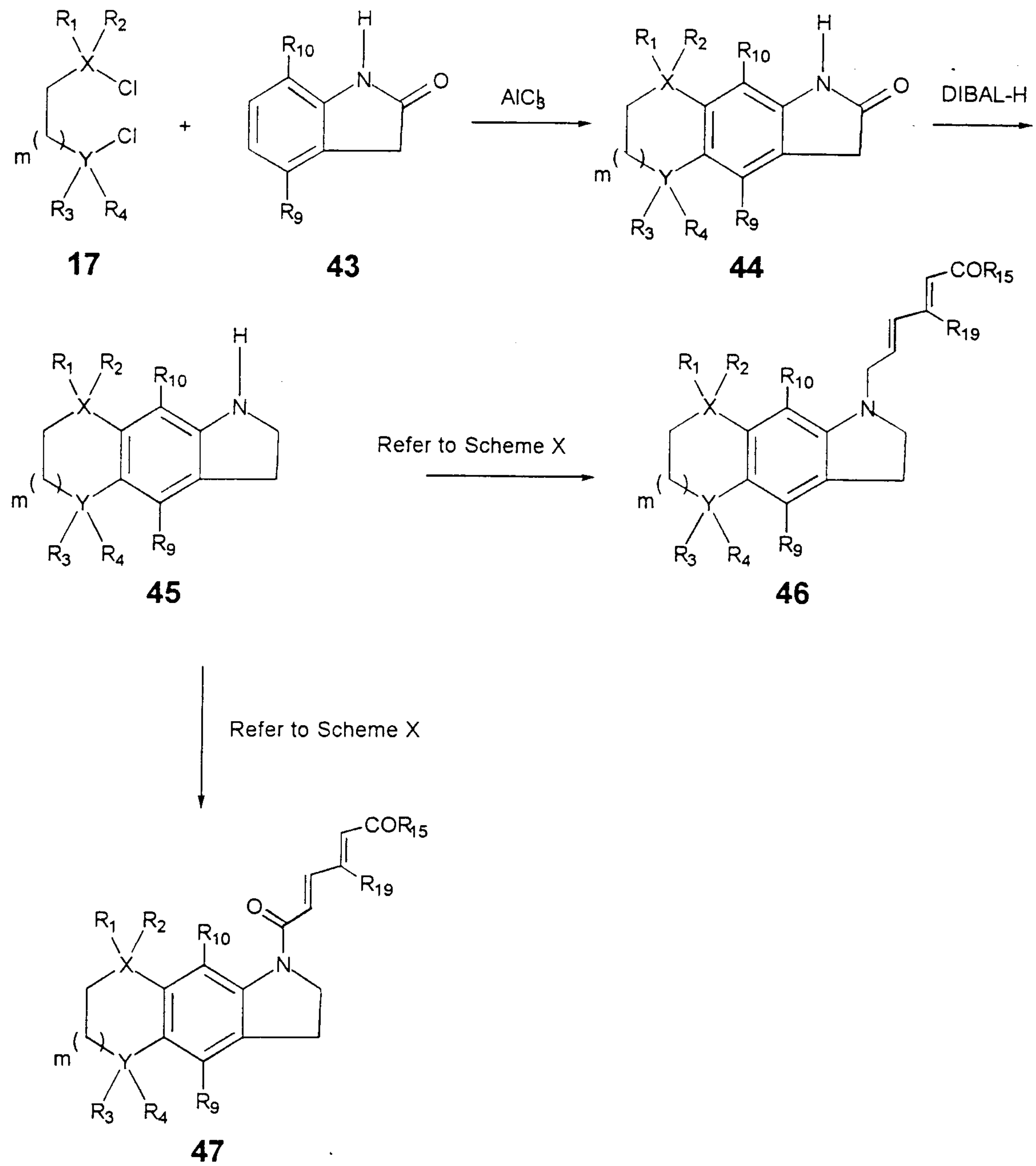


17 43 44 45 46 47

The tricyclic derivatives of general structure **46** and **47** can be prepared in accordance with reaction Scheme XI. The tricyclic amides **44** can be prepared from oxindole of general structure **43** and 2,5-dichloro-2,5-dialkylhexanes of general structure **17** by aluminum trichloride catalyzed Friedel-Crafts alkylation/cyclization in dichloromethane at ambient temperature. Amides of general structure **44** can be reduced with DIBAL in methylene chloride at 25°C to provide the corresponding amines **45**. The amine dienes **46** can be prepared from the amines **45** in a manner analogous to that described in the preparation process of Scheme X for tricyclic derivatives of general structure **40**. The desired tricyclic amides **47** are prepared from the corresponding tricyclic amines of **45** in a manner analogous to that described in the preparation process of Scheme X for tricyclic derivatives of general structure **42**. The acids and salts derived from **46** and **47** are readily obtainable from the corresponding esters by the same processes as those employed in the

Scheme I for tricycles of structures **6** and **7**.

## Scheme XII: Synthesis of Compounds of Structure (III):

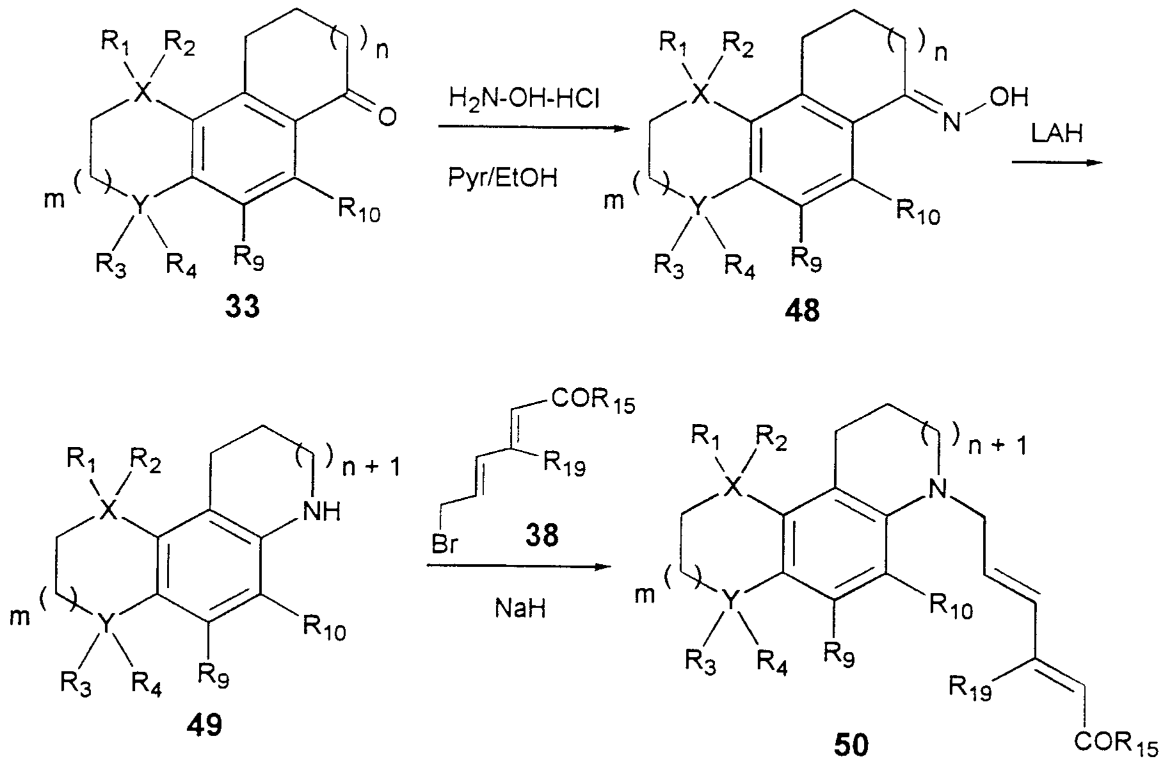


33 48 49 38 50

The tricyclic derivatives of general structure **50** can be prepared in accordance with reaction Scheme XII. The tricyclic ketones **33** are transformed to amines of general structure **49** via Beckman type rearrangement of the intermediate oximes **48**. Alkylation of the derived amines **49** with suitable alkyl halides of the type **38** provide the desired tricyclic derivatives of general structure **50**. The acids and salts derived from **50** are readily obtainreadily obtainable from the corresponding esters by the same processes as those employed in Scheme I for tricycles of structures **6** and **7**.

## Scheme XIII: Synthesis of Compounds of Structure (III):

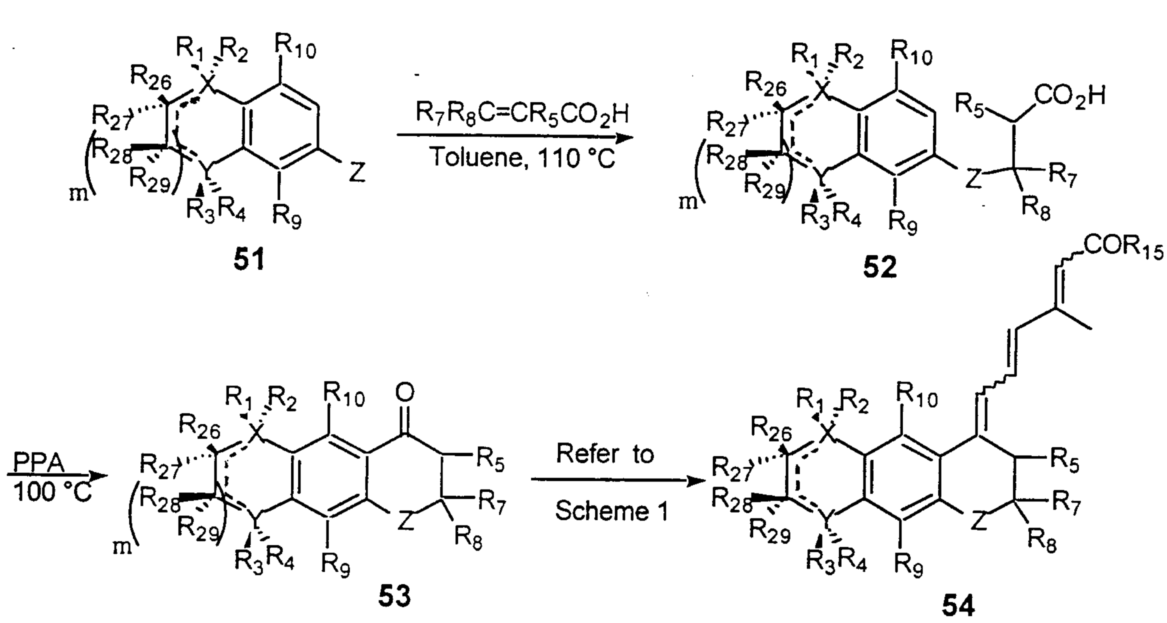


An alternative means for making the intermediate tricyclic derivatives of general structure **54** is in accordance with reaction Scheme XIII. The reviously described tricyclic ketones **1** (see Example 1) can be prepared in an alternative fashion, starting from the tetrahydronaphthylene **51**. A Michael addition is effected by the addition of an acyrylic acid (which can be trisubstituted) to the tetrahydronaphthylene **52** in toluene and heating the soution to 110 °C for 8-16 hours. The tricyclic ketones **53** are formed by intramolecular acylation in polyphosphoric acid (PPA) at 100 °C for 8-14 hours. The ketones are converted into tricyclic derivatives **54** by condensation with the phosphonate **5** by the same processes as those employed in the process of Scheme I.

Scheme XIV: Synthesis of Compounds of Structure (#):

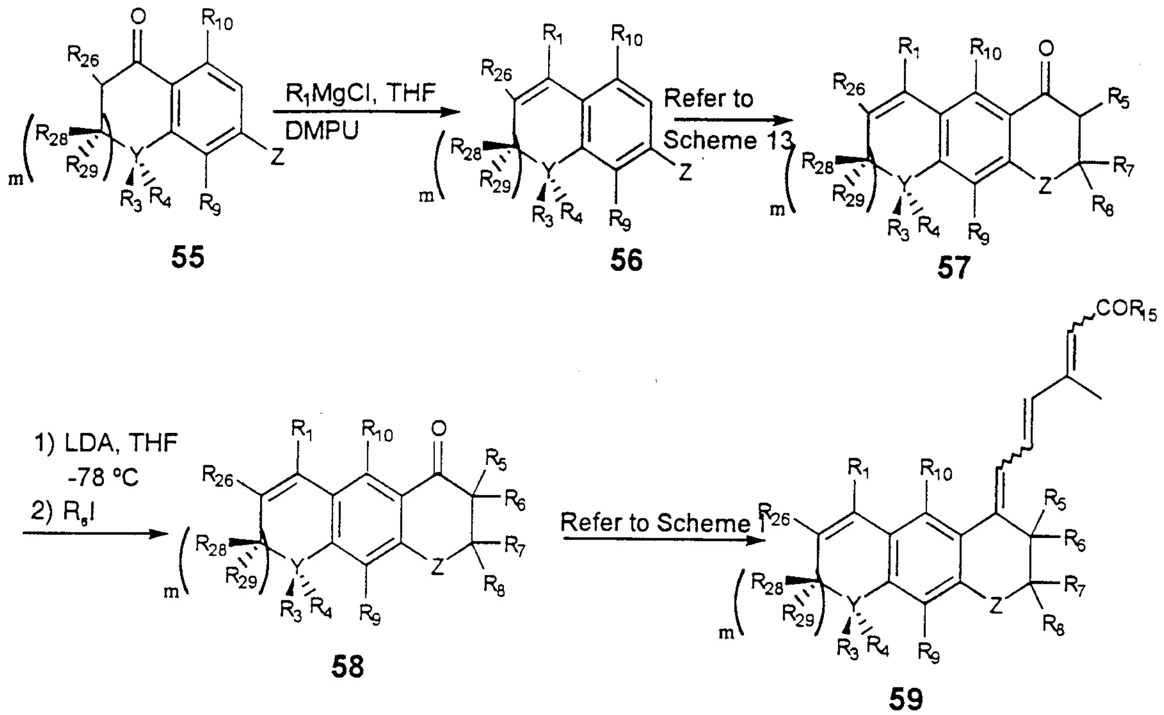


55 56 57 58 59

The tricyclic derivatives of general structure **59** can be prepared in accordance with reaction Scheme XIV. Tricyclic ketones are condensed with Grignard reagents in THF or diethyl ether at -40 °C, in the presence of a co-solvent such as DMPU. Michael addition, followed by intramolecular cyclization in the presence of PPA, as in Scheme XIII, allows for the preparation of intermediate ketones such as **57**. The resulting ketone can be alkylated with a varitety of electrophiles, in particular alkyl halides not restricted to methyl iodide. The ketones are converted into tricyclic derivatives **59** by condensation with the phosphonate **5** by the same proceses as those employed in the processs of Scheme I.

## Scheme XV: Synthesis of Compounds of Structure (V):

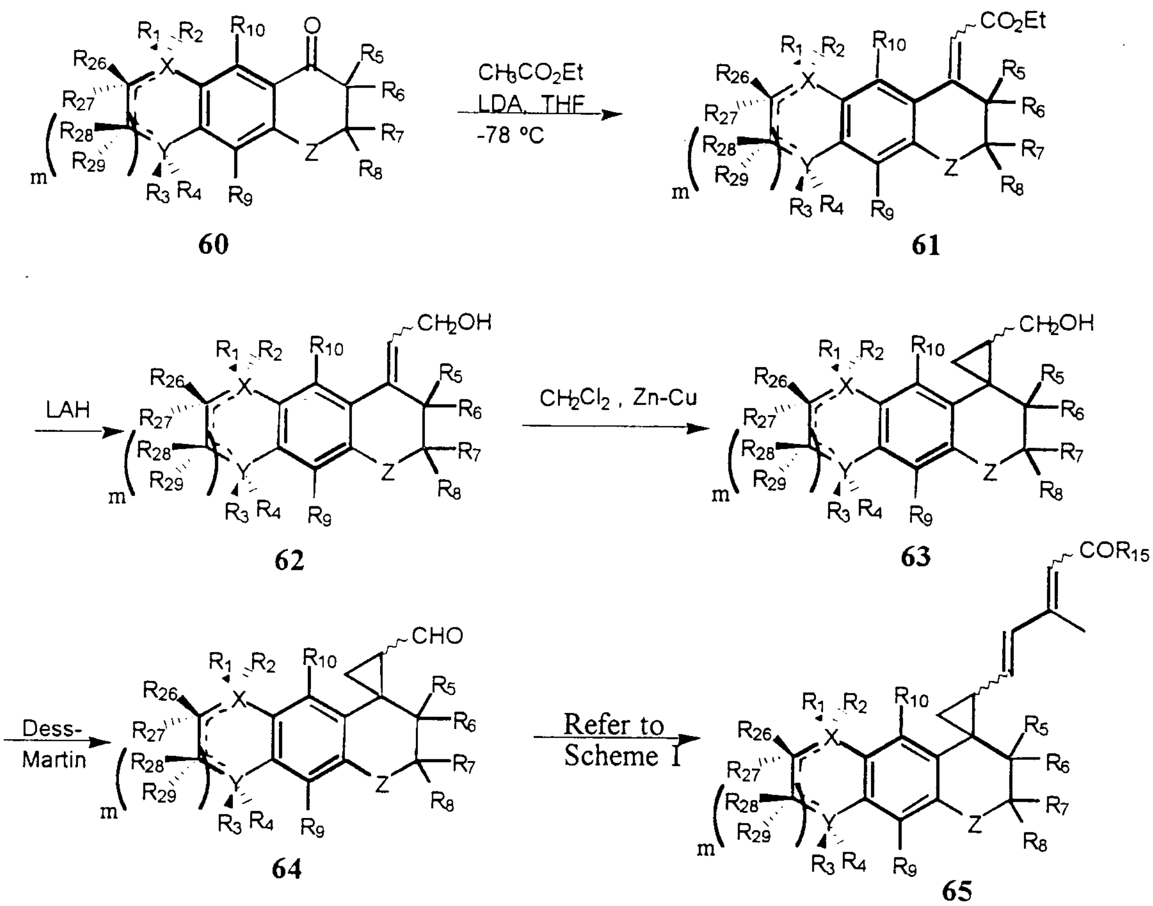


60 61 62 63 64 65

The tricyclic derivatives of general structure **65** can be preapred in accordance with reaction Scheme XV. The tricycli ketones **60** can be transformed into the esters of general structure **61** by low temperature lithio enolate chemistry or a Reformatsky reaction procedure. Reduction of the ester to the alcohol **62** which facilitates the Simmons-Smith reaction. The Simmons-Smith reaction gives the spiro-cyclic cyclopropyl compound of general strucuture **63** Oxidation of **63** followed by the same processes as those employed in Scheme I prepare the acids and salts of general structure **65**.

Scheme XVI: Synthesis of Compounds of Structure (II):

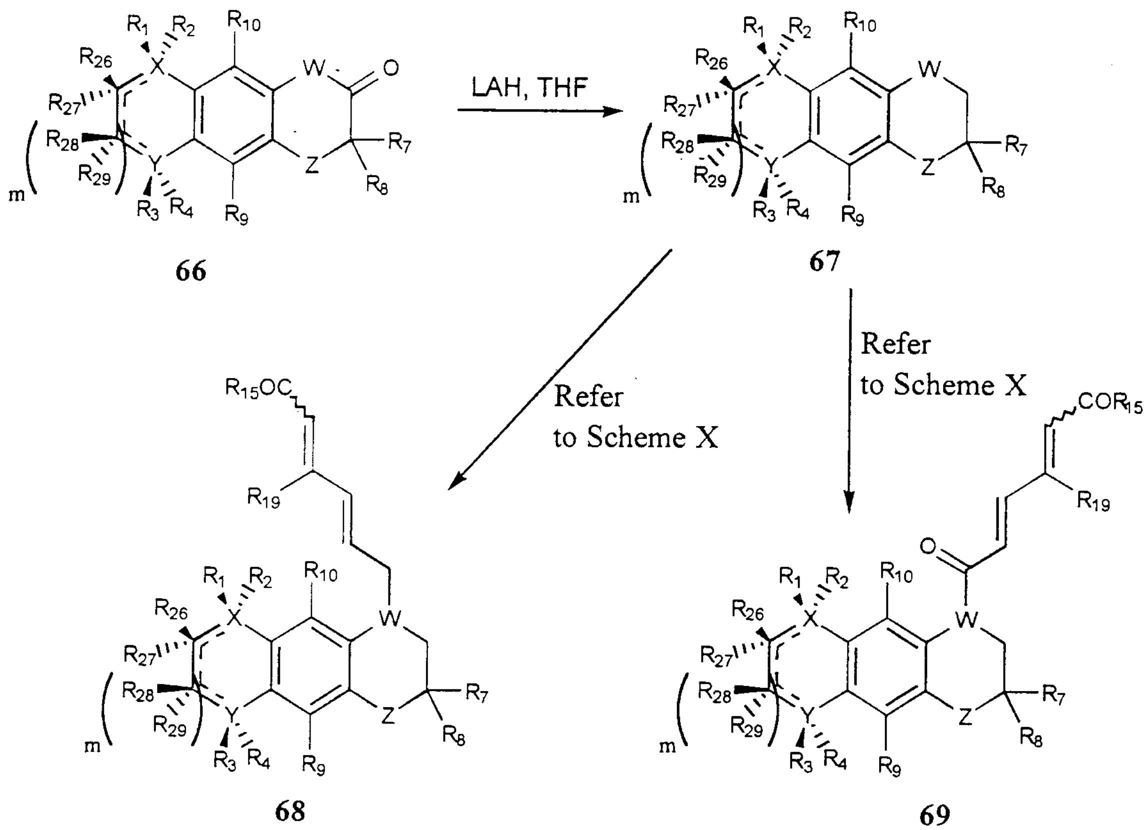


66

67

68

69

The tricyclic derivatives of general structure **68** or **69** can be prepared in accordance with reaction Scheme XVI The tricyclic cyclic-carboxylic derivatives (esters or amides) can be reduced with lithium aluminum hydride in THF to give the heterocycles of general structure **67**. The acids and salts derived from **68** or **69** are readily obtained from the corresponding esters by the same processses ad tose employed in Scheme X.

## Scheme XVII: Synthesis of Compounds of Structure (III):

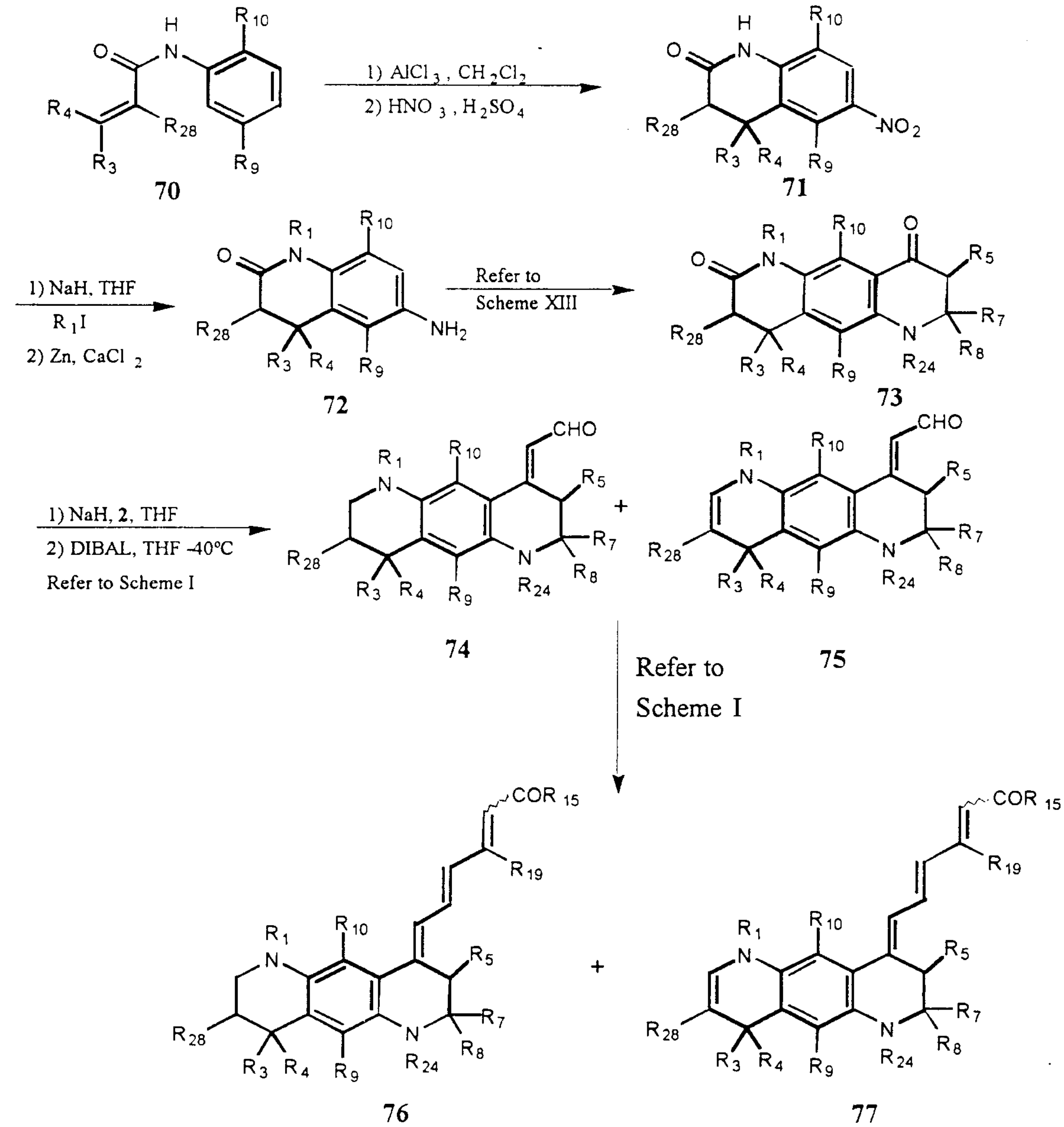


70 71 72 73 74 75 76 77

The tricyclic derivatives of general structure **76** or **77** can be prepared in accordance with reaction Scheme XVII. The acrylic acid derivative **70** can be cyclized with $AlCl_3$ in dichloromethane or other nonpolar solvent to give the bicylic compound **71**. If X=N then the amide can be alkylated to add the R1 group function using sodium hydride in THF and a alkyl halide electrophhile. The nitro group can be reduced to the amine using zinc and calcium chloride monohydrate. Followin the building of the tricylic compound **73** as shown in Scheme XIII The ketone **73** is converted to the nitrile as in Scheme I and reduction with DIBAL gives the aldehyde and amine **74**. A intermidate of the amide reduction process is the enamine compound of general strucuture **75** which is stable and can be isoloted. The mixture of products can be treated with the phosphonate reagent in Scheme I and following the procedures given there the acids and salts of general structure **76** or **77** can be isolated.

Scheme XVIII: Synthesis of Compounds of Structure (III):

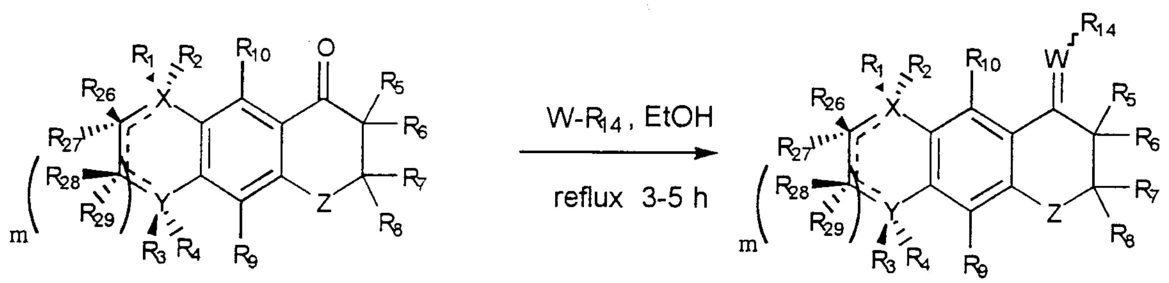


60

78

The tricyclic derivatives of general structure **78** can be preapred in acccordance with reaction Scheme XVIII. The tricyclic derivatives of general structure **60** can be reacted with various nucleophiles, in particular, but not limited to amine, hydroxylamine or hyrazine, which following dehydration give compounds of the general structure **78**.

It will be understood by those skilled in the art that certain modifications can be made to the above-described methods that remain within the scope of the present invention. For example, the compounds of the present invention may also be produced in the form of the corresponding amides or esters, or pharmaceutically acceptable salts.

The structural formulas for Compounds 101a and 101b through 144a and 144b of the present invention are given on the following pages.

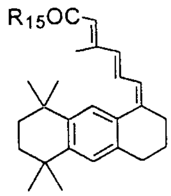

**101a**: $R_{15}$ = ethoxy
**101b**: $R_{15}$ = OH

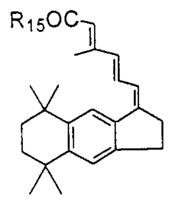

**102a**: $R_{15}$ = ethoxy
**102b**: $R_{15}$ = OH

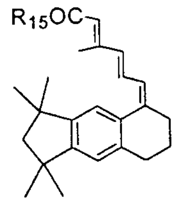

**103a**: $R_{15}$ = ethoxy
**103b**: $R_{15}$ = OH

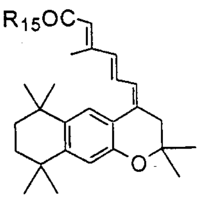

**104a**: $R_{15}$ = ethoxy
**104b**: $R_{15}$ = OH

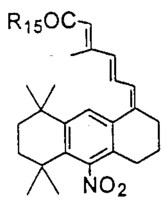

**105a**: $R_{15}$ = ethoxy
**105b**: $R_{15}$ = OH

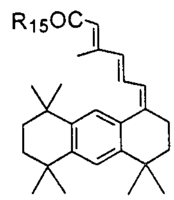

**106a**: $R_{15}$ = ethoxy
**106b**: $R_{15}$ = OH

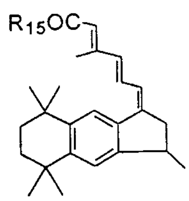

**107a**: $R_{15}$ = ethoxy
**107b**: $R_{15}$ = OH

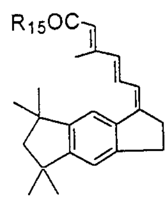

**108a**: $R_{15}$ = ethoxy
**108b**: $R_{15}$ = OH

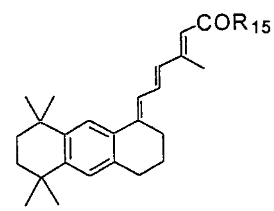

**109a**: $R_{15}$ = ethoxy
**109b**: $R_{15}$ = OH

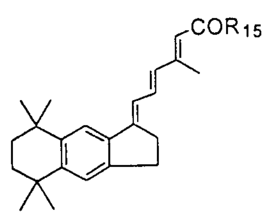

**110a**: $R_{15}$ = ethoxy
**110b**: $R_{15}$ = OH

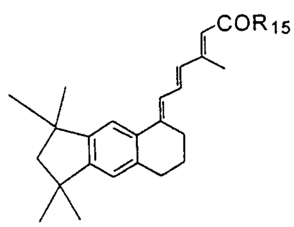

**111a**: $R_{15}$ = ethoxy
**111b**: $R_{15}$ = OH

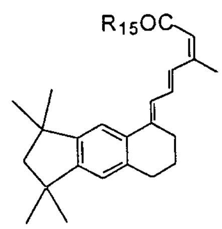

**112a**: $R_{15}$ = ethoxy
**112b**: $R_{15}$ = OH

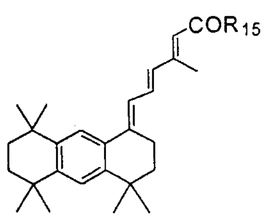

**113a**: $R_{15}$ = ethoxy
**113b**: $R_{15}$ = OH

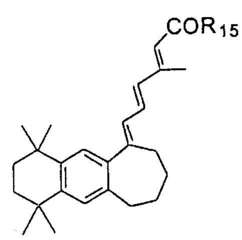

**114a**: $R_{15}$ = ethoxy
**114b**: $R_{15}$ = OH

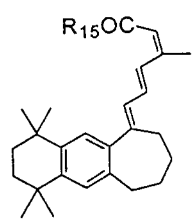

**115a**: $R_{15}$ = ethoxy
**115b**: $R_{15}$ = OH

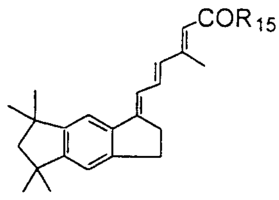

**116a:** $R_{15}$ = ethoxy
**116b:** $R_{15}$ = OH

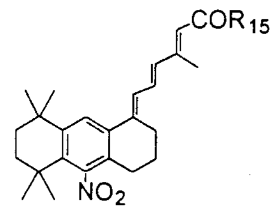

**117a:** $R_{15}$ = ethoxy
**117b:** $R_{15}$ = OH

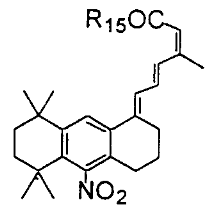

**118a:** $R_{15}$ = ethoxy
**118b:** $R_{15}$ = OH

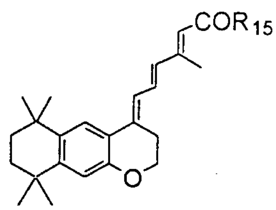

**119a:** $R_{15}$ = ethoxy
**119b:** $R_{15}$ = OH

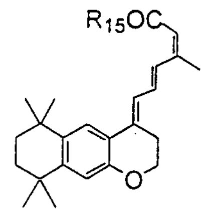

**120a:** $R_{15}$ = ethoxy
**120b:** $R_{15}$ = OH

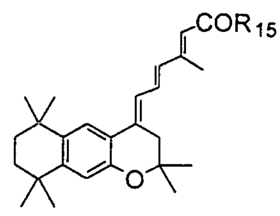

**121a:** $R_{15}$ = ethoxy
**121b:** $R_{15}$ = OH

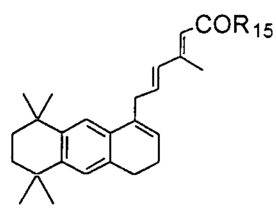

**122a:** $R_{15}$ = ethoxy
**122b:** $R_{15}$ = OH

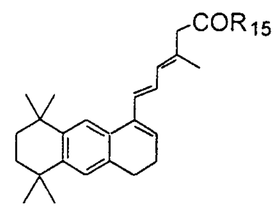

**123a:** $R_{15}$ = ethoxy
**123b:** $R_{15}$ = OH

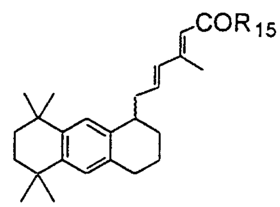

**124a:** $R_{15}$ = ethoxy
**124b:** $R_{15}$ = OH

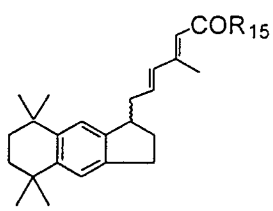

**125a:** $R_{15}$ = ethoxy
**125b:** $R_{15}$ = OH

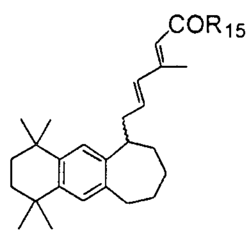

**126a:** $R_{15}$ = ethoxy
**126b:** $R_{15}$ = OH

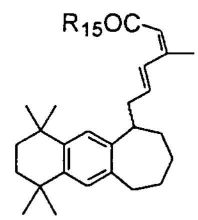

**127a:** $R_{15}$ = ethoxy
**127b:** $R_{15}$ = OH

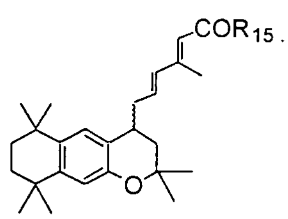

**128a:** $R_{15}$ = ethoxy
**128b:** $R_{15}$ = OH

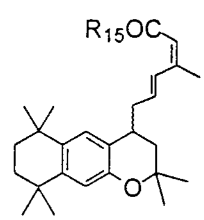

**129a:** $R_{15}$ = ethoxy
**129b:** $R_{15}$ = OH

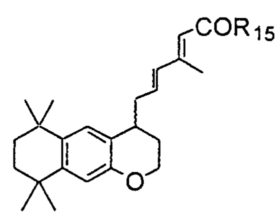

**130a:** $R_{15}$ = ethoxy
**130b:** $R_{15}$ = OH

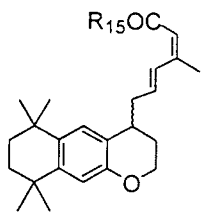

**131a:** $R_{15}$ = ethoxy
**131b:** $R_{15}$ = OH

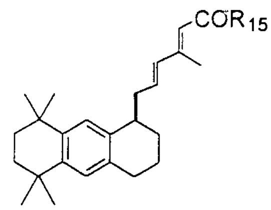

**132a:** $R_{15}$ = ethoxy
**132b:** $R_{15}$ = OH

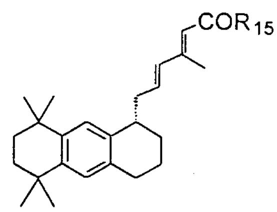

**133a:** $R_{15}$ = ethoxy
**133b:** $R_{15}$ = OH

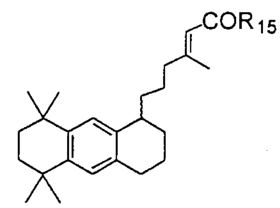

**134a:** $R_{15}$ = ethoxy
**134b:** $R_{15}$ = OH

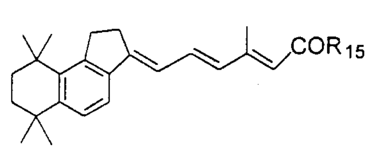

**135a:** $R_{15}$ = ethoxy
**135b:** $R_{15}$ = OH

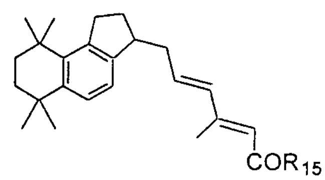

**136a:** $R_{15}$ = ethoxy
**136b:** $R_{15}$ = OH

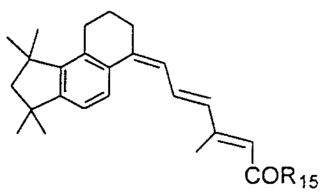

**137a:** $R_{15}$ = ethoxy
**137b:** $R_{15}$ = OH

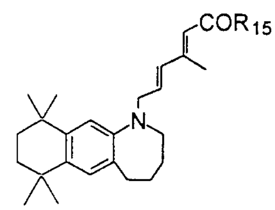

**138a:** $R_{15}$ = ethoxy
**138b:** $R_{15}$ = OH

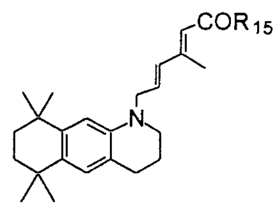

**139a:** $R_{15}$ = ethoxy
**139b:** $R_{15}$ = OH

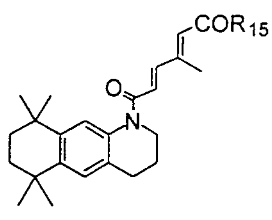

**140a:** $R_{15}$ = ethoxy
**140b:** $R_{15}$ = OH

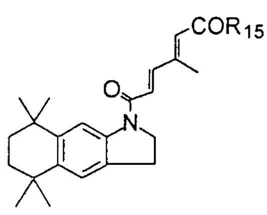

**141a:** $R_{15}$ = ethoxy
**141b:** $R_{15}$ = OH

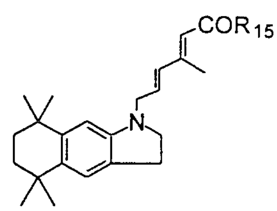

**142a:** $R_{15}$ = ethoxy
**142b:** $R_{15}$ = OH

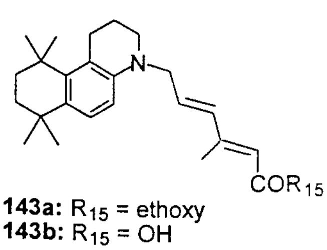

**143a:** $R_{15}$ = ethoxy
**143b:** $R_{15}$ = OH

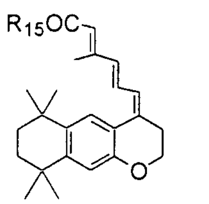

**144a:** $R_{15}$ = ethoxy
**144b:** $R_{15}$ = OH

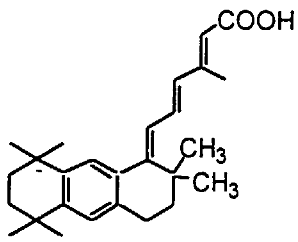

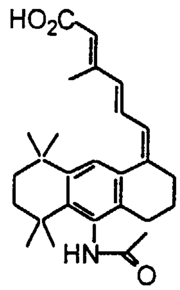

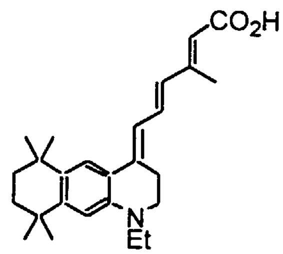

**145a:** $R_{15}$ = ethoxy
**145b:** $R_{15}$ = OH

**146a:** $R_{15}$ = ethoxy
**146b:** $R_{15}$ = OH

**147a:** $R_{15}$ = ethoxy
**147b:** $R_{15}$ = OH

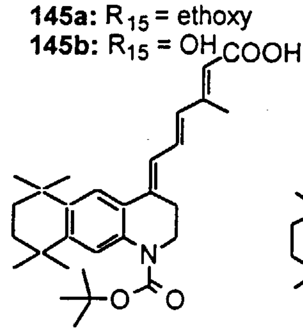

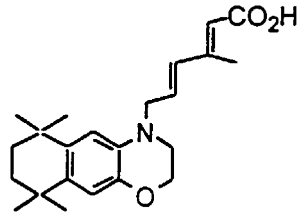

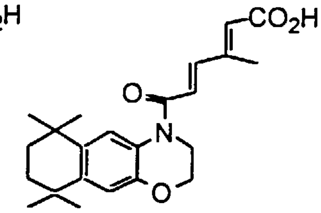

**148a:** $R_{15}$ = ethoxy
**148b:** $R_{15}$ = OH

**149a:** $R_{15}$ = ethoxy
**149b:** $R_{15}$ = OH

**150a:** $R_{15}$ = ethoxy
**150b:** $R_{15}$ = OH

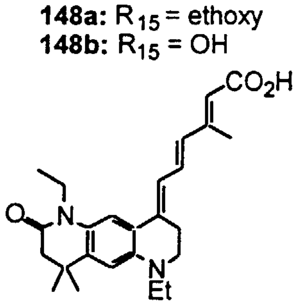

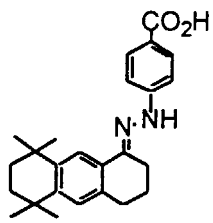

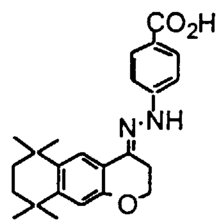

**151a:** $R_{15}$ = ethoxy
**151b:** $R_{15}$ = OH

**152a:** $R_{15}$ = ethoxy
**152b:** $R_{15}$ = OH

**153a:** $R_{15}$ = ethoxy
**153b:** $R_{15}$ = OH

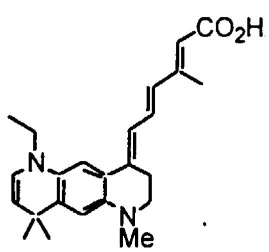

**154a:** $R_{15}$ = ethoxy
**154b:** $R_{15}$ = OH

In another aspect, the retinoid compounds of the present invention are combined in a mixture with a pharmaceutically acceptable carrier to provide pharmaceutical compositions useful for treating the biological conditions or disorders noted herein in mammalian, and more preferably, in human patients. The particular carrier employed in these pharmaceutical compositions may take a wide variety of forms depending upon the type of administration desired, e.g., intravenous, oral, topical, suppository or parenteral.

In preparing the compositions in oral liquid dosage forms (e.g., suspensions, elixirs and solutions), typical pharmaceutical media, such as water, glycols, oils, alcohols, flavoring agents, preservatives, coloring agents and the like can be employed. Similarly, when preparing oral solid dosage forms (e.g., powders, tablets and capsules), carriers

such as starches, sugars, diluents, granulating agents, lubricants, binders, disintegrating agents and the like will be employed. Due to their ease of administration, tablets and capsules represent the most advantageous oral dosage form for the pharmaceutical compositions of the present invention.

For parenteral administration, the carrier will typically comprise sterile water, although other ingredients that aid in solubility or serve as preservatives, may also be included. Furthermore, injectable suspensions may also be prepared, in which case appropriate liquid carriers, suspending agents and the like will be employed.

For topical administration, the compounds of the present invention may be formulated using bland, moisturizing bases, such as ointments or creams. Examples of suitable ointment bases are petrolatum, petrolatum plus volatile silicones, lanolin, and water in oil emulsions such as Eucerin™ (Beiersdorf). Examples of suitable cream bases are Nivea™ Cream (Beiersdorf), cold cream (USP), Purpose Cream™ (Johnson & Johnson) hydrophilic ointment (USP), and Lubriderm™ (Warner-Lambert).

The pharmaceutical compositions and compounds of the present invention will generally be administered in the form of a dosage unit (e.g., tablet, capsule etc.) at from about 1 μg/kg of body weight to about 500 mg/kg of body weight, more preferably from about 10 μg/kg to about 250 mg/kg, and most preferably from about 20 μg/kg to about 100 mg/kg. As recognized by those skilled in the art, the particular quantity of pharmaceutical composition according to the present invention administered to a patient will depend upon a number of factors, including, without limitation, the biological activity desired, the condition of the patient, and tolerance for the drug.

The compounds of this invention also have utility when labeled and used in assays to determine the presence of RARs and RXRs. They are particularly useful due to their ability to selectively bind to members of the RAR and RXR subfamilies and can therefore be used to determine the presence of RAR and RXR isoforms in the presence of other retinoid receptors or related intracellular receptors.

Due to the selective specificity of the compounds of this invention for retinoid receptors, these compounds can also be used to purify samples of RARs and RXRs *in vitro*. Such purification can be carried out by mixing samples containing retinoid receptors with one of more of the compounds of the present invention so that the compound (ligand) binds to the receptor, and then separating out the bound ligand/receptor combination by separation techniques which are known to those of skill in the art. These techniques include column separation, filtration, centrifugation, tagging and physical separation, and antibody complexing, among others.

The compounds of the present invention also include racemate, individual stereoisomers and mixtures thereof. These isomers are then isolated by standard resolution techniques, including fractional crystallization and reverse phase and chiral column chromatography.

The compounds and pharmaceutical compositions of the present invention can advantageously be used in the treatment of the diseases and conditions described herein. In this regard, the compounds and compositions will prove particularly useful in the treatment of skin-related diseases and conditions, such as acne, psoriasis, and photo damage, cancerous and precancerous conditions, diseases of the eye, cardiovascular diseases, inflammatory and neurodegenerative diseases, diseases associated with human papilloma virus, improper pituitary function, modulation of apoptosis, diseases of the immune system, wound healing and restoration of hair growth.

Furthermore, the compounds and pharmaceutical compositions of the present invention possess a number of advantages over previously identified retinoid compounds. For example, the compounds are extremely potent activators of RARs and RXRs, preferably displaying 50% maximal activation of one or more of the retinoid receptors at a concentration of less than 100 nM, more preferably at a concentration of less than 50 nM, more preferably yet at a concentration ofless than 20 nM, and most preferably at a concentration of less than 10 nM. Also, the RAR and RXR selective compounds of the present invention preferentially activate one subfamily of retinoid receptors at a level at least 2 times greater, preferably at least 5 times greater, more preferably at least 10 times greater, and most preferably at least 100 times greater than the other subfamily of retinoid receptors. In addition, the compounds of the present invention also are easier to synthesize, provide greater stability and bioavailability, and appear to be less teratogenic in comparison to all-*trans* retinoic acid and 9-*cis* retinoic acid, known RAR and RXR active compounds, respectively.

The invention will be further illustrated by reference to the following non-limiting Examples.

## EXAMPLE 1

Preparation of compound **101a** according to Scheme I

**Ethyl (2E,4E)-3-methyl-6-[(Z)-3,4,5,6,7,8-hexahydro-5,5,8,8-tetramethyl-2H-anthracen-1-ylidene]hexa-2,4-dienoate** (structure **6**, where $R_1$, $R_2$, $R_3$, $R_4$, and $R_{19}$ are methyl, $R_5$, $R_6$, $R_7$, $R_8$, $R_9$, and $R_{10}$ are hydrogen, $R_{15}$ is ethoxy, X, Y and Z are carbon, m = n = 1)

To a solution of 1,2,3,4,6,7,8,9-octahydro-6,6,9,9-tetramethylanthracene (2.0 g, 8.3 mmol) [prepared by Friedel-Crafts alkylation/annulation of 1,2,3,4-tetrahydronaphthalene with 2,5-dichloro-2,3-dimethylhexane in the presence of aluminum trichioride at 0°C in dichloromethane] in $CH_2Cl_2$ (100 ml) and pyridine (15 ml) at 0°C was added $CrO_3$ (8.26 g, 82.6 mmol) in several portions. The reaction mixture was stirred at 0°C for 30 min, then allowed to warm up to room temperature and stirred for 10 h. The reaction mixture was poured over an ice-acid mixture (1N HCl, 100ml), extracted with $Et_2O$ (200 ml), dried ($MgSO_4$), concentrated, and purified by column chromatography (25% ether in hexane) to give 3,4,5,6,7,8-hexahydro-5,5,8,8-tetramethyl-2H-anthracen-1-one (740 mg, 35%): $^1H$ NMR(400 MHz, $CDCl_3$) $\delta$ 8.01(s, 1H, ArH), 7.17(s, 1H, ArH), 2.90(t, J=6.5 Hz, 2H, CH2, benzylic), 2.60(t, J=6.3 Hz, 2H, $CH_2$), 2.10(m, 2H, $CH_2$), 1.68(s, 4H, $2CH_2$), 1.30(s, 6H, $2CH_3$), 1.29(s, 6H, $2CH_3$).

To a solution of diethyl cyanomethylphosphonate (1.8 g, 10.4 mmol) in THF (4 ml) and DMPU (4 ml) at 0°C was added NaH (375 mg, 65% in oil, 10.4 mmol) in one portion. The resulting solution was warmed to room temperature for 30 min. To this solution was added 3,4,5,6,7,8-hexahydro-5,5,8,8-tetramethyl-2H-anthracen-1-one (1.08 g, 4.2 mmol) in THF (2 ml). The mixture was then refluxed at 80°C for 3 hr, cooled, quenched with saturated $NH_4Cl$ (20 ml), extracted with $Et_2O$ (100 ml), dried ($MgSO_4$), concentrated, and purified by column chromatography (10% ether in hexane) to afford *cis*-(1,2,3,4,6,7,8,9-octahydro-6,6,9,9-tetramethyl-2H-anthracen-1-ylidene)ethanitrile (100 mg, 10%) and *trans*-(1,2,3,4,6,7,8,9-octahydro-6,6,9,9-tetramethyl-2H-anthracen-1-ylidene)ethanitrile (660 mg, 66%). The *cis*-nitrile (structure **3**) had $^1H$ NMR(400 MHz, $CDCl_3$) $\delta$ 8.31(s, 1H, ArH), 7.09(s, 1H, ArH), 5.19(s, 1H, olefinic), 2.80(t, J=6.5 Hz, 2H, $CH_2$, benzylic), 2.55(t, J=6.3 Hz, 2H, $CH_2$), 1.90(m, 2H, $CH_2$), 1.64(s, 4H, $2CH_2$), 1.26(s, 6H, $2CH_3$), 1.25(s, 6H, $2CH_3$). The *trans*-nitrile (structure **4**) had $^1H$ NMR(400 MHz, $CDCl_3$) $\delta$ 7.46(s, 1H, ArH), 7.09(s, 1H, ArH), 5.68(s, 1H, CH, olefinic), 2.80(m, 4H, $2CH_2$), 1.90(m, 2H, $CH_2$), 1.65(s, 4H, $2CH_2$), 1.26(s, 6H, $2CH_3$), 1.25(s, 6H, $2CH_3$).

To a solution of the above Z-(3,4,5,6,7,8-hexahydro-5,5,8,8-tetramethyl-2H-anthracen-1-ylidene)ethanitrile (56 mg, 0.2 mmol) in $CH_2Cl_2$ (2 ml) at -78°C was added DIBAL (0.4 ml, 1M in $CH_2Cl_2$, 0.4 mmol). The mixture was stirred at that temperature for 10 min, then quenched with saturated potassium sodium tartrate (10 ml) at -78°C, warmed to room temperature, extracted with EtOAc (50 ml), dried ($MgSO_4$), and concentrated to give essentially pure aldehyde, which was employed for the next reaction without further purification.

A solution of diethyl 3-ethoxycarbonyl-2-methylprop-2-enylphosphonate (86 mg, 0.33 mmol) in THF (1 ml) and DMPU (1ml) at 0°C was treated with nBuLi (0.13 ml, 2.5 M in hexane, 0.33 mM), then warmed to room temperature for 30 min The solution was cooled to -78°C, and the above aldehyde (32 mg, 0.11 mmol) in THF (1 ml) was slowly added. Subsequently, the reaction mixture was allowed to warm to room temperature for 30 min, quenched with a saturated solution of $NH_4Cl$ (5 ml), extracted with $Et_2O$ (50 ml), dried ($MgSO_4$), concentrated, and purified by column chromatography (10% ether in hexane) to give the title ester (**101a**) (39 mg, 85%): Rf= 0.48(10 % ether in hexane); $^1H$ NMR(400 MHz, $CDCl_3$) $\delta$ 7.33(s, 1H, ArH), 7.19(dd, J=15.2, 11.2 Hz, 1H, olefinic), 7.07(s, 1H, ArH), 6.32(d, J=15.2 Hz, 1H, olefinic), 6.14(d, J=11.2 Hz, 1H, olefinic), 4.15(q, 2H, $OCH_2$), 2.79(t, J=6.5 Hz, 2H, $CH_2$), 2.50(t, J=5.8 Hz, 2H, $CH_2$), 2.28(s, 3H, $CH_3$), 1.91(m, 2H, $CH_2$), 1.68(s, 4H $2CH_2$), 1.29(s, 6H, $2CH_3$), 1.27(s, 6H, $2CH_3$), 1.24(t, J=6.8 Hz, 3H, $CH_3$).

## EXAMPLE 2

Preparation of compound **101b** according to Scheme I

**(2E,4E)-3-Methyl-6-[(Z)-3,4,5,6,7,8-hexahydro-5,5,8,8-tetramethyl-2H-anthracen-1-ylidene)hexa-2,4-dienoic acid** (structure **6**, where $R_1$, $R_2$,$R_3$, $R_4$, and $R_{19}$ are methyl, $R_5$, $R_6$, $R_7$, $R_8$, $R_9$, and $R_{10}$ are hydrogen, $R_{15}$ is hydroxy, X, Y, and Z are carbon, m = 1, and n = 1)

To a solution of ester **101a** (39 mg, 0.1 mmol) in MeOH (1 ml) and $H_2O$ (1 ml) was added KOH (100 mg, 2.5 mmol) at 25°C. The reaction mixture was heated at 80°C for 3 hr, cooled, acidified (1.1 ml, 2.4 N HCl), extracted with $Et_2O$ (40 ml), dried ($MgSO_4$), concentrated, and purified using column chromatography (50% ether in hexane) to obtain the title compound **101b** (35 mg, 95%): $R_f$= 0.32(50 % ether in hexane); mp 225 - 227°C; $^1H$ NMR(400 MHz, $CDCl_3$) $\delta$ 7.32(s, 1H, ArH), 7.24(dd, J=15.2, 11.2 Hz, 1H, olefinic), 7.07(s, 1H, ArH), 6.34(d, J=15.2 Hz, 1H, olefinic), 6.16(d, J=11.2 Hz, 1H, olefinic), 5.80(s, 1H, olefinic), 2.79(t, J=6.4 Hz, 2H, $CH_2$, benzylic), 2.51(t, J=5.8 Hz, 2H, $CH_2$), 2.34(s, 3H $CH_3$), 1.92(m, 2H, $CH_2$), 1.68(s, 4H, $2CH_2$), 1.29(s, 6H, $2CH_3$), 1.27(s, 6H, $2CH_3$).

## EXAMPLE 3

Preparation of compound **102a** according to Scheme I

**Ethyl (2E,4E)-3-methyl-6-[(Z)-2,3,5,6,7,8-hexahydro-5,5,8,5-tetramethyl-cyclopenta[b]napthalen-1-ylidene]hexa-2,4-dienoate** (structure **6**, where $R_1$, $R_2$, $R_3$, $R_4$, and $R_{19}$ are methyl, $R_5$, $R_6$, $R_7$, $R_8$, $R_9$, and $R_{10}$ are hydrogen, $R_{15}$ is ethoxy, X, Y, and Z are carbon, m = 1, and n = 0).

The title compound was prepared in a manner similar to that of compound **101a** except that 2,3,5,6,7,8-hexahydro-5,5,8,8-tetramethylcyclopenta[b]naphthalen-1-one [US patent 2,815,382 (1957)] was used as the starting ketone (structure **1**) in Example 1. Compound **102a** had $R_f$= 0.56(10 % ether in hexane); $^1$H NMR (400 MHz, $CDCl_3$) δ 7.68(s, 1H, ArH), 7.45(dd, J=15.0, 11.6 Hz, 1H, olefinic), 7.24(s, 1H, ArH), 6.25( d, J=15.0 Hz, 1H, olefinic), 6.22(d, J=11.6 Hz, 1H, olefinic), 5.75(s, 1H, olefinic), 4.15(q, 2H, $OCH_2$), 2.88(bm, 2H, $CH_2$, benzylic), 2.79(bm, 2H, $CH_2$, allylic), 2.48(s, 3H, $CH_3$), 1.68(s, 4H, $2CH_2$), 1.31(s, 26H, $CH_3$), 1.29(m, 9H, $3CH_3$).

## EXAMPLE 4

Preparation of compound **102b** according to Scheme I

**(2E,4E)-3-Methyl-6-[(Z)-2,3-5,6,7,8-hexahydro-5,5,8,8,-tetramethylcyclopenta[b]naphthalen-1-ylidene]hexa-2,4-dienoic acid** (structure **6**, where $R_1$, $R_2$, $R_3$, $R_4$, and $R_{19}$ are methyl, $R_5$, $R_6$, $R_7$, $R_8$, $R_9$, and $R_{10}$ are hydrogen, $R_{15}$ is hydroxy, X, Y, and Z are carbon, m = 1, and n = 0).

The title compound was prepared by hydrolysis of compound **102a** employing the standard hydrolysis conditions (KOH, MeOH/$H_2O$) used in Example 2. Compound **102b** had $R_f$=0.45 (50% ether in hexane); mp 240 - 241°C; $^1$H NMR(400 MHz, $CDCl_3$) δ 7.67(s, 1H, ArH), 7.53(dd, J=15.0, 11.6 Hz, 1H, olefinic), 7.24(s, 1H, ArH), 6.29(d, J=15.0 Hz, 1H, olefinic), 6.26(d, J=11.6 Hz, 1H, olefinic), 5.80(s, 1H, olefinic), 2.91(bm, 2H, $CH_2$, benzylic), 2.81(bm, 2H, $CH_2$, allylic), 2.40(s, 3H, $CH_3$), 1.69(s, 4H, $2CH_2$), 1.31(s, 6H, $2CH_3$), 1.27(s, 6H, $2CH_3$).

## EXAMPLE 5

Preparation of compound **103a** according to Scheme I

**Ethyl (2E,4E)-3-methyl-6-[(Z)-1,2,3,6,7,8-hexahydro-1,1,3,3-tetramethylcycopenta[b]naphthalen-5-ylidene]hexa-2,4-dienoate** (structure **6**, where $R_1$, $R_2$, $R_3$, $R_4$ and $R_{19}$ are methyl, $R_5$, $R_6$, $R_7$, $R_8$, $R_9$, and $R_{10}$ are hydrogen, $R_{15}$ is ethoxy, X, Y, and Z are carbon, m=0 and n=1).

The title compound was prepared from 1,2,3,6,7,8-hexahydro-1,1,3,3-tetramethyl-cyclopenta[b]naphthalen-5-one. The preparation of this naphthalen-5-one was achieved as follows. 3,3-Dimethylcrotyl chloride was treated with 1,2,3,4-tetrahydronaphthalene in the presence of aluminum trichloride to give the corresponding 1,2,3,5,6,7,8-heptahdyro-1,1-dimethyl-3-oxo-cyclopenta[b]naphthalene, which was then geminally dimethylated with dimethyl zinc in the presence of titanium tetrachloride to afford 1,2,3,5,6,7,8-heptahdyro-1,1,3,3-tetramethyl-cyclopenta[b]naphthalene. The naphthalene derivative was then oxidized using $CrO_3$ in acetic acid in a manner similar to that described in Example 1. The title compound **103a** had $R_f$=0.85 (10% ether in hexane); $^1$H NMR (400 MHz, $CDCl_3$) δ 7.20 (dd, J=15.2, 11.2Hz, 1H, olefinic), 7.15 (s, 1H, ArH), 6.91 (s, 1H, ArH), 6.32 (d, J=15.2 Hz, 1H, olefinic), 6.19 (s, J=11.2 Hz, 1H, olefinic), 5.77 (s, 1H, olefinic), 4.16 (q, 2H, $OCH_2$), 2.83 (t, J=6.5 Hz, 2H, $CH_2$ benzylic), 2.53 (t, J=6.1 Hz, 2H, $CH_2$ allylic), 2.28 (s, 3H, $CH_3$), 1.91 (m, 2H, $CH_2$, methylene), 1.90 (s, 3H, $CH_2$, methylene), 1.43 (s, 6H, $2CH_3$), 1.32 (s, 6H, $2CH_3$), 1.27 (t, J=6.1 Hz, 3H, $CH_3$).

## EXAMPLE 6

Preparation of compound **103b** according to Scheme I

**(2E,4E)-3-Methyl-6-[(Z)-1,2,3,6,7,8-hexahydro-1,1,3,3-tetra-cyclopenta[b]naphthalen-5-ylidene]hexa-2,4-dienoic acid** (structure **6**, where $R_1$, $R_2$, $R_3$, $R_4$, and $R_{19}$ are methyl, $R_5$, $R_6$, $R_7$, $R_8$, $R_9$, and $R_{10}$ are hydrogen, $R_{15}$ is hydroxy, X, Y, and Z are carbon, m=0, and n=1).

The title compound was prepared by hydrolysis of ester **103a** using the standard hydrolysis conditions

employed in Example 2. Compound **103b** had $R_f$=0.30 (40% ether in hexane); mp 192-194°C; $^1$H NMR (400 MHz, $CDCl_3$) δ 7.26 (dd, J=15.2, 11.2 Hz, 1H, olefinic), 7.14 (s, 1H, ArH), 6.92 (s, 1H, ArH), 6.35 (d, J=15.2 Hz, 1H, olefinic), 6.19 (d, J=11.2 Hz, 1H, olefinic), 5.80(s, 1H, olefinic), 2.83 (t, J=6.5 Hz, 2H, $CH_2$, benzylic), 2.55 (t, J=6.1 Hz, 2H, $CH_2$, allylic), 2.30 (s, 3H, $CH_3$), 1.95 (s, 2H, $CH_2$, methylene), 1.93 (m, 2H, $CH_2$, methylene), 1.32 (s, 6H, $2CH_3$), 1.31 (s, 6H, $2CH_3$).

## EXAMPLE 7

Preparation of compound **104 b** according to Scheme I

**(2E,4E)-3-Methyl-6-(2,3,6,7,8,9-hexahydro-2,2,6,6,9,9-hexamethylbenzo[b]-chromen-4-ylidene)-hexa-2,4-dienoic acid** (structure **6**, where $R_1$, $R_2$, $R_3$, $R_4$, $R_7$, $R_8$, and $R_{19}$ are methyl, $R_5$, $R_6$, $R_9$, $R_{10}$ are hydrogen, $R_{15}$ = OH, X and Y are carbon, Z is oxygen, m = n = 1).

The title compound was synthesized in a manner similar to that of compound **101b** except 3,4,5,6,7,8-hexahydro-2,2,5,5,8,8-hexamethylnaphtho(2,3-b)-1,2-pyran-4-one (structure **1**) was employed as the starting ketone. The synthesis of the pyran-4-one is detailed here. Aluminum trichloride (25 g, 0.18 M) was added in portions to a solution of phenol (49.5 g, 0.52 M) and 2,5-dichloro-2,5-dimethylhexane (101.0 g, 0.55 M) in dichloromethane (700 ml). The reaction mixture was allowed to stir at 25-40°C for 2 h, then the dark red mixture was poured onto ice. Aqueous work up (EtOAc extraction) gave 5,6,7,8-tetrahydro-5,5,8,8-tetramethylnaphthalen-2-ol as a white solid, which was recrystallized from hexane to give colorless needles (84.8 g, 0.42 moles, 80%): $^1$H NMR (400 MHz, $CDCl_3$) δ 7.17 (d, 1H), 6.78 (d, 1H), 6.62 (dd, 1H), 4.55 (s, 1H), 1.65 (s, 4H), 1.25 (s, 12H). The hydroxynaphthalene (19.1g, 93.6 mmol) was treated dropwise with acetyl chloride (7.7 g, 98.2 mmol) in 1,2-dichloroethane (250ml) at 0°C. After completion of the addition, aluminum chloride (10 g, 75.2 mmol) was added in portions over 5 min. The mixture was refluxed for 10 h, then stirred at 25°C for 8 h. GLC analysis indicated the desired keto-phenol was present in 98.6% purity. The reaction mixture was poured onto ice and aqueous work up (EtOAc extract) gave a brown-black solid, which was dissolved in hot methanol, filtered, and concentrated to give a brown viscous semi-solid. Flash chromatography (15 % EtOAc/hexane) gave 1-(3-hydroxy-5,5,8,8-tetramethyl-5,6,7,8-tetrahydro-naphthalen-2-yl)ethanone as a light yellow solid. Recrystallization from hexane afforded white crystals (15.2 g, 61.8 mmol, 66%): $^1$H NMR (400 MHz, $CDCl_3$) δ 7.63 (s, 1H,), 6.9 (s, 1H), 2.61 (s, 3H), 1.67 (s, 4H), 1.29 (s, 6H), 1.27 (s, 6H).

A solution of the above hydroxyacetophenone (1.86 g, 7.5 mmol), pyrrolidine (590 mg, 7.5 mmol), acetone (850 mg, 14.6 mmol), and a catalytic amount of p-TsOH in benzene (650 ml) was heated at reflux for 48 hours. The mixture was cooled to 25°C and diluted with 1 N HCl (35 ml). Aqueous work up ($Et_2O$ extraction) gave a yellowish brown solid. Recrystallization from hexane afforded 2,3,6,7,8,9-hexahydro-2,2,6,6,9,9-hexamethylbenzo[g]chromen-4-one (structure **1**) as fine white crystals (560 mg, 1.95 mmol, 26%): $^1$H NMR (400 MHz, $CDCl_3$) δ 7.3 (s, 1H), 6.85 (s, 1H), 2.68 (s, 2H), 1.68 (s, 4H), 1.45 (s, 6H), 1.26 (s, 12H).

The above tricyclic ketone (556 mg, 1.95 mmol) was olefinated by means of the procedure described in Example 1 to give a crude mixture of *cis* and *trans* unsaturated nitriles. Flash chromatography (8% EtOAc/hexane) afforded (*E*)[2,3,6,7,8,9-hexahydro-2,2,6,6,9,9-hexamethylnaphtho-(2,3-b)-1,2-pyran-4-ylidene]ethanitrile as a light yellow solid (210 mg, 0.68 mmol): $^1$H NMR (400 MHz, $CDCl_3$) d7.35 (s, 1H), 6.8 (s, 1H), 5.7 (s, 1H), 2.82 (s, 2H,), 1.65 (s, 4H), 1.40 (s, 6H), 1.26 (d, 12H); and the (Z)-[2,3,6,7,8,9-hexahydro-2,2,6,6,9,9-hexamethylnaphtho (2,3-b)-1,2-pyran-4-ylidene]ethanitrile as a yellow solid (120 mg, 0.38 mmol): $^1$H NMR (400 MHz, $CDCl_3$) δ 8.30(s, 1H), 6.3(s, 1H), 5.08 (s, 1H), 2.52 (s, 2H), 1.67 (s, 4H), 1.40 (s, 6H), 1.30 (s, 6H), 1.27 (s, 6H), 1.25 (s, 6H ).

The *cis*-nitrile (120 mg, 0.38 mmol) in THF mL was reduced to the corresponding aldehyde (by treatment with DIBAL at -78° under nitrogen). GLC analysis was used to monitor the reaction due to the tendency of the *cis* product to isomerize in the presence of DIBAL. The reaction was stopped when the appearance of the *trans* product was detected by GLC. Aqueous work up (EtOAc extraction) gave an orange oily solid (95 mg). Flash chromatography (8% EtOAc/hexane) afforded [(E)-2,3,6,7,8,9-hexahydro-2,2,6,6,9,9-hexamethylnaphtho (2,3-b)-1,2 pyran-4-ylidene]acetaldehyde as a light yellow solid (20 mg, 0.06 mmol, 16% yield): $^1$H NMR (400 MHz, $CDCl_3$) δ 10.1 (d, 1H), 7.26 (s, 1H), 6.8 (s, 1H), 5.9 (d, 1H), 2.58 (s, 2H), 1.70 (s, 4H), 1.4 (s, 6H), 1.26 (s, 6H), 1.24 (s, 6H).

The above aldehyde (19mg, 0.06 mmol) was converted to the title triene ester using the procedure similar to that described in Example 1. A total of 2.4 eq of phosphonate and base (nBuLi) was used. Aqueous work up, followed by flash chromatography (20% EtOAc/hexane) afforded a mixture of ethyl (2E,4E)- and (2Z,4E)-6-(2,3,6,7,8,9-hexahydro-benzo[g]-chromen-4-ylidene)-3-methylhexa-2,4-dienoate as a yellow oil (23.5mg, 0.055 mmol, 94% yield). $^1$H NMR indicated a 9:1 ratio of isomers favoring the *trans* product: $^1$H NMR (400 MHz, $CDCl_3$) δ 7.40 (s, 1H), 7.35 (dd, 1H), 6.77 (m, 1H), 6.40 (d, 1H), 6.05 (d, 1H), 5.8 (s, 1H), 4.20 (m, 2H), 2.43 (s, 2H), 2.35 (s, 3H), 1.67 (s, 4H), 1.33 (s, 6H), 1.31 (t, 3H), 1.30 (s, 6H), 1.25 (s, 6H). The isomers could not be separated by chromatography and were used as a mixture in subsequent reactions.

The above mixture of esters (23mg, 0.054 mmol) was hydrolyzed to the corresponding acids according to the procedure described in Example 2. Removal of solvent gave the title compound as a yellow solid (19mg, 0.048 mmol, 90%). A 2.5mg sample of the crude material was purified by HPLC (80:20=MeOH: 10 mM ammonium acetate) to give the title acid **104b** as a bright yellow solid (1.5mg): TLC, Rf=0.31(20% acetone/hexane) $^1$H NMR (400 MHz, $CDCl_3$) δ 7.40 (dd, 1H), 7.35 (s, 1H), 6.77 (s, 1H), 6.40 (d, 1H), 6.08 (d, 1H), 5.85 (br s, 1H), 2.45 (s, 2H), 2.35 (s, 3H), 1.70(s, 4H), 1.36 (s, 6H), 1.30 (s, 6H), 1.25 (s, 6H).

## EXAMPLE 8

Preparation of compound **105a** according to Scheme I

**Ethyl (2E,4E)-3-methyl-6-[(Z)-3,4,5,6,7,8-hexahydro-10-nitro-5,5,8,8-tetramethyl-2H-anthracen-1-ylidene]hexa-2,4-dienoate** (structure **6**, where $R_1$, $R_2$, $R_3$, $R_4$, and $R_{19}$ are methyl; $R_5$, $R_6$, $R_7$, $R_8$, and $R_{10}$ are hydrogen; $R_9$ is nitro; $R_{15}$ is ethoxy; X, Y, Z are carbon, m = n = 1)

The title compound was prepared according to Example 1 except that 10-nitro-1,2,3,4,5,6,7,8-octahydro-5,5,8,8-tetramethylanthracene was used as the starting material. The synthesis of this intermediate is described below.

1,2,3,4,5,6,7,8-Octahydro-5,5,8,8-tetramethylanthracene (1.1 g, 4.5 mmol) was suspended in acetic acid (4 mL) and concentrated sulfuric acid (0.5 mL). The mixture was cooled to 10°C, and nitric acid (3 mL) was added dropwise so that the internal temperature remained below 20°C. The mixture was allowed to warm to room temperature and stirred for 1 h. The dark red mixture was diluted with ice/water (10 mL) and extracted with EtOAc (3 x 20 mL). The EtOAc layer was washed with water, saturated aqueous $NaHCO_3$, water, and brine. The solution was dried ($Na_2SO_4$), filtered, and concentrated to give a brown oil. The crude product was crystallized with $CH_2Cl_2$/ether/hexanes to give 10-nitro-1,2,3,4,5,,6,7,8-octahydro-5,5,8,8-tetramethylanthracene 1.2 g (94%) as a yellow solid: mp 129.3-130.7°C; IR (thin film) 2930 s, 2864 s, 1526 s, 1470 m, 1435 m, 1369 s, 1269 w, 1128 w, 918 w, 754 m $cm^{-1}$; $^1$H NMR (400 MHz, $CDCl_3$) δ 7.14 (s, 1H, aromatic), 2.72 (br s, 2H, $CH_2$), 2.50 (br s, 2H, $CH_2$), 1.75 (m, 6H, $3CH_2$), 1.65 (m, 2H, $CH_2$), 1.32 (s, 6H, $2CH_3$), 1.29 (s, 6H, $2CH_3$).

10-Nitro-1,2,3,4,5,6,7,8-octahydro-5,5,8,8-tetramethylanthracen-1-one (structure **1**, where $R_1$, $R_2$, $R_3$, $R_4$ are methyl; $R_5$, $R_6$, $R_7$, $R_8$, and $R_{10}$ are hydrogen; $R_9$ is nitro; X, Y, Z are carbon, m = n = 1) was prepared according to Example 1 except chromium trioxide in acetic acid was used as the oxidant to give 148 mg (83% based on recovered starting material) of the ketone as a yellow solid: mp 134.0-136.5°C; IR (thin film) 2964 s, 2931 s, 1693 s, 1597 s, 1531 s, 1468 s, 1413 m, 1389 s, 1372 s, 1337 m, 1263 s, 1249 s, 1232 m, 1178 m, 1053 m, 912 m, 750 m $cm^{-1}$; $^1$H NMR (400 MHz, $CDCl_3$) δ 8.17 (s 1H, aromatic), 2.71 (t, J=6.2 Hz, 2H, $CH_2$), 2.63 (t, J=6.6 Hz, 2H, $CH_2$), 2.12 (m, 2H, $CH_2$); 1.74 (m, 2H, $CH_2$), 1.70 (m, 2H, $CH_2$), 1.35 (s, 6H, $2CH_3$), 1.33 (s, 6H, $2CH_3$); MS (Cl) m/e 302 ($MH^+$, 100), 289 (30), 272 (9).

Using the procedure described in Example 1, the above ketone was transformed into the title compound **105a** which was obtained as a yellow oil: $^1$H NMR (400 MHz, $CDCl_3$) δ 7.42 (s, 1H, aromatic), 6.98 (dd, J=15.2, 11.0 Hz, 1H, olefonic), 6.34 (d, J=15.2 Hz, 1H, olefonic), 6.21 (d, J=11.0 Hz, 1H, olefonic), 5.77 (s, 1H, oleforic), 4.14 (q, J=7.0 Hz, 2H, $-OCH_2$), 2.56 (t, J=6.6 Hz, 2H, $CH_2$), 2.44 (br t, J=6.2 Hz, 2H, $CH_2$), 2.23 (d, J=0.8 Hz, 3H, $CH_3$ allylic), 1.88 (m, 2H, $CH_2$), 1.71 (m, 2H, $CH_2$), 1.66 (m, 2H, $CH_2$), 1.30 (s, 6H, $2CH_3$), 1.28 (s, 6H, $2CH_3$), 1.28 (t, J=7.0 Hz, 3H, $CH_3$ ethyl).

## EXAMPLE 9

Preparation of compound **105b** according to Scheme I

**(2E,4E)-3-Methyl-6-[(Z)-3,4,5,6,7,8-hexahydro-10-nitro-5,5,8,8-tetramethyl-2H-anthracen-1-ylidene]hexa-2,4-dienoic acid** (structure **6**, where $R_1$, $R_2$, $R_3$, $R_4$, and $R_{19}$ are methyl; $R_5$, $R_6$, $R_7$, $R_8$, and $R_{10}$ are hydrogen; $R_9$ is nitro; $R_{15}$ is hydroxy, X, Y, Z are carbon, m = n = 1).

Compound **105a** was hydrolyzed using the procedure of Example 2, to give the title compound **105b** 3.3 mg (31%) as a yellow film: $^1$H NMR (400 MHz, $CDCl_3$) δ 7.45 (s, 1H, aromatic), 7.08 (dd, J=15.2, 11.2 Hz, 1H, olefonic), 6.42 (d, J=15.2 Hz, 1H, olefonic), 6.25 (d, J=11.2 Hz, 1H, olefonic), 5.84 (s, 1H, olefonic), 2.60 (t, J=6.6 Hz, 2H, $CH_2$), 2.49 (br t, J=6.3 Hz, 2H, $CH_2$), 2.29 (d, J=0.8 Hz, 3H, $CH_3$ allylic), 1.93 (m, 2H, $CH_2$), 1.75 (m, 2H, $CH_2$), 1.69 (m, 2H, $CH_2$), 1.34 (s, 6H, $2CH_3$), 1.33 (s, 6H, $2CH_3$).

## EXAMPLE 10

Preparation of compound **106a** according to Scheme II

**Ethyl (2E,4E)-3-methyl-6-[(Z)-3,4,5,6,7,8-hexahydro-4,5,,5,5,8,8-hexamethyl-2*H*-anthracen-1-ylidene]hexa-2,4-dienoate** (structure **6**, where $R_1$, $R_2$, $R_3$, $R_4$, and $R_{19}$ are methyl, $R_5$, $R_6$, $R_7$, $R_8$, $R_9$, $R_{10}$ are H, $R_{15}$ is ethoxy, X and Y are carbon; Z is $C(CH_3)_2$; m= n=1).

A 100 mL round-bottom flask was flame dried under a nitrogen atmosphere, charged with dichloromethane (20.0 mL) and cooled to -40°C. A solution of titanium tetrachloride in toluene (2.0 M, 11.7 mL; 23.4 mM) was added, followed by a solution of dimethylzinc in toluene (10 M, 11.7mL, 11.7 mM). The mixture was stirred at -40°C for 30 min and a solution of 3,4,5,6,7,8-hexahydro-5,5,8,8-tetramethyl-2H-anthracen-1-one (1.0 g, 3.9 mmol, from Example 1) in dichloromethane (20 ml) was slowly added. The reaction mixture was warmed to room temperature and stirred for 36 h. The mixture was poured onto a solution of methanol and dry ice, followed by saturated ammonium chloride. The solution mixture was extracted with $CH_2Cl_2$, washed with water (3 x 20 mL), and brine (3 x 20 mL). The solvent was evaporated and the crude residue was purified by flash chromatography using hexanes as an eluent to give 937 mg of 1,1,5,5,8,8-hexamethyl-1,2,3,4,5,6,7,8-octahydroanthracene, 89% yield: $^1H$ NMR (400 MHz, $CDCl_3$) δ 7.23 (1 H, Ar), 6.93 (1 H, Ar), 2.70 (t, J=8.0 Hz, 2H), 1.78 (m, 2 H), 1.65 (s, 4 H), 1.63 (m, 2 H), 1. 27 (s, 3H), 1.26 (s, 6 H), 1.25 (s, 3H).

A solution of chromium trioxide (13.55 mL of a 10% $CrO_3$/AcOH solution) was slowly added to a solution of finely ground 1,1,5,5,8,8-hexamethyl-1,2,3,4,5,6,7,8-octahydroanthracene (861 mg, 3.18 mmol) in AcOH (12 mL) at 25°C. The mixture was stirred for 30 min. Ice water (100 mL) was added, and the mixture was extracted with EtOAc (3 x 50 mL). The organic extracts were combined and twice treated with $Et_3N$ (10 mL), and washed and with sat. $NaHCO_3$ (20 mL), water (2 x 20 mL) and brine (2 x 20 mL). The solvent was evaporated and the crude residue was purified by silica gel chromatography (hexanes:EtOAc 9:1) to give 831 mg of pure 3,4,5,6,7,8-hexahydro-4,4,5,5,8,8-hexamethyl-2H-anthracen-1-one (structure **1** where $R_1$, $R_2$, $R_3$, and $R_4$ are methyl, $R_5$, $R_6$, $R_7$, $R_8$, $R_9$, and $R_{10}$ are hydrogen, X and Y are carbon, Z is $C(CH_3)_2$ and m = n = 1): $^1H$ NMR (400 MHz, $CDCl_3$) δ 7.97 (s, 1H, Ar), 7.31 (s, 1H, Ar), 2.68 (t, J=8.0 Hz, 2H), 1.98 (t, J=6.0 Hz, 2H), 1.68 (s, 4H), 1.37 (s, 3H), 1.30 (s, 3H), 1.29 (s, 3H). The above ketone was transformed to the title compound as outlined in Scheme II.

A 50 mL round-bottom flask was flame-dried and charged with anhydrous THF (10 mL) and ethyl magnesium bromide (2.65 mL of 1 M solution in THF, 2.65 mM) and the structure was cooled to 0°C. Under a nitrogen atmosphere, a solution of ethyl ethynyl ether (0.375 mL of a 50% solution in hexanes, Aldrich Inc., 2.63 mM) was slowly added. The crude mixture was warmed to room temperature and stirred for 20 min. A solution of 4,4,5,5,8,8-hexamethyl-1,2,3,4,5,6,7,8-octahydroanthracen-1-one (0.50 g, 1.76 mmol) in THF (10 mL) was slowly added and stirring was continued for 2 h. The reaction mixture was quenched with a sat solution of ammonium chloride and extracted with diethyl ether. The organic layer was dried over $MgSO_4$, and the solvent evaporated. The crude residue was dissolved in ethanol (20 mL) and $CO_2$ (dry ice) was bubbled through the solution for 3 h. After stirring for 12 h at 25°C, the solvent was evaporated and the residue was purified by silica gel chromatography to give 455 mg (73% yield) of a mixture of (Z) and (E) esters in a 3.5:1 ratio. The mixture of esters (455 mg, 1.28 mmol) was dissolved in anhydrous dichloromethane (5.0 mL) and cooled to -78°C. DBAL in dichloromethane (3.2 mL of 1.0 M solution, 3.2 mM) was added and the mixture stirred for 15 min. The reaction was quenched at -78°C using a saturated solution of Rochelle salt and extracted with dichloromethane. The organic layer was dried over $MgSO_4$, then evaporated to give a white residue which was purified by chromatography to afford the corresponding *cis* allylic alcohol (211 mg) and *trans* allylic alcohol (37 mg). The *cis*-alcohol 2-[(Z)-3,4,5,6,7,8-hexahydro-4,4,5,5,8,8-hexamethyl-2H-anthracene-1-ylidine] ethanol: $^1H$ NMR (400 MHz, $CDCl_3$) δ 7.25 (s, 1H, Ar), 7.03 (s, 1H, Ar), 5.57 (t, J=4.5 Hz, 1H), 4.43 (t, J=4.5 Hz, 2H), 2.46 (m, 2H), 1.75 (m, 2H), 1.67 (s, 4H), 1.28 (s, 18H). The *trans*-alcohol 2-[(E)-3,4,5,6,7,8-hexahydro-4,4,5,5,8,8-hexamethyl-2H-anthracene-1-ylidine] ethanol; $^1H$ NMR (400 MHz, $CDCl_3$) δ 7.56 (s, 1 H, Ar), 7.02 (s, 1H, Ar), 6.15 (t, J=4.5 Hz, 1H), 4.39 (t, J=4.5 Hz, 2H), 2.46 (m, 2H), 1.75 (m, 2H), 1.68 (s, 4H), 1.27 (s, 18H).

To a solution of 2-[(Z)-3,4,5,6,7,8-hexahydro-4,4,5,5,8,8-hexamethyl-2H-anthracene-1-ylidine] ethanol (100 mg, 0.32 mmol) in dichloromethane (2.0 mL) was added a total of 200 mg of $MnO_2$ in 4 portions. The mixture was stirred for 1 h, then filtered through a pad of celite and thoroughly rinsed with dichloromethane (total of 20 mL). The solvent was evaporated to give 93 mg of the virtually pure corresponding aldehyde (98% yield) which was used directly in the next step. A flame-dried 25 mL round-bottom flask was charged into diethyl 3-ethoxycarbonyl-2-methylprop-2-enylphosphonate (142 mg, 0.53 mmol) and anhydrous THF (15.0 mL) and the solution was cooled to 0°C. Anhydrous DMPU (0.25 mL) and *n*BuLi in hexanes (0.34 mL of 1.5 M solution, 0.51 mM) were added. The mixture was stirred at 0°C for 20 min, then cooled to -78°C. A solution of the above aldehyde in THF (3.0 mL) was added dropwise. After stirring at -78°C for 10 min, the mixture was allowed to warm to room temperature. After 30 min, the reaction was quenched with a saturated aqueous solution of ammonium chloride and extracted with EtOAc. The organic layer was dried over $MgSO_4$ and the solvent was evaporated. The residue was purified by chromatography to give the title ester **106a** (108

mg, 86% yield): $^1$H NMR (400 MHz, $CDCl_3$) δ 7.25 (m, 3H, 2ArH and 1 vinylic H), 6.38 (d, J=16 Hz, 1H), 6.27 (d, J=12 Hz, 1H), 5.80 (s, 1H), 4.1 (q, J=4.5 Hz, 2H, O-$CH_2$-), 2.55 (t, J=4.5 Hz, 2H), 2.32 (s, 3H, $CH_3$), 1.78 (t, J=4.0 Hz, 2H), 1.69 (s, 4H), 1.30 (s, 18H), 1.28 (t, J=4.0 Hz, 3H). $^1$H NMR data indicated the presence of a geometric isomer at the 2,3 double bond in a ratio of *trans:cis* 5:1.

## EXAMPLE 11

Preparation of compound **106b** according to Scheme II

**(2E,4E)-3-Methyl-6-[(Z)-1,2,3,4,5,6,7,8-hexahydro-4,4,5,5,8,8-hexamethyl-2H-anthracen-1-ylidene]hexa-2,4-dienoic acid** (structure **6** where $R_1$, $R_2$, $R_3$, $R_4$, and $R_{19}$ are methyl; $R_5$, $R_6$, $R_7$, $R_8$, $R_9$, and $R_{10}$ are H; $R_{15}$ is hydroxy; X and Y are carbon; Z is $C(CH_3)_2$; m = n = 1).

To a solution of the above compound **106a** (108 mg, 0.23 mmol) in ethanol (3.0 mL) was added NaOH (100 mg) and water (3.0 mL). The mixture was heated at 70°C for 4 h, then cooled to room temperature, acidified using 1 N HCl, and extracted with EtOAc (20 mL). The organic layer was washed with water (2 x 10 mL), brine (2 x 10 mL) and dried over $MgSO_4$. The solvent was evaporated and the residue was recrystallized from acetone-water three times to give the geometrically pure title acid **106b** in 56%: $^1$H NMR (400 MHz, $CDCl_3$) δ 7.25 (m, 3H, 2ArH and 1 vinylic H), 6.38 (d, J=16 Hz, 1H), 6.27 (d, J=12 Hz, 1H); 5.80 (s, 1H), 2.55 (t, J=4.5 Hz, 2), 2.32 (s, 3H, $CH_3$), 1.78 (t, J=4.0 Hz, 2H), 1.69 (s, 4H), 1.3 (s, 18H).

## EXAMPLE 12

Preparation of compound **107b** according to Scheme II

**(2E,4E)-3-Methyl-6-[(Z)-2,3,5,6,7,8-hexahydro-3,5,5,8,8-pentamethyl-cyclopenta[b]naphthalen-1-ylidene]hexa-2,4-dienoic acid** (structure **6**, where $R_1$, $R_2$, $R_3$, $R_4$ and $R_{19}$ are methyl; $R_5$, $R_6$, $R_9$ and $R_{10}$ are hydrogen; $R_{15}$ is hydroxy; X and Y are carbon; Z is $CHCH_3$; m = 1 and n = 0)

The title acid was prepared from 2,3,5,6,7,8-hexahydro-3,5,5,8,8-pentamethyl-cyclopenta[b]naphthalen-1-one. The preparation of the above naphthalen-1-one is shown below. Friedel-Crafts alkylation of crotonic acid with 1,2,3,4-tetrahydro-1,1,4,4-tetramethylnaphthalene in the presence aluminum trichloride gave the corresponding enone, which was then cyclized to the naphthalen-1-one using PPA at 110°C for 3h). Procedures similar to those in Examples 10 and 11 afforded the compound **107b**: $R_f$=0.25 (40% ether in hexane); mp 216-218°C; $^1$H NMR (400 MHz, $CDCl_3$) d: 7.66(s, 1H, ArH), 7.54(dd, J=15.1, 11.6 Hz, 1H, olefinic), 7.22(s, 1H, ArH), 6.31(d, J=15.1 Hz, 1H, olefinic), 6.27(d, J=11.6 Hz, 1H, olefinic), 5.82(s, 1H, olefinic), 3.20(m, 1H), 2.99(m, 1H, allylic), 2.42(m, 4H, $CH_3$, allylic), 1.71(s, 4H, $2CH_2$), 1.33(m, 6H, $2CH_3$), 1.30(m, 9H, $3CH_3$).

## EXAMPLE 13

Preparation of compound **108b** accordine to Scheme II

**(2E,4E)-3-Methyl-6-[(Z)-3,5,6,7-tetrahydro-5,5,7,7-tetramethyl-2H-5-indacen-1-ylidene]hexa-2,4-dienoic acid** (structure **6**, where $R_1$, $R_2$, $R_3$, $R_4$, and $R_{19}$ are methyl; $R_5$, $R_6$, $R_7$, $R_8$, $R_9$ and $R_{10}$ are hydrogen; $R_{15}$ is hydroxy; X, Y and Z are carbon; m = n = 0)

The title acid was prepared from 5,5,7,7-tetramethyl-3,5,6,7-tetrahydro-2H-5-indacen-1-one using procedures similar to those of Examples 10 and 11. The above indacen-1-one was prepared in a manner similar to that of 1,2,3,6,7,8-hexahydro-1,1,3,3-tetramethyl-cyclopenta[b]naphthalene-5-one in Example 5, except that indane was employed as the starting material. The title compound **108b** had $R_f$=0.35 (50% ether in hexane); mp 159-161°C; $^1$H NMR (400 MHz, $CDCl_3$) d: 7.52(dd, J=15.4, 12.9 Hz, 1H, olefinic), 7.43(s, 1H, ArH), 7.06(s, 1H, ArH), 6.31(d, J=15.4 Hz, 1H, olefinic), 6.28(d, J=12.9 Hz, 1H, olefinic), 5.83(s, 1H, olefinic), 2.95(m, 2H, $CH_2$, benzylic), 2.88(m, 2H, $CH_2$, allylic), 2.40(s, 3H, $CH_3$), 1.96(s, 2H, $CH_2$), 1.34(m, 6H, $2CH_3$), 1.32(s, 6H, $2CH_3$).

## EXAMPLE 14

Preparation of compound **109a** according to Scheme III

**Ethyl (2E,4E)-3-methyl-6-[(E)-3,4,5,6,7,8-hexahydro-5,5,8,8-tetramethyl-2H-anthracen-1-ylidene]hexa-2,4-dienoate** (structure **7**, where $R_1$, $R_2$, $R_3$, $R_4$, and $R_{19}$ are methyl; $R_5$, $R_6$, $R_7$, $R_8$, $R_9$, $R_{10}$ are hydrogen; $R_{15}$ is ethoxy; X, Y, and Z are carbon; m = n = 1).

To a solution of 3,4,5,6,7,8-hexahydro-5,5,8,8-tetramethyl-2H-anthracen-1-one (512 mg, 2 mmol, from Example 1) in MeOH(10 ml) was added $NaBH_4$ (76 mg, 2 mmol) at 0°C. The reaction mixture was stirred at that temperature for 30 min, then quenched with sat. aqueous $NH_4Cl$ (5 ml), extracted with ether (50 ml), dried ($MgSO_4$), and concentrated under reduced pressure to give the corresponding tricyclic alcohol, which was used without further purification. To the above alcohol (516 mg, 2 mmol) in MeOH (5 ml) was added $Ph_3P$-HBr (686 mg, 2 mmol) at 25°C. The mixture was heated at 85°C for 5 h. Removal of the solvent, followed by addition of hexane (100 ml) gave a white solid, which was then filtered to give pure 3,4,5,6,7,8-hexahydro-5,5,8,8-tetramethyl-2H-anthracen-1-triphenylphosphonium bromide (697 mg, 60%).

To a solution of the above phosphonium salt (581 mg, 1 mmol) in THF (8 ml) was added nBuLi (0.4 ml, 2.5 M, 1 mM) at 0°C and the resulting dark-red solution was stirred at that temperature for 30 min to afford the ylide. To this freshly prepared ylide was added ethyl (2E,4E)-3-methyl-5-formylpenta-2,4-dienoate. [The ethyl dienoate was prepared by the condensation of ethyl-3-methyl-4-oxocrotonate and (triphenylphosphoranylidene)acetaldehyde in benzene at 85°C for 3 h using benzoic acid as a catalyst (168 mg, 1 mmol) in THF(5 ml) at 0°C, and the resulting mixture was stirred at this temperature for 30 min.] The reaction mixture was quenched with $NH_4Cl$ (10 ml), extracted with ether (50 ml), dried($MgSO_4$), concentrated, and purified by chromatography (10% ether in hexane) to give the pure title compound **109a** (372 mg, 95%): $R_f$=0.52 (10% ether in hexane); $^1$H NMR(400 MHz, $CDCl_3$) $\delta$ 7.58 (s, 1H, ArH), 7.04 (dd, J=11.2, 15.0 Hz, 1H, olefinic), 7.02 (s, 1H, ArH), 6.69 (d, J=11.2 Hz, 1H, olefinic), 6.39 (d, J=15.0 Hz, 1H, olefinic), 5.79 (s, 1H, olefinic), 4.16 (q, 2H, $OCH_2$), 2.75 (t, J=6.1 Hz, 2H, $CH_2$, benzylic), 2.68 (t, J=5.9 Hz, 2H, $CH_2$, allylic), 2.36 (s, 3H, $CH_3$), 1.85 (m, 2H, $CH_2$), 1.65 (s, 4H, $2CH_2$), 1.31 (s, 6H, $2CH_3$), 1.25 (m, 9H, $3CH_3$).

## EXAMPLE 15

Preparation of compound **109b** according to Scheme III

**(2E,4E)-3-Methyl-6-[(E)-3,4,5,6,7,8-hexahydro-5,5,8,8-tetramethyl-2H-anthracen-1-ylidene]hexa-2,4-dienoic acid** (structure **7**, where $R_1$, $R_2$, $R_3$, $R_4$, and $R_{19}$ are methyl, $R_5$, $R_6$, $R_7$, $R_8$, $R_9$, and $R_{10}$ are hydrogen, $R_{15}$ is hydroxy, X, Y, and Z are carbon, m = n = 1).

The title acid was prepared from hydrolysis of compound **109a** using the standard conditions employed in Example 2. Compound **109**b had $R_f$=0.25 (50% ether in hexane); mp 230 - 231°C; $^1$H NMR (400 MHz, $CDCl_3$) $\delta$ 7.60(s, 1H, ArH), 7.09(dd, J=1.2, 15.0 Hz, 1H, olefinic), 7.03(s, 1H, ArH), 6.70 (d, J=11.2 Hz, 1H, olefinic), 6.43 (d, J=15.0 Hz, 1H, olefinic), 5.82 (s, 1H, olefinic), 2.77 (t, J=6.1 Hz, 2H, CH2, benzylic), 2.70 (t, J=5.9 Hz, 2H, $CH_2$, allylic), 2.37 (s, 3H, $CH_3$), 1.86 (m, 2H, $CH_2$), 1.67 (s, 4H, $2CH_2$), 1.31 (s, 6H, $2CH_3$), 1.26 (s, 6H, $2CH_3$).

## EXAMPLE 16

Preparation of compound **110a** according to Scheme III

**Ethyl (2E,4E)-3-methyl-6-[(E)-2,3,5,6,7,8-hexahydro-5,5,8,8-tetramethylcyclopenta[b]naphthalen-1-ylidene]hexa-2,4-dienoate** (structure **7**, where $R_1$, $R_2$, $R_3$, $R_4$, and $R_{19}$ are methyl; $R_5$, $R_6$, $R_7$, $R_8$, $R_9$, and $R_{10}$ are hydrogen; $R_{15}$ is ethoxy; X, Y, and Z are carbon; m = 1, n = 0).

The title compound was prepared in a manner similar to that of compound **59a** except that 2,3,5,6,7,8-hexahydro-5,5,8,8-tetramethylcyclopenta[b]-1-one [US patent 2.815.382 (1957)] was used in place of 3,4,6,7,8-hexahydro-5,5,8,8-tetramethyl-2H-anthracen-1-one for the $NaBH_4$ reduction step in Example 14. Compound **110a** had mp 128 - 129°C, $^1$H NMR(400 MHz, $CDCl_3$) $\delta$ 7.47 (s, 1H, ArH), 7.23 (s, 1H, ArH), 6.85 (dd, J=11.3, 15.2 Hz, 1H, olefinic), 6.62 (d, J=11.3 Hz, 1H, olefinic), 6.31 (d, J=15.2 Hz, 1H, olefinic), 5.75 (s, 1H, olefinic), 4.17 (q, 2H, $OCH_2$), 3.05-2.85 (m, 4H, $2CH_2$, benzylic and allylic), 2.45 (s, 3H, $CH_3$), 1.65 (s, 4H, $CH_2$), 1.28 (s, 6H, $2CH_3$), 1.27 (m, 9H, $3CH_3$).

**EXAMPLE 17**

Preparation of compound **110b** according to Scheme III

**(2E,4E)-3-Methyl-6-[(E)-2,3,5,6,7,8-hexahydro-5,5,8,8-tetramethylcyclopenta[b]naphthalen-1-ylidene]hexa-2,4-dienoic acid** (structure **7**, where $R_1$, $R_2$, $R_3$, $R_4$, and $R_{19}$ are methyl; $R_5$, $R_6$, $R_7$, $R_8$, $R_9$, and $R_{10}$ are hydrogen; $R_{15}$ is hydroxy; X, Y, and Z are carbon; m = 1, and n = 0).

The title acid was prepared by hydrolysis of compound **110a** using the standard hydrolysis conditions in Example 2. Compound **110b** had mp 200-202°C, $^1H$ NMR (400 MHz, $CDCl_3$) δ 7.47 (s, 1H, ArH), 7.23 (s, 1H, ArH), 6.92 (dd, J=11.3, 15.1 Hz, 1H, olefinic), 6.63 (d, J=11.3 Hz, 1H, olefinic), 6.35 (d, J=15.1 Hz, 1H, olefinic), 5.79 (s, 1H, olefinic), 3.02-2.91 (m, 4H, $2CH_2$, benzylic and allylic), 2.37 (s, 3H, $CH_3$), 1.67 (s, 4H, $2CH_2$), 1.30 (s, 6H, $2CH_3$), 1.27 (s, 6H, $2CH_3$).

**EXAMPLE 18**

Preparation of compound **111a** according to Scheme III

**Ethyl (2E,4E)-3-methyl-6-[(E)-1,2,3,6,7,8-hexahydro-1,1,3,3-tetramethylcyclopenta[b]naphthalen-5-ylidene]hexa-2,4-dienoate** (structure **7**, where $R_1$, $R_2$, $R_3$, $R_4$, and $R_{19}$ are methyl; $R_5$, $R_6$, $R_7$, $R_8$, $R_9$, and $R_{10}$ are hydrogen; $R_{15}$ is ethoxy; X, Y, and Z are carbon; m=0, and n=1).

The title compound was prepared in a manner similar to that of compound **59a** except that 1,2,3,6,7,8-hexahydro-1,1,3,3-tetramethylcyclopenta[b]naphthalen-1-one (the preparation of the title ketone is described in example 5) was used as the starting ketone in the $NaBH_4$ reduction step of Example 14. Compound **111a** had $R_f$=0.84 (10% ether in hexane); $^1H$ NMR (400 MHz, $CDCl_3$) δ 7.4 (s, 1H, ArH), 7.07 (dd, J=15.0, 11.3 Hz, 1H, olefinic), 6.86 (s, 1H, ArH), 6.75 (d, J=11.3 Hz, 1H, olefinic), 6.42 (d, J=15.0 Hz, 1H, olefinic), 5.81 (s, 1H, olefinic), 4.15 (q, 2H, $OCH_2$), 2.82 (t, J=6.0 Hz, 2H, $CH_2$, benzylic), 2.73 (t, J=5.71 Hz, 2H, $CH_2$, allylic), 2.38 (s, 3H, $CH_3$), 1.91 (s, $CH_2$, 2H), 1.89 (m, 2H, $CH_2$), 1.32 (s, 6H, $2CH_3$), 1.30 (m, 9H, $3CH_3$).

**EXAMPLE 19**

Preparation of compound **111b** according to Scheme III

**(2E,4E)-3-Methyl-6-[(E)-1,2,3,6,7,8-hexahydro-1,1,3,3-tetramethylcyclopenta[b]-5-ylidene]hexa-2,4-dienoic acid** (structure **7**, where $R_1$, $R_2$, $R_3$, $R_4$, and $R_{19}$ are methyl, $R_5$, $R_6$, $R_7$, $R_8$, $R_9$, and $R_{10}$ are hydrogen, $R_{15}$ is hydroxy, X, Y, and Z are carbon, m=0, and n=1)

The title compound was prepared from hydrolysis of ester **111a** using the standard hydrolysis conditions employed in Example 2. Acid **111b** had $R_f$=0.40 (30% ether in hexane), mp 207-209°C, $^1H$ NMR (400 MHz, $CDCl_3$) δ 7.41 (s, 1H, ArH), 7.10 (dd, J=15.0, 11.3 Hz, 1H, olefinic), 6.87 (s, 1H, ArH), 6.77 (d, J=11.3 Hz, 1H, olefinic), 6.45 (d, J=15.0 Hz, 1H, olefinic), 5.84 (s, 1H, olefinic), 2.83 (t, J=6.0 Hz, 2H, $CH_2$, benzylic), 2.73 (t, J=5.7 Hz, 2H, $CH_2$, allylic), 2.40 (s, 3H, $CH_3$), 1.91 (s, 2H, $CH_2$), 1.88 (m, 2H, $CH_2$), 1.34 (s, 6H, $2CH_3$), 1.30 (s, 6H, $2CH_3$).

**EXAMPLE 20**

Preparation of compound **112a** according to Scheme I & III

**Ethyl (2Z,4E)-3-methyl-6-[(E)-1,2,3,6,7,8-hexahydro-1,1,3,3-tetramethylcyclopenta[b]naphthalen-5-ylidene]hexa-2,4-dienoate** (structure **7**, where $R_1$, $R_2$, $R_3$, $R_4$, and $R_{19}$ are methyl; $R_5$, $R_6$, $R_7$, $R_8$, $R_9$, and $R_{10}$ are hydrogen; $R_{15}$ is ethoxy; X, Y, and Z are carbon; m=0, and n=1)

The title compound was obtained as a by-product from the preparation of compound **111a** of Example 18. Compound **112a** had $R_f$=0.85 (10% ether in hexane); $^1H$ NMR (400 MHz, $CDCl_3$) δ 7.92 (d, J=15.2 Hz, 1H, olefinic), 7.46 (s, 1H, ArH), 7.12 (dd, J=15.2, 11.2 Hz, 1H, olefinic), 6.90 (d, J=11.2 Hz, 1H, olefinic), 6.86 (s, 1H, ArH), 5.82 (s, 1H, olefinic), 4.20 (q, 2H, $OCH_2$), 2.90 (t, J=5.9 Hz, 2H, $CH_2$, benzylic), 2.75 (t, J=5.7 Hz, 2H, $CH_2$, allylic), 2.10 (s, 3H, $CH_3$), 1.95 (s, 2H, $CH_2$), 1.93 (m, 2H, $CH_2$,), 1.32 (s, 6H, $2CH_3$), 1.30 (m, 9H, $3CH_3$).

**EXAMPLE 21**

Preparation of compound **112b** according to Scheme I

**(2Z,4E)-3-Methyl-6-[(E)-1,2,3,6,7,8-hexahydro-1,1,3,3-tetramethylcyclopenta[b]naphthalen-5-ylidene]hexa-2,4-dienoic acid** (structure **7**, where $R_1$, $R_2$, $R_3$, $R_4$, and $R_{19}$ are methyl; $R_5$, $R_6$, $R_7$, $R_8$, $R_9$, and $R_{10}$ are hydrogen; $R_{15}$ is hydroxy; X, Y, and Z are carbon; m=0, and n=1)

The title compound was prepared from hydrolysis of compound **112a** using the standard hydrolysis conditions described in Example 2. Compound **112b** had $R_f$=0.50 (30% ether in hexane); $^1$H NMR (400 MHz, $CDCl_3$) $\delta$ 7.85 (d, J=15.2 Hz, 1H, olefinic), 7.46 (s, 1H, ArH), 7.12 (dd J=15.2, 11.3 Hz, 1H, olefinic), 6.90 (d, J=11.3 Hz, 1H, olefinic), 6.86 (s, 1H, ArH), 5.69 (s, 1H, olefinic), 2.82 (t, J=6.1 Hz, 2H, $CH_2$, benzylic), 2.23 (t, J=5.9 Hz, 2H, $CH_2$, allylic), 2.14 (s, 3H, $CH_3$), 1.91 (s, 2H, $CH_2$), 1.89 (m, 2H, $CH_2$), 1.25 (s, 6H, $2CH_3$), 1.22 (s, 6H, $2CH_3$).

**EXAMPLE 22**

Preparation of compound **113b** according to Scheme II

**(2E,4E)-3-Methyl-6-[(E)-3,4,5,6,7,8-hexahydro-4,4,5,5,8,8-hexamethyl-2H-anthracen-1-ylidene]hexa-2,4-dienoic acid** (structure **7**, where $R_1$, $R_2$, $R_3$, $R_4$, and $R_{19}$ are methyl; $R_5$, $R_6$, $R_7$, $R_8$, $R_9$, and $R_{10}$ are hydrogen; $R_{15}$ is hydroxy; X and Y are carbon; Z is $C(CH_3)_2$; m=n=1).

This compound was prepared starting from 2-[(E)-3,4,5,6,7,8-hexahydro-2H-4,4,5,5,8,8-hexamethylanthracene-1-ylidine] ethanol as described for the (2E,4E,6Z)-isomer (Examples 10 and 11). Compound **113b** had: $^1$H NMR (400 MHz, $CDCl_3$) $\delta$ 7.59 (s, 1 H, Ar); 7.08 (d J=12 Hz, 1H,), 7.0 (s, 1H, Ar), 6.7 (d, J=12 Hz, 1H), 6.28 (d, J=12 Hz, 1H), 5.82 (s, 1H), 2.7 (t, J=4.5 Hz, 2H), 2.37 (s, 3H, $CH_3$), 1.68 (t, J=4.0 Hz, 2H), 1.66 (s, 4H), 1.3 (s, 18H).

**EXAMPLE 23**

Preparation of compounds **114a** according to Scheme I

**Ethyl (2E,4E,6E)-3-methyl-6-[1,2,3,4,7,8,9,10-octahydro-1,1,4,4-tetramethyl-6H-naphthocycloheptene-10-yl]hexa-2,4,6-trienoate** (structure **7** where $R_1$, $R_2$, $R_3$, $R_4$ and $R_{19}$ are methyl, $R_5$, $R_6$, $R_7$, $R_8$, $R_9$, and $R_{10}$ are hydrogen; $R_{15}$ is ethoxy; X, Y and Z are carbon; m=1 and n =2).

The title ester was prepared from 1-benzosuberone (Aldrich) according to Scheme I following the representative procedure for compounds **59a** and **59b** as outlined in Examples 14 and 15. Compound **114a** had $R_f$= 0.5 (5% EtOAc-hexanes); $^1$H-NMR (400 MHz, $CDCl_3$) $\delta$ 7.11 (s, 1H, ArH), 7.00 (s, 1H, ArH), 6.99 (m, 1H, olefinic), 6.33 (d, J=16 Hz,1H, olefinic), 6.21 (d, J=16 Hz,1H, olefinic), 5.80 (s, 1H, olefinic), 4.20 (m, 2H, $-OCH_2CH_3$), 2.67 (d, J=16 Hz, 4H, $2CH_2$), 2.39 (s, 3H, $CH_3$), 1.78 (s, 4H, $2CH_2$), 1.68 (s, 4H, $2CH_2$), 1.33 (m, 15H, $4CH_3$; $-CH_2CH_3$).

**EXAMPLE 24**

Preparation of compound **114b** according to Scheme I

**(2E,4E,6E)-3-Methyl-6-[1,2,3,4,7,8,9,10-octahydro-1,1,4,4-tetramethyl-6H-naphthocyclohepten-10-yl]hexa-2,4,6-trienoic acid.** (structure **7** where $R_1$, $R_2$, $R_3$, $R_4$ and $R_{19}$ are methyl; $R_5$ $R_6$, $R_7$, $R_8$, $R_9$, and $R_{10}$, are hydrogen; $R_{15}$ is hydroxy; X, Y and Z are carbon; m=1 and n =2).

The title compound was prepared from ester **114a** following the standard hydrolysis procedure as outlined in Example 2. Compound **114b** had $R_f$=0.3 (15% EtOAc-hexanes); mp 244-246°C; $^1$H-NMR (400 MHz, $CDCl_3$) $\delta$ 7.12 (s, 1H, ArH), 7.02 (m, 1H, olefinic), 7.00 (s, 1H, ArH), 6.38 (d, J=16 Hz, 1H, olefinic), 6.25 (d, J=16 Hz, 1H, olefinic), 5.85, (s, 1H, olefinic), 2.72 (t, J=16 Hz, 2H, $CH_2$), 2.62 (t, J=16 Hz, 2H, $CH_2$), 2.40 (s, 3H, $CH_3$), 1.80 (m, 4H, $2CH_2$), 1.68 (s, 4H, $2CH_2$), 1.31 (s, 6H, $2CH_3$), 1.30 (s, 6H, $2CH_3$).

**EXAMPLE 25**

Preparation of compound **115a** according to Scheme I

**Ethyl (2Z,4E,6E)-3-methyl-6-[1,2,3,4,7,8,9,10-octahydro-1,1,4,4-tetramethyl-6H-naphthocyclohepten-10-yl]hexa-2,4,6-trienoate** (Structure **7**, where $R_1$, $R_2$, $R_3$, $R_4$ and $R_{19}$ are methyl, $R_5$, $R_6$, $R_7$, $R_8$, $R_9$, and $R_{10}$ are hydrogen; $R_{15}$ is ethoxy; X, Y and Z are carbon; m=1 and n =2).

The title compound was prepared as a by-product from the synthesis of compound **114a** (Example 23). The title compound **115a** had $R_f$=0.3 (15% EtOAc-hexanes); $^1$H-NMR (400 MHz, $CDCl_3$) δ 7.82 (d, J=16 Hz,1H, olefinic), 7.12 (s, 1H, ArH)., 7.00 (s, 1H, ArH), 6.38 (d, J=16 Hz,1H, olefinic), 5.68 (s, 1H, olefinic), 4.15 (q, J=8 Hz, 2H, -$OCH_2CH_3$), 2.65 (d, J=16 Hz, 4H, $2CH_2$), 2.28 (s, 3H, $CH_3$), 1.72 (s, 4H, $2CH_2$), 1.68 (s, 4H, $2CH_2$), 1.23 (s, 6H, $2CH_3$), 1.27 (s, 6H, $2CH_3$), 0.9 (t, J=8 Hz, 3H, -$OCH_2CH_3$).

**EXAMPLE 26**

Preparation of compound **115b** according to Scheme I

**(2Z, 4E, 6E)-3-Methyl-6-[1,2,3,4,7,8,9,10-octahydro-1,1,4,4-tetramethyl-6H-naphthocycloheptene-10-yl]hexa-2,4,6-trienoic acid** (structure **7**, where $R_1$, $R_2$, $R_3$, $R_4$, and $R_{19}$ are methyl; $R_5$, $R_6$, $R_7$, $R_8$, $R_9$ and $R_{10}$ are hydrogen; $R_{15}$ is ethoxy; X, Y and Z are carbon; m = 1 and n = 2).

The title acid was prepared from ester **115a** using the hydrolysis procedure outlined in Example 2. Compound **115b** had $R_f$=0.5 (10% MeOH-$CHCl_2$) ; $^1$H-NMR (400 MHz, $CDCl_3$) δ 7.76 (d, J=16 Hz,1H, olefinic), 7.11 (s, 1H, ArH)., 7.04 (m,1H, olefinic), 6.99 (s, 1H, ArH), 6.32 (d, J=16 Hz, 1H, olefinic), 5.69 (s, 1H, olefinic), 2.65 (d, J=16 Hz, 4H, $2CH_2$), 2.10 (s, 3H, $CH_3$), 1.65 (s, 4H, $2CH_2$), 1.27 (s, 6H, $2CH_3$), 1.31 (s, 6H, $2CH_3$).

**EXAMPLE 27**

Preparation of compound **116b** according to Scheme I

**(2E,4E)-3-Methyl-6-[(E)-3,5,6,7-tetrahydro-5,5,7,7-tetramethyl-2H-5-indacen-1-ylidene]hexa-2,4-dienoic acid** (structure **7**, where $R_1$, $R_2$, $R_3$, $R_4$, and $R_{19}$ are methyl; $R_5$, $R_6$, $R_7$, $R_8$, $R_9$ and $R_{10}$ are hydrogen; $R_{15}$ is hydroxy; X, Y and Z are carbon; m = n = 0).

The title acid was prepared from 5,5,7,7-tetramethyl-3,5,6,7-tetrahydro-2H-5-indacen-1 one in a manner similar to that of compound **59b** in Example 14. Compound **116b** had $R_f$=0.36 (50% ether in hexane), amorphous material; $^1$H NMR(400 MHz, $CDCl_3$) δ: 7.27 (s, 1H, ArH), 7.02 (s, 1H, ArH), 6.92 (dd, J=15.1, 12.5 Hz, 1H, olefinic), 6.68 (a, J=12.5 Hz, 1H, olefinic), 6.35 (d, J=15.1 Hz, 1H, olefinic), 5.89 (s, 1H, olefinic), 3.02 (m, 2H, $CH_2$), 2.98 (m, 2H, $CH_2$), 2.38 (s, 3H, $CH_3$), 1.94 (s, 2H, $CH_2$), 1.32 (s, 6H, $2CH_3$), 1.30 (s, 6H, $2CH_3$).

**EXAMPLE 28**

Preparation of compound **117a** according to Scheme I

**Ethyl (2E,4E)-3-methyl-6-[(E)-3,4,5,6,7,8-hexahydro-10-nitro-5,5,8,8-tetramethyl-2H-anthracen-1-ylidene]hexa-2,4-dienoate** (structure **7**, where $R_1$, $R_2$, $R_3$, $R_4$, and $R_{19}$ are methyl; $R_5$, $R_6$, $R_7$, $R_8$, and $R_{10}$ are hydrogen; $R_9$ is nitro; $R_{15}$ is ethoxy, X, Y, Z are carbon, m =n = 1).

The title compound was prepared from 10-nitro-1,2,3,4,5,6,7,8-octahydro-5,5,8,8-tetramethylanthracen-1-one (of Example 8) using a procedure similar to that described in Example 1. Compound **117a** was obtained as a yellow oil: $^1$H NMR (400 MHz, $CDCl_3$) δ 7.72 (s, 1H, aromatic), 7.01 (dd, J=15.1, 11.2 Hz, 1H, olefinic), 6.72 (d, J=11.2 Hz, 1H, olefinic), 6.46 (d, J=15.1 Hz, 1H, olefinic), 5.84 (s, 1H, olefinic), 4.19 (q, J=7.1 Hz, 2H, -$OCH_2$), 2.67 (br t, J=5.6 Hz, 2H, $CH_2$), 2.55 (t, J=6.3 Hz, 2H, $CH_2$), 2.37 (d, J=1.0 Hz, 3H, $CH_3$ allylic), 1.85 (app quintet, J=6.3 Hz, 2H, $CH_2$), 1.75 (m, 2H, $CH_2$), 1.67 (m, 2H, $CH_2$), 1.35 (s, 6H, $2CH_3$), 1.32 (s, 6H, $2CH_3$), 1.30 (t, J=7.1 Hz, 3H, $CH_3$ ethyl).

**EXAMPLE 29**

Preparation of compound **117b** according to Scheme 1

**anthracen-1-ylidene]hexa-2,4-dienoic acid** (structure **7**, where $R_1$, $R_2$, $R_3$, $R_4$, and $R_{19}$ **(2E,4E)-3-Methyl-6-[(E)-3,4,5,6,7,8-hexahydro-10-nitro-5,5,8,8-tetramethyl-2H-**are methyl; $R_5$, $R_6$, $R_7$, $R_8$, and $R_{10}$ are hydrogen; $R_9$ is nitro; $R_{15}$ is hydroxy, X, Y, Z are carbon, m = n = 1).

The title acid was prepared from compound **117a** using standard hydrolysis conditions outlined in Example 2. Compound **117b** was obtained as a yellow solid (75% yield): $^1H$ NMR (400 MHz, $CDCl_3$) δ 7.73 (s, 1H, aromatic), 7.02 (br t, 1H, olefinic), 6.70 (d, J=11.1 Hz, 1H, olefinic), 6.49 (d, J=14.9 Hz, 1H, olefinic), 5.86 (s, 1H, olefinic), 2.68 (br t, 2H, $CH_2$), 2.55 (br t, 2H, $CH_2$), 2.36 (s, 3H, $CH_3$ allylic), 1.86 (m, 2H, $CH_2$), 1.74 (m, 2H, $CH_2$), 1.69 (m, 2H, $CH_2$), 1.35 (s, 6H, $2CH_3$), 1.32 (s, 6H, $2CH_3$).

**EXAMPLE 30**

Preparation of compound **118b** according to Scheme I

**(2Z,4E)-3-Methyl-6-[(E)-3,4,5,6,7,8-hexahydro-10-nitro-5,5,8,8-tetramethyl-2H-anthracen-1-ylidene]hexa-2,4-dienoic acid** (structure **7**, where $R_1$, $R_2$, $R_3$, $R_4$, and $R_{19}$ are methyl; $R_5$, $R_6$, $R_7$, $R_8$, and $R_{10}$ are hydrogen; $R_9$ is nitro; $R_{15}$ is hydroxy, X, Y, Z are carbon, m = n = 1).

Obtained as a by-product from synthesis of compound **117a** (Example 28) after hydrolysis of the ester using conditions outlined in Example 2. Compound **118b** had $^1H$ NMR (400 MHz, $CDCl_3$) δ 7.91 (d, J=15.2 Hz, 1H, olefinic), 7.74 (s, 1H, aromatic), 7.02 (br t, 1H, olefinic), 6.81 (d, J=11.4 Hz, 1H, olefinic), 5.78 (br s, 1H, olefinic), 2.67 (br t, J=6.2 Hz, 2H, $CH_2$), 2.55 (t, J=6.2 Hz, 2H, $CH_2$), 2.11 (s, 3H, $CH_3$ allylic), 1.85 (m, 2H, $CH_2$), 1.74 (m, 2H, $CH_2$), 1.68 (m, 2H, $CH_2$), 1.35 (s, 6H, $2CH_3$), 1.32 (s, 6H, $2CH_3$).

**EXAMPLE 31**

Preparation of compound **119b** according to Scheme I and Scheme IV

**(2E,4E)-3-Methyl-6-[(E)-2,3,6,7,8,9-hexahydro-6,6,9,9-tetramethylbenzo[g]chromen-4-ylidene]hexa-2,4-dienoic acid** (structure **7**, where $R_1$, $R_2$, $R_3$, $R_4$, and $R_{19}$ are methyl; $R_5$, $R_6$, $R_7$, $R_8$, $R_9$, $R_{10}$ are hydrogen; $R_{15}$ is hydroxy; X and Y are carbon; Z is oxygen; m = n = 1).

In a manner similar to that described in Example 7 above, an isomeric mixture of (E, Z)-(chromen-4-ylidene)acetonitrile (550mg, 3.5 mmol) was converted to (E)-(2,3,6,7,8,9-hexahydro-6,6,9,9-tetramethylbenzo[g]chromen-4-ylidene)acetonitrile. Flash chromatography (20% EtOAc/hexanes) of the crude brown solid gave the tricyclic nitrile as a light yellow solid (511 mg 1.82 mmol, 52%): $^1H$ NMR (400 MHz, $CDCl_3$) δ 7.4 (s, 1H), 6.82 (s, 1H), 5.7 (s, 1H), 4.25 (t, 2H), 3.0 (t, 2H), 1.67 (s, 4H), 1.26 (s, 6H), 1.24 (s, 6H).

The tricyclic nitrile (1.85 g, 6.6 mmol) was then converted to (E)-(2,3,6,7,8,9-hexahydro-6,6,9,9-tetramethylbenzo[g]chromen-4-ylidene)acetaldehyde using $CH_2Cl_2$ as solvent in place of THF. The crude brown oil was purified by flash chromatography (20% EtOAc/hexane) to give the aldehyde as a yellow/orange solid (640 mg, 2.3 mmol, 40%): $^1H$ NMR (400 MHz, $CDCl_3$) δ 10.12 (d, 1H), 7.6 (s, 1H), 6.82 (s, 1H), 6.55 (d, 1H), 4.30 (t, 2H), 3.25 (t, 2H), 1.68 (s, 4H), 1.25 (s, 12H).

The tricyclic aldehyde (640 mg, 2.2 mmol) was then transformed to a mixture of ethyl (2E,4E)- and (2Z,4E)-3-methyl-6-[(E) 2,3,6,7,8,9-hexahydro-6,6,9,9-tetramethylbenzo[g]chromen-4-ylidene]hexa-2,4-dienoates. NaH was used in place of n-BuLi and 2 equivalents of phosphonate and base were used. The aldehyde was added at 0°C and warmed to 15°C over 1 h. Aqueous work up (and $Et_2O$ extraction) gave the esters as a yellow oil (820 mg, 2.08 mmol, 93%). Flash chromatography (8% EtOAc/hexane) gave the *trans* -isomer as a yellow solid (191 mg): $^1H$ NMR (400 MHz, $CDCl_3$) δ 7.55 (s, 1H), 6.95 (dd, 1H), 6.8 (s, 1H), 6.72 (d, 1H), 6.42 (d, 1H), 5.80 (s, 1H), 4.20 (m, 4H), 2.8 (t, 2H), 2.36 (s, 3H), 1.68 (s, 4H), 1.3 (t, 3H), 1.27 (s, 12H).

Using the procedure of Example 7, the above ester (191 mg, 0.48 mmol) was converted into the title acid. Compound **119b** was obtained as a yellow solid (168 mg, 45 mmol, 96%): TLC $R_f$=0.20 (40% $Et_2O$/Hexane); mp 198.2°C; $^1H$ NMR (400 MHz, $CDCl_3$) δ 7.55 (s, 1H), 6.95 (dd, 1H), 6.8 (s, 1H), 6.72 (d, 1H), 6.42 (d, 1H), 5.80 (s, 1H), 4.20(m, 2H), 2.8 (t, 2H), 2.36 (s, 3H), 1.68 (s, 4H), 1.27 (s, 12H).

## EXAMPLE 32

Preparation of compound **120b** according to Schemes II and IV

**(2Z,4E)-3-Methyl-6-[(E)-2,3,6,7,8,9-hexahydro-6,6,9,9-tetramethylbenzo[g]chromen-4-ylidene]hexa-2,4-dienoic acid** (structure **7**, where $R_1$, $R_2$, $R_3$, $R_4$, and $R_{19}$ are methyl; $R_5$, $R_6$, $R_7$, $R_8$, $R_9$, $R_{10}$ are hydrogen; $R_{15}$ is hydroxy; X and Y are carbon; Z is oxygen; m = n = 1).

The isomeric mixture of esters described above in Example 31 was purified by flash chromatography (8% EtOAc/hexane) to give ethyl(2Z,4E)-3-methyl-6-[(E) 2,3,6,7,8,9-hexahydro-6,6,9,9-tetramethyl-benzo[g]chromen-4-ylidene]hexa-2,4-dienoate as a yellow oil (131 mg): $^1$H NMR (400 MHz, $CDCl_3$) δ 7.95 (d, 1H), 7.58 (s, 1H), 6.95 (dd, 1H), 6.85(d, 1H), 6.80 (s, 1H), 5.67 (s, 1H), 4.20 (m, 4H), 2.8 (t, 2H), 2.10 (s, 3H), 1.68 (s, 4 H), 1.3 (t, 3H), 1.27 (s, 12H).

Employing the procedure described in Example 2, the above ester (131 mg, 0.33 mmol) was converted to the title acid. Acid **120b** was obtained as a yellow oily solid (108mg, 29.7 mmol, 90%): TLC $R_f$=0.28 (40% $Et_2O$/Hexane); $^1$H NMR (400 MHz, $CDCl_3$) δ 7.95 (d, 1H), 7.58 (s, 1H), 6.95 (dd, 1H), 6.85 (d, 1H), 6.80 (s, 1H), 5.67 (s, 1H), 4.20 (m, 2H), 2.8 (t, 2H), 2.10 (s, 3H), 1.68 (s, 4H), 1.27 (s, 12H).

## EXAMPLE 33

Preparation of compound **121b** according to Schemes I and II

**(2E,4E)-3-Methyl-6-[(E)-2,3,6,7,8,9-hexahydro-2,2,6,6,9,9-hexamethylbenzo[g]chromen-4-ylidene]hexa-2,4-dienoic acid** (structure **7**, where $R_1$, $R_2$, $R_3$, $R_4$, $R_7$, $R_8$, and $R_{19}$ are methyl; $R_5$, $R_6$, $R_9$, $R_{10}$ are hydrogen; $R_{15}$ is hydroxy; X and Y are carbon; Z is oxygen; m = n = 1).

Using the procedure described in Example 31, 2-[(E)-3,4,5,6,7,8-hexahydro-2,2,5,5,8,8-hexamethylnaphtho-(2,3-b)-1,2-pyran-4-ylidene]ethanitrile (500 mg, 1.6 mmol) was reduced with DIBAL in $CH_2Cl_2$ to give the corresponding aldehyde. Aqueous work up gave 2-(E)-[3,4,5,6,7,8-hexahydro-2,2,5,5,8,8-hexamethylnaphtho-(2,3-b)-1,2-pyran-4-ylidene]acetaldehyde which was purified by flash chromatography (8% EtOAc/hexane) to afford a light yellow solid (220mg, 0.71 mmol, 45% yield): $^1$H NMR (400 MHz, $CDCl_3$) δ 10.1 (d, 1H), 7.52 (s, 1H), 6.8 (s, 1H), 6.60 (d, 1H), 3.05 (s, 2H), 1.70 (s, 4H), 1.4 (s, 6H), 1.25 (s, 12H).

The above aldehyde was convened to the corresponding triene ester in a manner similar to that described in Example 23. Aqueous work up gave a yellow oil which partially solidified on standing. Flash chromatography (20% EtOAc/hexane) afforded ethyl (2E,4E)-3-methyl-6-[(E)-5,6,7,8-tetrahydro-5,5,8,8-tetramethyl naphthyl (2,3-b ) 2,2-dimethylpyran-4-yl]hexa-2,4-dienoate as a bright yellow solid (115 mg, 0.27 mmol , 83% yield): $^1$H NMR (400 MHz, $CDCl_3$) δ 7.50 (s, 1H), 6.95 (dd, 1H), 6.75 (m, 2H), 6.45 (d, 1H), 5.8 (s, 1H), 4.10 (m, 2H), 2.66 (s, 2H), 2.39 (s, 3H), 1.65 (s, 4H), 1.33 (s, 6H), 1.31 (t, 3H), 1.30 (s, 6H), 1.25 (s, 6H).

The above ester (60.5mg, 0.143 mmol) was hydrolyzed to the corresponding acid using the procedure described in Example 2 to give the title compound as a bright yellow solid (50mg, 0.127 mmol , 88%): TLC Rf=0.10 (20% EtoAc/Hexane); mp 224°C; $^1$H NMR (400 MHz, $CDCl_3$) δ 7.50 (s, 1H), 7.02 (dd, 1H), 6.79 (d, 1H), 6.75(s, 1H), 6.50 (d, 1H), 5.87 (s, 1H), 2.66 (s, 2H), 2.40(s, 3H), 1.68(4s, H), 1.38(s, 6H), 1.30 (s, 6H), 1.25 (s, 6H).

## EXAMPLE 34

Preparation of compound **122a** according to Scheme V

**Ethyl (2E,4E)-3-methyl-6-(3,4,5,6,7,8-hexahydro-5,5,8,8-tetramethylanthracen-1-yl)hexa-2,4-dienoate** (structure **20**, where $R_1$, $R_2$, $R_3$, $R_4$, and $R_{19}$ are methyl; $R_5$, $R_7$, $R_8$, $R_9$, and $R_{10}$ are hydrogen; $R_{15}$ is ethoxy; X, Y, and Z are carbon; m = n = 1).

To a solution of trimethyl phosphonoacetate (5.68 g, 31 mmol) in THF (15 ml) was added dropwise nBuLi (9.3 ml, 2.5 M, 23 mM) at 0°C, and the resulting solution was stirred at that temperature for 30 min. 3,4,5,6,7,8-hexahydro-5,5,8,8-tetramethyl-2H-anthracen-1-one (2.0 g, 7.8 mmol) in THF (10 ml) was added at 25°C, and the mixture was stirred at that temperature for 30 min. Standard work-up procedures as detailed in Example 1, followed by purification of the product by column chromatography (10 % ether in hexane) gave methyl (3,4,5,6,7,8-hexahydro-5,5,8,8-tetramethylanthracen-1-yl)acetate (1.33 g, 55%): $^1$H NMR(400 MHz, $CDCl_3$) δ 7.15 (s, 1H, ArH), 7.04 (s, 1H, ArH), 5.94 (br t, 1H, olefinic), 3.68 (s, 2H, $OCH_3$), 3.43 (s, 2H, $CH_2$), 2.74 (t, J=7.9 Hz, 2H, $CH_2$, benzylic), 2.31 (m, $CH_2$, 2H, allylic), 1.66 (s, 4H, $2CH_2$), 1.27 (s, 12H, $4CH_3$).

To a solution of the above methyl ester (312 mg, 1 mmol) in $CH_2Cl_2$ (5 ml) was added dropwise DIBAL (1 ml, 1 M, 1.0 mM) at -78°C. The resulting mixture was stirred at -78°C for 30 min, then warmed to room temperature, followed by standard work-up procedure as described in Example 1. Purification of the corresponding aldehyde by chromatography (10 % ether in hexane) gave pure (3,4,5,6,7,8-hexahydro-5,5,8,8-teramethylanthracen-1-yl)-acetaldehyde (140 mg, 50 %): $^1$H NMR(400 MHz, $CDCl_3$) δ 9.64 (t, J=2.6 Hz, 1H, CHO), 7.07 (s, 1H, ArH), 7.03 (s, 1H, ArH), 5.98 (br t, 1H, olefinic), 3.44 (s, 2H, $CH_2$), 2.77 (t, J=7.9 Hz, 2H, $CH_2$, benzylic), 2.35 (m, 2H, $CH_2$, allylic), 1.66 (s, 4H, $2CH_2$), 1.27 (s, 6H, $2CH_3$), 1.26 (s, 6H, $2CH_3$).

The above aldehyde was coupled with the anoin of diethyl 3-ethoxycarbonyl-2-methylprop-2-enylphosphonate, as described for compound **51a** in Example 1, to give the title compound **122a**: $R_f$=0.25 (10% ether in hexane) $^1$H NMR(400 MHz, $CDCl_3$) δ 7.15 (s, 1H, ArH), 7.09 (s, 1H, ArH), 6.07 (m, 2H, olefinic), 5.85 (s, 1H, olefinic), 5.65 (br t, 1H, olefinic), 4.02 (q, 2H, $OCH_2$), 3.12 (br d, 2H, $CH_2$, allylic), 2.61 (t, J=7.8 Hz, 2H, $CH_2$, benzylic), 2.30 (s, 3H, $CH_3$), 2.09 (m, 2H, $CH_2$, allylic), 1.60 (s, 4H, $2CH_2$), 1.27 (s, 6H, $2CH_3$), 1.24 (s, 6H, $2CH_3$), 0.98 (t, J=7.0 Hz, 3H, $CH_3$).

## EXAMPLE 35

Preparation of compound **122b** according to Scheme V

**(2E,4E)-3-Methyl-6-(3,4,5,6,7,8-hexahydro-5,5,8,8-tetramethylanthracen-1-yl)-hexa-2,4-dienoic acid** (structure **20a**, where $R_1$, $R_2$, $R_3$, $R_4$, and $R_{19}$ are methyl; $R_5$, $R_7$, $R_8$, $R_9$, and $R_{10}$ are hydrogen; $R_{15}$ is hydroxy; X, Y, and Z are carbon; m = n =1).

The title compound was prepared by hydrolysis of compound **122a** using the standard conditions described in Example 2. Compound **122b** had mp 171-173°C, $^1$H NMR (400 MHz, $CDCl_3$) δ 7.15 (s, 1H, ArH), 7.06 (s, 1H, ArH), 6.26 (m, 2H, olefinic), 5.81 (brt, 1H, olefinic), 5.74 (s, 1H, olefinic), 3.30 (br s, 2H, $CH_2$, allylic), 2.72 (t, J=8.0 Hz, 2H, $CH_2$, benzylic), 2.27 (m, 2H, $CH_2$, allylic), 2.26 (s, 3H, $CH_3$), 1.66 (s, 4H, $2CH_2$), 1.25 (s, 6H, $2CH_3$), 1.24 (s, 6H, $2CH_3$).

## EXAMPLE 36

Preparation of compound **123b** according to Scheme V

**(3E,5E)-3-Methyl-6-(3,4,5,6,7,8-hexahydro-5,5,8,8-tetramethylanthracen-1-yl)hexa-3,5-dienoic acid** (structure **20b**, where $R_1$, $R_2$, $R_3$, $R_4$, and $R_{19}$ are methyl; $R_5$, $R_6$, $R_7$, $R_8$, $R_9$, and $R_{10}$ are hydrogen; $R_{15}$ is hydroxy; X, Y, and Z are carbon; m= n=1)

The title compound was prepared as a by-product from the hydrolysis of compound **122a** of Examples 34 and 35. Compound **123b** had $R_f$=0.30 (50% ether in hexane); mp 183-185°C; $^1$H NMR: (400 MHz, $CDCl_3$) δ 7.32 (s, 1H, ArH), 7.10 (s, 1H, ArH), 7.07 (d, J=10.8 Hz, 1H, olefinic), 6.78 (dd, J=15.8, 10.8 Hz, 1H, olefinic), 6.43 (d, J=15.8Hz, 1H, olefinic), 6.15 (br t, 1H, olefinic), 3.15 (s, 2H, $CH_2$, α-methylene), 2.69 (t, J=7.5 Hz, 2H, $CH_2$, benzylic), 2.30 (m, 2H, $CH_2$, allylic), 1.90 (s, 3H, $CH_3$), 1.68 (s, 4H, $2CH_2$), 1.3 0(s, 6H, $2CH_3$), 1.29 (s, 6H, $2CH_3$).

## EXAMPLE 37

Preparation of compound **124a** according to Scheme VI

**Ethyl (2E,4E)-3-methyl-6-(1,2,3,4,5,6,7,8-octrahydro-5,5,8,8-tetramethylanthracen-1-yl)-hexa-2,4-dienoate** (structure **22**, where $R_1$, $R_2$, $R_3$, $R_4$, and $R_{19}$ are methyl; $R_5$, $R_6$, $R_7$, $R_8$, $R_9$, and $R_{10}$ are hydrogen; $R_{15}$ is ethoxy; X, Y, and Z are carbon; m = n=1).

To a solution of [(E)-3,4,5,6,7,8,9-hexahydro-5,5,8,8-tetramethyl-2H-anthracen-1-ylidene]ethanitrile (56 mg, 0.2 mmol from Example 1) in EtOAc (5 ml) was added 5 mg of 10% Pd-C catalyst, and the reaction mixture was treated with hydrogen gas at 1 atm for 3 h. Removal of the catalyst by filtration, and concentration of organic solvent gave essentially pure saturated nitrile which was used for the next reaction without further purification. The saturated nitrile was then sequentially subjected to DIBAL reduction and Wittig coupling reaction using conditions similar to those described in Example 1 to afford, after column chromatography, the title compound **124a**: $R_f$=0.55(10 % ether in hexane); $^1$H NMR(400 MHz, $CDCl_3$) δ 7.09 (s, 1H, ArH), 6.98 (s, 1H, ArH), 6.20 (m, 1H, olefinic), 6.12 (d, J=15.7 Hz, 1H, olefinic), 5.70 (s, 1H, olefinic), 4.13 (q, 2H, $OCH_2$), 2.85 (m, 1H, CH), 2.79 (br t, 2H, $CH_2$, benzylic), 2.60 (m, 1H, CH, allylic), 2.37 (m, 1H, CH, allylic), 2.30 (s, 3H, $CH_3$), 1.80 (m, 2H, $CH_2$, methylene), 1.65 (m, 2H, $CH_2$, methylene), 1.63 (s, 6H, $2CH_2$), 1.27 (s, 6H, $2CH_3$), 1.26 (s, 6H, $2CH_3$), 1.26 (t, J=6.8 Hz, 3H, $CH_3$).

**EXAMPLE 38**

Preparation of compound **124b** according to Scheme VI

**(2E,4E)-3-Methyl-6-(1,2,3,4,5,6,7,8-octahydro-5,5,8,8-tetramethylanthracen-1-yl)hexa-2,4-dienoic acid** (structure **22**, where $R_1$, $R_2$, $R_3$, $R_4$, and $R_{19}$ are methyl; $R_5$, $R_6$, $R_7$, $R_8$, $R_9$, and $R_{10}$ are hydrogen; $R_{15}$ is hydroxy; X, Y, and Z are carbon; m = n = 1).

Compound **124a** was subjected to hydrolysis using the standard conditions of Example 2 to obtain the title acid **124b**: $R_f$=0.48(50 % ether in hexane); mp 178-179°C; $^1$NMR(400 MHz, $CDCl_3$) δ 7.07 (s, 1H, ArH), 6.98 (s, 1H, ArH). 6.23 (m, 1H, olefinic), 6.15 (d, J=15.6 Hz, 1H, olefinic), 5.72 (s, 1H, olefinic), 2.86 (m, 1H), 2.70 (br t, 2H, $CH_2$, benzylic), 2.60 (m, 1H, allylic), 2.42 (m, 1H, allylic), 2.30 (s, 3H, $CH_3$), 1.80 (m, 2H, $CH_2$, methylene), 1.68 (m, 2H, $CH_2$, methylene), 1.64 (s, 4H, $2CH_2$), 1.25 (s, 6H, $2CH_3$), 1.24 (s, 6H, $2CH_3$).

**EXAMPLE 39**

Preparation of compound **125a** according to Scheme VI

**Ethyl (2E,4E)-3-methyl-6-(1,2,3,5,6,7,8-heptahydro-5,5,8,8-tetramethylcyclopenta[b]napthalen-1-yl)hexa-2,4-dienoate** (structure **22**, where $R_1$, $R_2$, $R_3$, $R_4$, and $R_{19}$ are methyl, $R_5$, $R_6$, $R_7$, $R_8$, $R_9$, $R_{10}$ are hydrogen, $R_{15}$ is ethoxy, X, Y, Z are carbon, m = 1, and n = 0).

The title compound was prepared in a manner similar to that of compound **124a** except that 2-[(E)-2,3,5,6,7,8-hexahydro-5,5,8,8-tetramethyl-cyclopenta[b]naphthalen-1-ylidene]ethanitrile was employed as the starting material. The starting nitrile was prepared by condensation of 2,3,5,6,7,8 hexahydro-5,5,8,8-tetramethyl-cyclopenta[b]indan-1-one (US patent 2,815,382 (1957)) with the sodium anion of cyanomethylphosphonate. Title compound **125a** had $R_f$=0.50 (10% ether in hexane); $^1$H NMR(400 MHz, $CDCl_3$) δ 7.15 (s, 1H, ArH), 7.12 (s, 1H, ArH), 6.20 (m, 1H, olefinic), 6.15 (d, J=15.7 HZ, 1H, olefinic), 5.69 (s, 1H, olefinic), 4.14 (q, 2H, $OCH_2$), 3.15 (m, 1H, CH), 2.85 (m, 1H, benzylic), 2.75 (m, 1H, benzylic), 2.65 (m, 1H, allylic), 2.32 (m, 1H, allylic), 2.27 (s, 3H, $CH_3$), 2.20 (m, 1H, methylene), 1.69 (m, 1H, methylene), 1.65 (s, 4H, $2CH_2$), 1.26 (s, 6H, $2CH_3$), 1.24 (m, 9H, $3CH_3$).

**EXAMPLE 40**

Preparation of compound **125b** according to Scheme VI

**(2E,4E)-3-Methyl-6-1,2,3,5,6,7,8-heptahydro-5,5,8,8-tetramethylcyclopenta[b]naphthalen-1-yl]hexa-2,4-dienoic acid** (structure **22**, where $R_1$, $R_2$, $R_3$, $R_4$, and $R_{19}$ are methyl; $R_5$, $R_6$, $R_7$, $R_8$, $R_9$, and $R_{10}$ are hydrogen; $R_{15}$ is hydroxy; X, Y, and Z are carbon; m = 1, and n = 0).

The title compound was prepared by hydrolysis of compound **125a** using the standard condition of Example 2. Compound **125b** had $R_f$=0.48(50 % ether in hexane); mp 169-170°C; $^1$H NMR(400 MHz, $CDCl_3$) δ 7.16 (s, 1H, ArH), 7.12 (s, 1H, ArH), 6.24 (m, 1H, olefinic), 6.18 (d, J=15.7 Hz, 1H, olefinic), 5.72 (s, 1H, olefinic), 3.18 (m, 1H, methine), 2.85 (m, 1H, benzylic), 2.78 (m, 1H, benzylic), 2.65 (m, 1H, allylic), 2.33 (m, 1H, allylic), 2.29 (s, 3H, $CH_3$), 2.17 (m, 1H, methylene), 1.70 (m, 1H, methylene), 1.65 (s, 4H, $2CH_2$), 1.27 (s, 6H, $2CH_3$), 1.25 (s, 6H, $2CH_3$).

**EXAMPLE 41**

Preparation of compound **126a** according to Scheme VI

**Ethyl (2E,4E)-3-methyl-6-[1,2,3,4,7,8,9,10-octahydro-1,1,4,4-tetramethyl-6H-naphthocycloheptan-10-yl]hexa-2,4-dienoate** (structure **22** where $R_1$, $R_2$, $R_3$, $R_4$ and $R_{19}$ are methyl; $R_5$, $R_6$, $R_7$, $R_8$, $R_9$, and $R_{10}$, are hydrogen; $R_{15}$ is ethoxy; X, Y and Z are carbon; m=1 and n =2)

To 11.0 g of diethyl cyanomethylphosphonate in 16 mL of dry THF was added 1.97 g of NaH to 25°C. The solution was stirred for 50 minutes, followed by addition of 4 g of 1-benzosuberone (Aldrich) in 5 mL of dry THF. The reaction was warmed to 80°C for 12-16 h, cooled to room temperature and poured into saturated $NH_4Cl$ solution. The products were extracted with EtOAc, washed with $H_2O$ and brine, dried over $MgSO_4$, filtered and concentrated. The crude product was purified by chromatography ($SiO_2$, 10 % EtOAc-hexanes) to give 4.1 g of (E)-2-[2-cyanomethenyl]benzosubarane: $^1$H-NMR (400 MHz, $CDCl_3$) δ 7.12-7.32 (m, 4H, ArH), 5.31 (s, 1H, olefinic), 2.75-2.78 (m, 4H, $2CH_2$), 1.90 (m,

2H, $CH_2$), 1.80 (m, 2H, $CH_2$).

To 2.1 g of (E)-2-[2-cyanomethenyl]benzosubarane in 20 mL of $CH_2Cl_2$ and 3.15 g of dichlorodimethylhexane at 25° was added 3.06 g of aluminum trichloride. The mixture was stirred for 10 minutes, poured into $H_2O$, and the product was extracted with 20% EtOAc-hexanes. The organic layer was washed with $H_2O$ and brine, dried over $MgSO_4$, filtered and concentrated. Purification by chromatography ($SiO_2$ hexanes) gave 1.8 g of pure 2-[1,1,4,4-tetramethyl-1,2,3,4,7,8,9,10-octahydro-6H-naphthocycloheptan-10-yl]cyanomethylene: $^1$H-NMR (400 MHz, $CDCl_3$) δ 7.02 (s, 1H, ArH), 7.01 (s, 1H, ArH), 5.30 (s, 1H, CH), 2.78 (t, J=12 Hz, 2H, $CH_2$), 2.70 (t, J=12 Hz, 2H, $CH_2$), 1.85 (m, 2H, $CH_2$), 1.75 (m, 2H, $CH_2$), 1.60 (s, 4H, $2CH_2$), 1.27 (s, 6H, $2CH_3$), 1.23 (s, 6H, $2CH_3$).

To 614 mg (2.10 mmol) of the above nitrile in $CH_2Cl_2$ (8 ml) at -78°C was added 1.68 mL (1.5 molar solution) of DIBAL. The mixture was stirred for 20 minutes at -78°C, then poured onto potassium-sodium tartrate solution, acidified with 1N HCl, and the product extracted with ether. The ether layer was washed with $H_2O$, brine, dried over $MgSO_4$, filtered and concentrated. The crude product was purified by silica gel flash chromatography (5% EtOAc-hexanes) to give 412 mg of [1,2,3,4,7,8,9,10-octahydro-1,1,4,4-tetramethyl-6H-naphthocycloheptan-10-yl]ethenal: $^1$H-NMR (400 MHz, $CDCl_3$) δ 10.12 (d, J=12 Hz, 1H, CHO), 7.12 (s, 1H, ArH), 7.00 (s, 1H, ArH), 6.10 (d, J=12 Hz, 1H, olefinic), 2.92 (t, J=16 Hz, 2H, $CH_2$), 2.72 (t, J=16 Hz, 2H, $CH_2$), 1.87 (m, 2H, $CH_2$), 1.80 (m, 2H, $CH_2$), 1.68 (s, 4H, $2CH_2$), 1.27 (s, 6H, $2CH_3$), 1.23 (s, 6H, $2CH_3$).

To 150 mg of aldehyde in EtOAc (3 ml) was added 15 mg of 10% Pd/C, and the mixture was stirred under one atmosphere of $H_2$ gas for 2 hours, then filtered through a silica gel plug, and concentrated to give 140 mg of (1,2,3,4,7,8,9,10-octahydro-1,1,4,4-tetramethyl-6H-naphthocycloheptan-10-yl)ethanal: $R_f$= 0.4 (10% EtOAc-hexanes); $^1$H-NMR (400 MHz, $CDCl_3$) δ 9.80 (s, 1H, CHO), 6.95 (s, 1H, ArH), 6.90 (s, 1H, ArH), 3.70 (m, 2H, $CH_2$), 3.50 (m, 1H, CH), 3.00 (m, 2H, $CH_2$), 2.6-2.8 (m, 2H, $CH_2$), 1.80 (m, 4H, $2CH_2$), 1.60 (s, 4H, $2CH_2$), 1.32 (s, 6H, $2CH_3$), 1.28 (s, 6H, $2CH_3$).

To 372 mg of triethyl 3-methyl-4-phosphonocrotonate in 14 mL of dry 2:1 anhydrous THF-DMPU at -78°C was added 0.57 mL of a 2.5 M nBuLi solution in THF. The reaction was stirred for 10 minutes at -78°C, followed by addition of 140 mg of the above (1,1,4,4-tetramethyl- 1,2,3,4,7,8,9,10-octahydro-6H-naphthocycloheptene-10-yl)ethanal in 3 mL of dry THF. The reaction was stirred for an additional 10 minutes at -78°C, then warmed to room temperature. The reaction was poured into saturated $NH_4Cl$ and the product extracted with ether. The ether layer was washed with $H_2O$ and brine, dried over $MgSO_4$, filtered and concentrated. The crude product was purified by preparative TLC ($SiO_2$, 5% EtOAc-hexanes) to give 140 mg of the title ester **126a**: $R_f$=0.6, (5% EtOAc-hexanes); $^1$H NMR (400 MHz, $CDCl_3$) δ 6.99 (s, 2H, ArH), 6.13 (m, 2H, olefinic), 5.65 (s, 1H, olefinic), 4.12 (m, 2H, -$OCH_2CH_3$), 2.91 (m, 2H, $CH_2$), 2.81 (t, J=16 Hz, 2H, $CH_2$), 2.70 (m, 1H, CH), 2.56 (m, 2H, $CH_2$), 1.68 (s, 4H, $2CH_2$), 1.20-1.30 (m, 15H, $OCH_2CH_3$ + $4CH_3$). A smaller amount (40 mg) of the corresponding Z,E-isomer was obtained as well.

## EXAMPLE 42

Preparation of compound **126b** according to Scheme VI

**(2E,4E)-3-Methyl-6-[1,1,4,4-tetramethyl-1,2,3,4,7,8,9,10-octahydro-6H-naphthocycloheptan-10-yl]hexa-2,4-dienoic acid.** (structure **22**, where $R_1$, $R_2$, $R_3$, $R_4$ and $R_{19}$ are methyl; $R_5$, $R_6$, $R_7$, $R_8$, $R_9$, and $R_{10}$, are hydrogen; $R_{15}$ is hydroxy; X, Y and Z are carbon; m=1 and n =2)

Compound **126a** (15 mg) was hydrolyzed by the standard method described in Example 2. The title compound was crystallized from EtOAc-hexanes to give 8.2 mg of **126b**: $R_f$=0.3 (15% EtOAc-hexanes); $^1$H-NMR (400 MHz, $CDCl_3$) δ 7.00 (s, 2H, ArH), 6.19 (m, 2H, olefinic), 5.70 (s, 1H, olefinic), 2.91 (m, 2H, CH), 2.80 (t, J=16 Hz, 2H, $CH_2$), 2.70 (m, 1H, CH), 2.51 (m, 2H, $CH_2$), 2.21 (s, 3H, $CH_3$), 1.70 (m, 4H, $2CH_2$), 1.61 (s, 4H, $2CH_2$), 1.20-1.22 (m, 12H, $4CH_3$).

## EXAMPLE 43

Preparation of compounds **127a** and **127b** according to Scheme VI

Ethyl **(2Z,4E)-3-methyl-6-[1,2,3,4,7,3,9,10-octahydro-1,1,4,4-tetramethyl-6H-naphthocycloheptan-10-yl]hexa-2,4-dienoate.** (structure **22**, where $R_1$, $R_2$, $R_3$, $R_4$ and $R_{19}$ are methyl; $R_5$, $R_6$, $R_7$, $R_8$, $R_9$, and $R_{10}$ are hydrogen; $R_{15}$ is ethoxy; X, Y and Z are carbon; m=1 and n =2)

Prepared as a minor by-product from Example 41. Compound **127a** had $R_f$=0.6; (5% EtOAc-hexanes); $^1$H-NMR (400 MHz, $CDCl_3$) δ 7.66 (d, J=16 Hz, 1H, olefinic), 6.98 (s, 2H, ArH), 6.13 (m, 1H, olefinic), 5.58 (s, 1H, olefinic), 4.42 (m, 2H, $OCH_2CH_3$), 2.91 (m, 2H, CH), 2.81 (t, J=8 Hz, 2H, $CH_2$), 2.74 (m, 1H, CH), 2.61 (m, 2H, $CH_2$), 2.00 (s,

3H, $CH_3$), 1.65 (m, 4H, $2CH_2$), 1.62 (s, 4H, $2CH_2$), 1.30-1.32 (m, 15H, $OCH_2CH_3 + 4CH_3$).

**(2Z,4E)-3-Methyl-6-[1,2,3,4,7,8,9,10-octahydro-1,1,4,4-tetramethyl-6H-naphthocycloheptan-10-yl]hexa-2,4-dienoic acid.** (structure **22**, where $R_1$, $R_2$, $R_3$, $R_4$ and $R_{19}$ are methyl; $R_5$, $R_6$, $R_7$, $R_8$, $R_9$, and $R_{10}$, are hydrogen; $R_{15}$ is hydroxy; X, Y and Z are carbon; m=1 and n =2)

The title compound was prepared from compound **127a** by the hydrolysis method described in Example 2. Compound **127b** had $R_f$=0.3 (15% EtOAc-hexanes); $^1$H-NMR (400 MHz, $CDCl_3$) δ 7.66 (d, J=16 Hz, 1H, olefinic), 6.99 (s, 2H, ArH), 6.25 (m, 1H, olefinic), 5.50 (s, 1H, olefinic), 2.91 (m, 2H, $CH_2$), 2.80 (m, 2H, $CH_2$), 2.70 (m, 1H, CH), 2.55 (m, 2H, $CH_2$), 2.00 (s, 3H, $CH_3$), 1.63 (m, 4H, $2CH_2$), 1.60 (s, 4H, $CH_2$), 1.20-1.22 (m, 12H, $CH_3$).

## EXAMPLE 44

Preparation of compound **128b** according to Schemes I and VI

**(2E,4E)-3-Methyl-6-[5,6,7,8-tetrahydro-5,5,8,8-tetramethylnaphthyl-(2,3-b)-2,2-dimethyl-pyran-4-yl]hexa-2,4-dienoic acid** (structure **22**, where $R_1$, $R_2$, $R_3$, $R_4$, $R_7$, $R_8$, and $R_{19}$ are methyl; $R_5$, $R_6$, $R_9$, $R_{10}$ are hydrogen; $R_{15}$ is hydroxy; X and Y are carbon; Z is oxygen; m =n = 1).

[(E)-3,4,5,6,7,8-Hexahydro-2,2,5,5,8,8-hexamethylnaphtho(2,3-b)-1,2-pyran-4-ylidene]ethanitrile (400mg, 1.30 mmol, from Example 8) was converted to the corresponding aldehyde by means of the procedure described in Example 1. Flash chromatography (10% EtOAc/hexane) gave [(E)-3,4,5,6,7,8-hexahydro-2,2,5,5,8,8-hexamethylnaphtho(2,3-b)-1,2-pyran-4-ylidene]acetaldehyde as a light yellow solid (220 mg, 0.70 mmol, 54%): $^1$H NMR (400 MHz, $CDCl_3$) δ 10.1 (d, 1H), 7.53 (s, 1H), 6.8 (s, 1H), 6.60 (d, 1H), 3.05 (s, 2H), 1.65 (s, 4H), 1.40 (s, 6H), 1.27 (s, 6H), 1.25 (s, 6H).

The above aldehyde (220 mg, 0.70 mmol) was hydrogenated using a procedure similar to that described in Example 41. TLC (20% EtOAc/hexane, cerium molybdate) indicated two products. Filtration and concentration gave a light yellow oil (140mg), which was purified by flash chromatography (20% EtOAc/hexane) to give [3,4,5,6,7,8-hexahydro-2,2,5,5,8,8-hexamethylnaphtho(2,3-b)-1,2-pyran-4-yl]acetaldehyde as a clear oil which solidified on standing (39mg, 0.12 mmol, 18% yield): $R_f$=0.43 (20% EtOAc/hexane); $^1$H NMR (400 MHz, $CDCl_3$) δ 9.9 (t, 1H), 7.00 (s, 1H), 6.7 (s, 1H), 3.45 (m, 1H), 3.05 (dd, 1H), 2.70 (dd, 1H), 2.00 (dd, 1H), 1.65 (s, 4H), 1.60 (dd, 1H), 1.31 (s, 6H), 1.26 (s, 6H), 1.24 (s, 6H).

Using the procedure described in Example 1, the above saturated aldehyde (39 mg, 0.124 mmol) was coupled with diethyl 3-ethoxycarbonyl-2-methylprop-2-enylphosphonate. A total of 1.5 equivalents of phosphonate and sodium hydride were used in place of the original conditions. Aqueous work up, followed by flash chromatography (20% EtOAc/hexane) afforded a mixture of ethyl (2E,4E)- and (2Z,4E)-3-methyl-6-[5,6,7,8-tetrahydro-5,5,8,8-tetramethylnaphthyl-(2,3-b)-2,2-dimethylpyran-4-yl]hexa-2,4-dienoate as a clear oil (38mg, 0.089 mmol, 73% yield.). $^1$H NMR indicated a 2:1 ratio of isomers favoring the *trans* product. These could not be separated by chromatography and were used as a crude mixture in the subsequent reaction: $^1$H NMR (400 MHz, $CDCl_3$) δ 7.79 (d, 1H), 7.15 (s, 2H), 6.7 (s, 2H), 6.20 (m, 1H), 6.15 (m, 2H,), 5.7 (d, 1H), 5.6 (d, 1H), 4.10 (m, 4H), 2.95 (m, 2H), 2.90 (m, 2H), 2.50 (m, 1H), 2.40 (m, 1H), 2.10 (s, 3H), 2.00 (s, 3H), 1.82 (m, 2H), 1.62 (s, 8H), 1.60 (dd, 2H), 1.31 (t, 6H), 1.29 (s, 12H ), 1.26 (s, 12H), 1.24 (s, 12H).

The above mixture of esters (38 mg, 0.089 mmol) was hydrolyzed according to the standard procedure described in Example 2. A mixture of (2E,4E)- and (2Z,4E)-3-methyl-6-[5,6,7,8-tetrahydro-5,5,8,8-tetramethylnaphthyl (2,3-b )2,2-dimethylpyran-4-yl]hexa-2,4-dienoic acid was isolated as a light yellow solid (34 mg, 0.86 mmol, 96%): $^1$H NMR indicated a 2:1 ratio of isomers favoring the *trans* product. A 5 mg sample of the crude material was purified by HPLC (MeOH / 10 mM ammonium acetate) to give the title acid (**128a**) as an oily yellow solid (2.4 mg): $^1$H NMR (400 MHz, $CDCl_3$) δ 7.15 (s, 1H), 6.7 (s, 1H), 6.22 (m, 2H), 5.75 (s, 1H), 2.90 (m, 2H), 2.40 (m, 1H), 2.30 (s, 3H), 1.82 (m, 1H ), 1.65 (s, 4H), 1.60 (dd, 1H), 1.29 (s, 6H), 1.26 (s, 6H), 1.24 (s, 6H).

**EXAMPLE 45**

Preparation of compound **129b** according to Schemes I and II

**(2Z,4E)-3-Methyl-6-[5,6,7,8-tetrahydro-5,5,8,8-tetramethylnaphthyl-(2,3-b)-2,2-dimethylpyran-4-yl]hexa-2,4-dienoic acid** (structure **22**, where $R_1$, $R_2$, $R_3$, $R_4$, $R_7$, $R_8$, and $R_{19}$ are methyl; $R_5$, $R_6$ $R_9$, $R_{10}$ are hydrogen; $R_{15}$ is hydroxy; X and Y are carbon; Z is oxygen; m = n = 1).

A mixture of (2E,4E)- and (2Z,4E)-3-methyl-6-[5,6,7,8-tetrahydro-5,5,8,8-tetramethyl naphthyl(2,3-b)-2,2-dimethylpyran-4-yl]hexa-2,4-dienoic acid from Example 44 was purified by HPLC (MeOH / 10 mM ammounium acetate) to give the title acid as an oily yellow solid (2.2mg): $^1$H NMR (400 MHz, $CDCl_3$) δ 7.65 (d, 1H), 7.15 (s, 1H), 6.7 (s, 1H), 6.20 (m, 1H), 5.68(s, 1H), 2.95 (m, 2H), 2.50 (m, 1H), 2.00 (s, 3H), 1.82 (m, 1H), 1.62 (s, 4H), 1.60 (dd, 1H), 1.29 (s, 6H), 1.26 (s, 6H), 1.24 (s, 6H).

**EXAMPLE 46**

Preparation of compound **130b** according to Schemes IV and VI

**(2E,4E)-3-Methyl-6-[5,6,7,8-tetrahydro-5,5,8,8-tetramethylnaphthyl-(2,3-b)-pyran-4-yl]-hexa-2,4-dienoic acid** (structure **22**, where $R_1$, $R_2$, $R_3$, $R_4$, and $R_{19}$ are methyl; $R_5$, $R_6$, $R_7$, $R_8$, $R_9$, $R_{10}$ are hydrogen; $R_{15}$ is hydroxy; X and Y are carbon; Z is oxygen; m = n = 1).

To a stirred solution of diethyl cyanomethylphosphonate (13.1g, 74.3 mmol) in THF (50 ml) at -20°C under nitrogen was added dropwise, n-BuLi (29.5ml of a 2.5M solution in hexanes). The resulting white viscous mixture was stirred for 1.5 h and treated with 4-chromanone (10.0g, 67.6 mmol, Aldrich) in THF (30 ml). The turbid, orange solution was stirred in an ice water bath, then allowed to warm to room temperature over 45 min. TLC of the reaction mixture (20 % EtOAc/hexanes, cerium molybdate) indicated the formation of two product spots, $R_f$=0.23 and 0.36 respectively. The reaction mixture was quenched by dropwise addition of saturated ammonium chloride. Aqueous workup (and EtOAc extract), followed by filtration through a silica gel plug to remove excess phosphonate gave an isomeric mixture of 4-cyanomethylidenebenzopyrans (4:1 favoring the *trans* product) as a yellow oil (10.1g, 59.1 mmol, 87%): $^1$H NMR (400 MHz, $CDCl_3$) δ 8.35 (dd, 1H), 7.5 (d, 2H), 7.32 (m, 2H), 7.0 (m, 1H), 6.9 (m, 2H), 5.73 (s, 1H), 5.17 (s, 1H ), 4.3 (m, 4H), 3.0 (dd, 2H ), 2.75 (dd, 2H).

To a solution of the above nitriles (3.06g, 17.9 mmol) in EtOAc (25 ml) was added 10% palladium on carbon (600 mg). This heterogeneous mixture was placed under an atmosphere of hydrogen and stirred at 25°C for 16 h. The reaction mixture was filtered and the solvent removed to give 4-cyanomethylbenzopyran as a rose colored oil (2.97 g, 17.2 mmol, 96%): $^1$H NMR (400 MHz, $CDCl_3$) δ 7.16 (m, 2H), 6.9 (dd, 1H), 6.83 (d, 1H), 4.2 (m, 2H), 3.25 (m, 1H), 2.82 (dd, 1H), 2.6 (dd, 1H), 2.3 (m, 1H), 2.15 (m, 1H).

To a solution of the above nitrile (2.9g, 16.8 mmol), 2,5-dichloro-2,5-dimethylhexane (3.1g, 16.8 mmol) in 1,2-dichloroethane (25 ml) at 25°C was added aluminum trichioride (3.1g, 16.8 mmol) in portions. The exothermic reaction was stirred for 2h, and the orange mixture was poured over ice. Aqueous work up (EtOAc extraction), followed by recrystallization (MeOH /water) gave [5,6,7,8-tetrahydro-5,5,8,8-tetramethylnaphthyl-(2,3-b)pyran-4-yl]cyanomethane, as white needles (3.0 g, 10.6 mmol, 63%): $^1$H NMR (400 MHz, $CDCl_3$) δ 7.05 (s, 1H ), 6.77 (s, 1H ), 4.15 (m, 2H), 3.22 (m, 1H), 2.6 (dd, 1H), 2.3 (dd, 1H), 2.25 (m, 1H), 1.95 (m, 1H), 1.65 (s, 4 H), 1.25 (s, 12H).

To a solution of the above tricyclic nitrile (1.3g, 4.6 mmol) in THF (25 ml) at -78°C under nitrogen was added dropwise, DIBAL (3 ml of 1.0 M solution in $CH_2Cl_2$ ). The mixture was stirred for 30 min, warmed to 25°C, and stirred for 16 h. The reaction mixture was quenched by addition of saturated ammonium chloride, followed with 1N HCl and sodium potassium tartrate. Extractive work-up (EtOAc) gave [5,6,7,8-tetrahydro-5,5,8,8-tetramethylnaphthyl (2,3-*b*)-pyran-4-yl]acetaldehyde as a light yellow solid (1.2 g, 4.2 mmol, 91.5%): $^1$H NMR (400 MHz, $CDCl_3$) δ 9.90 (t, 1H), 7.00 (s, 1H), 6.75 (s, 1H), 4.15 (m, 2H), 3.45 (m, 1H), 2.93 (dd, 1H), 2.73 (dd, 1H), 2.20 (m, 1H), 1.80 (m, 1H), 1.62 (s, 4H), 1.26 (s, 6H), 1.24 (s, 6H).

To a solution of diethyl 3-ethoxycarbonyl-2-methylprop-2-enylphosphonate (475 mg, 1.80 mM) in THF (10ml ) at -78°C under nitrogen was added n-BuLi (0.75 ml of a 2.5M solution in hexanes). The mixture was warmed to -20°C over 40 min, and the above aldehyde (467 mg, 1.63 mmol) in THF (2 ml) was added in one portion. The reaction mixture was stirred at 0°C for 1 h, then quenched with saturated ammonium chloride. Extractive work-up (EtOAc) gave ethyl (2E,4E)- and (2Z,4E)-3-methyl-6-[5,6,7,8-tetrahydro-5,5,8,8-tetramethylnaphthyl (2,3-b) pyran-4yl]hexa-2,4-dienoate as a yellow oil. Flash chromatography (20% EtOAc/hexane) gave the mixture of esters as a pale yellow oil (400 mg, 1.01 mmol, 62.9%): $^1$H NMR indicated the isomers in a 3:1 ratio favoring the *trans* product. These could not be resolved by chromatography and were used as a mixture in subsequent reactions: $^1$H NMR (400 MHz, $CDCl_3$) δ 7.6 (d, 1H),

7.05(s, 2H), 6.72(s, 1H), 6.25(m, 3H), 5.75(s, 1H), 5.65 (s, 1H), 4.15(m, 8H), 2.90(m, 2H), 2.71(m, 2H), 2.42 (m, 2H), 2.30 (s, 3H), 2.05 (m, 2H), 2.03 (s, 3H), 1.8 (m, 2H), 1.62 (s, 8H), 1.30 (t, 6H), 1.28 (s, 12H), 1.25 (s, 12H).

The mixture of esters (400 mg, 1.01 mmol) in ethanol (5 ml) was treated with sodium hydroxide (500 mg, 12.5 mmol) in a 60% ethanol/water solution (5 ml) and refluxed for 3 h. The reaction mixture was cooled to 25°C, diluted with water, acidified with 1N HCl and extracted (EtOAc) to give a mixture of (2E,4E) and (2Z,4E)-3-methyl-6-[5,6,7,8-tetrahydro-5,5,8,8-tetramethylnaphthyl(2,3-b )pyran-4- yl]hexa-2,4-dienoic acid as a light yellow solid (284 mg, 78 mmol, 78%). Recrystallization (MeOH/water) afforded **130b** as an off-white solid (74 mg): TLC $R_f$=0.24 (20% EtOAc/Hexane); mp 195.6°C; $^1$H NMR (400 MHz, $CDCl_3$) δ 7.05 (s, 1H), 6.72 (s, 1H), 6.25 (m, 2H), 5.75 (d, 1H), 4.15 (dd, 2H), 2.90 (m, 1H), 2.71 (m, 1H), 2.42 (m, 1H), 2.30 (s, 3H), 2.05 (m, 1H), 1.8 (m, 1H), 1.62 (s, 4H), 1.26 (s, 6H), 1.24 (s, 6H).

**EXAMPLE 47**

Preparation of compound **131b** according to Schemes IV and VI

**(2Z,4E)-3-Methyl-6-[5,6,7,8-tetrahydro-5,5,8,8-tetramethyl naphthyl (2,3-b ) pyran-4-yl]-hexa-2,4-dienoic acid** (structure **22**, where $R_1$, $R_2$, $R_3$, $R_4$, and $R_{19}$ are methyl; $R_5$, $R_6$, $R_7$, $R_8$, $R_9$, $R_{10}$ are hydrogen; $R_{15}$ is hydroxy; X and Y are carbon; Z is oxygen; n = m = 1).

Concentration of the second crop mother liquors from Example 46 gave a yellow oil. $^1$H NMR confirmed the structure as the corresponding (2Z,4E) acid. GC analysis of this material indicated 89% geometric purity with the remainder being the (2E, 4E) isomer: Compound **131b** had TLC $R_f$=0.23 (20% EtOAc/Hexane); $^1$H NMR (400 MHz, $CDCl_3$) δ 7.60 (d, 1H), 7.05 (s, 1H), 6.72 (s, 1H), 6.25 (m, 1H), 5.65 (m, 1H), 4.18 (dd, 2H), 2.95 (m, 1H), 2.71 (m, 1H), 2.50 (m, 1H), 2.05 (m, 1H), 2.03 (s, 3H), 1.8 (m, 1H), 1.62 (s, 4 H), 1.26 (s, 6H), 1.24 (s, 6H).

**EXAMPLE 48**

Preparation of compounds **132b** and **133b** according to Scheme VII

**(+)-(2E,4E)-3-Methyl-6-(1,2,3,4,5,6,7,8-octahydro-5,5,8,8-tetramethylanthracen-1-yl)hexa-2,4-dienoic acid** (structure **28**, where $R_1$, $R_2$, $R_3$, $R_4$ and $R_{19}$ are methyl; $R_5$, $R_7$, $R_8$, $R_9$ and $R_{10}$ are hydrogen; $R_{15}$ is hydroxy; X, Y and Z are carbon and m = n = 1). The assigned absolute stereochemistry is arbitrary.

Ester **18** (where $R_1$, $R_2$, $R_3$, and $R_4$ are methyl; $R_5$, $R_7$, $R_8$, $R_9$, and $R_{10}$ are hydrogen; X, Y and Z are carbon) (prepared in Example 34) (740 mg, 2.49 mmol) was dissolved in EtOAc (5.0 mL) and Pd/C 10% (20 mg) was added. The mixture was stirred under an atmosphere of hydrogen for 12 h. The mixture was filtered through a pad of celite and thoroughly rinsed with excess EtOAc. The solvent was evaporated to give 730 mg (98% yield) of the ester: methyl (R,S)-2-(1,2,3,4,5,6,7,8-octahydro-5,5,8,8-tetramethylanthracen-1-yl)acetate: $^1$H NMR (400 MHz, $CDCl_3$) δ 7.07 (s, 1H, Ar), 6.99 (s, 1H, Ar), 3.72 (s, 3H, $OCH_3$), 3.30 (m, 1H), 2.72 (m, 3H), 2.53 (dd, J=9.9, 8.2 Hz, 1H), 1.89 (m, 1H), 1.79 (m, 2H), 1.75 (m, 1H), 1.75 (s, 4H), 1.24 (s, 12H).

The above ester (730 mg, 2.44 mmol) was dissolved in THF (5.0 mL) and LiOH (2.0 mL of a 2.0 M aqueous solution) was added. The mixture was stirred at room temperature for 40 h, then acidified using 1 N HCL and extracted with EtOAc. The organic layer was dried over $MgSO_4$, filtered, and evaporated to give the corresponding acid as a white solid, 648 mg (88% yield). The crude acid (648 mg, 2.16 mmol) was dissolved in dichloromethane (5.0 mL) and a drop of dimethylformamide was added. Oxalyl chloride (0.380 mL, 4.32 mM) was added dropwise, followed by stirring at room temperature until the evolution of $CO_2$ ceased (~ 2 h). The solvent was evaporated and the residue dried under high vacuum (~0.1 mmHg) to give the corresponding acid chloride (689 mg), (R,S)-2-(1,2,3,4,5,6,7,8-octahydro-5,5,8,8-tetramethylanthracen-1-yl)-acetyl chloride.

A 50 mL round-bottom flask was flame-dried and charged with (S)-4-benzyl-2-oxazolidinone (576 mg, 3.25 mmol) and anhydrous THF (10 mL). The solution was cooled to -78°C and n-BuLi (2.1 mL, 1.55 M in hexanes) was added. The solution was stirred at -78°C for 30 min and a solution of the above acid chloride in THF (10 mL) was added slowly. Stirring was continued for 30 min, and the mixture was warmed to room temperature. After 5 min, the reaction mixture was cooled to -78°C, quenched with a saturated solution of ammonium chloride, and extracted with EtOAc (2 x 20 mL). The organic layer was washed with water (2 x 10 mL), brine (2 x 10 ml) and dried over $MgSO_4$. Evaporation of the solvent gave a diastereomeric mixture of the corresponding amides (70% yield), which were chromatographically separated (hexanes: EtOAc=95:5). The less polar amide had $^1$H NMR (400 MHz, $CDCl_3$) δ 7.36-7.22 (m, 5H, Ar), 7.18 (s, 1H, Ar), 6.99 (s, 1H, Ar), 4.71 (m, 1H), 4.18 (m, 2H), 3.42 (m, 1H), 3.35 (dd, J=14.9, 3.9 Hz, 1H), 3.29 (s, 1H), 3.27 (s, 1H), 2.80 (d, J=12 Hz, 1H), 2.80 (d, J=12 Hz, 1H), 2.75 (m, 2H), 1.90 (m, 2H), 1.76 (m, 2H), 1.65 (s, 4H), 1.26 (s, 6H), 1.24 (s, 6H); the more polar amide had $^1$H NMR (400 MHz, $CDCl_3$) δ 7.36-7.22 (m, 5 H, Ar), 7.00 (s, 1 H, Ar), 4.73

(m, 1 H), 4.20 (m, 2 H), 3.50 (m, 1 H), 3.34 (m, 2 H), 3.20 (m, 1 H), 2.75 (m, 3 H), 1.90 (m, 2H), 1.75 (m, 2H), 1.66 (s, 4H), 1.28 (s, 6H), 1.27 (s, 6H).

A solution of the above more polar amide (190 mg, 4.13 mmol) in THF (10.0 mL) was cooled to -78°C. $LiAlH_4$ (0.425 mL of 1.0 M solution in THF) was added at -78°C, stirred for 30 min, then warmed to 0°C. The mixture was quenched with water (0.1 mL), NaOH (15%, 0.1 mL), and water (0.3 mL), then extracted with EtOAc (30 mL). The organic layer was dried over $MgSO_4$, and the solvent evaporated. The crude residue was purified by silica gel chromatography (hexanes: EtOAc=4:1) to give 61 mg of the desired alcohol, (+)-2-(1,2,3,4,5,6,7,8-octahydro-5,5,8,8-tetramethylanthracen-1-yl)ethan-1-ol (55% yield), $[a]_D$ +12.7 (c 0.59, $CHCl_3$).

The above alcohol (60 mg, 0.21 mmol) was dissolved in dichloromethane (5.0 mL) and Dess-Martin reagent (2 x 135 mg, 0.63 mmol) was added in two portions and stirred for 60 min. A saturated solution of sodium bicarbonate (3.0 mL) was added, followed by solid sodium thiosulfate. The mixture was stirred vigorously and extracted with dichloromethane. The organic layer was washed with water (2 x 5 mL), brine (2 x 5 mL) and dried over $MgSO_4$. The solvent was evaporated and the residue was purified over a short silica gel chromatography column to give 48 mg of the desired aldehyde, (+)-2-(1,2,3,4,5,6,7,8-octahydro-5,5,8,8-tetramethylanthracen-1yl)ethan-1-al (81% yield).

The above aldehyde was transformed to the title compound using a procedure similar to that described for the racemic material (Examples 37 and 38). Compound **132b** had $[a]_D$ +16.2 (c 0.36, $CHCl_3$). The $^1$HNMR spectrum was indistinguishable from that of the racemate **124b**.

**(-)-(2E,4E)-3-Methyl-6-(1,2,3,4,5,6,7,8-octahydro-5,5,8,8-tetramethylanthracen-1-yl)hexa-2,4-dienoic acid (133b)** (structure **29**, where $R_1$, $R_2$, $R_3$, $R_4$ and $R_{19}$ are methyl; $R_5$, $R_7$, $R_8$, $R_9$ and $R_{10}$ are hydrogen; $R_{15}$ is hydroxy; X, Y, and Z are carbon; and m = n = 1).

The title compound was prepared from the diastereomeric, less polar amide described above using the procedure for compound **132b**. Compound **133b** had $[\alpha]_D$ -16.4 (c 0.33, $CHCl_3$). $^1$HNMR data is indistinguishable from racemic **124b**.

## EXAMPLE 49

Preparation of compound **134a** according to Scheme VIII

**Methyl (2E)-3-methyl-6-(1,2,3,4,6,7,8,9-octahydro-6,6,9,9-tetramethylanthracen-1-yl)hex-2-enoate** (structure **32**, where $R_1$, $R_2$, $R_3$, $R_4$ and $R_{19}$ are methyl; $R_5$, $R_6$, $R_7$, $R_8$, $R_9$, and $R_{10}$ are hydrogen; $R_{15}$ is methoxy; X, Y, and Z are carbon; m= n=1)

(1,2,3,4,6,7,8,9-octahydro-6,6,9,9-tetramethylanthracen-1-yl)acetaldehyde, an intermediate for the preparation of compound **124a** of Example 37, was condensed with the sodium anion of diethyl (2-oxopropyl)phosphonate to give (3E)-1-(1,2,3,4,6,7,8,9-octahydro-6,6,9,9-tetramethylanthracen-1-yl)pent-2-en-4-one: $^1$H NMR (400 MHz, $CDCl_3$) $\delta$ 7.07 (s, 1H, ArH), 7.00 (s, 1H, ArH), 6.89 (m, 1H, olefinic), 6.12 (d, J=15.9Hz, 1H, olefinic), 2.94 (m, 1H, benzylic), 2.72 (br t, 2H, $CH_2$, benzylic), 2.68-2.47 (m, 2H, $CH_2$, allylic), 2.26 (s, 3H, $CH_3$), 1.82-1.62 (m, 4H, $2CH_2$), 1.66 (s, 4H, $2CH_2$), 1.26 (s, 6H, $2CH_3$), 1.24 (s, 6H, $2CH_3$).

The above enone was subjected to hydrogenation using 10% Pd on C as a catalyst, followed by condensation with trimethyl phosphonoacetate using NaH as a base to give methyl (2E)-3-methyl-6-(1,2,3,4,6,7,8,9-octahydro-6,6,9,9-tetramethylanthracen-1-yl)hex-2-enoate: $R_f$=0.25 (hexane); $^1$H NMR (400 MHz, $CDCl_3$) $\delta$ 7.05 (s, 1H, ArH), 6.97 (s, 1H, ArH), 5.69 (s, 1H, olefinic), 3.68 (s, 3H, $OCH_3$), 2.69 (m, 3H, benzylic), 2.17 (s, 3H, $CH_3$), 2.16 (m, 2H, allylic), 1.81 (m, 2H, $CH_2$), 1.68 (s, 4H, $2CH_2$), 1.68-1.54 (m, 6H, $3CH_2$), 1.26 (s, 6H, $2CH_3$), 1.24 (s, 6H, $2CH_3$).

## EXAMPLE 50

Preparation of compound **134b** according to Scheme VIII

**(2E)-3-methyl-6-(1,2,3,4,6,7,8,9-octahydro-6,6,9,9-tetramethylanthracen-1-yl)-hex-2-enoic acid** (structure **32,** where $R_1$, $R_2$, $R_3$, $R_4$ and $R_{19}$ are methyl; $R_5$, $R_6$, $R_7$, $R_8$, $R_9$, and $R_{10}$ are hydrogen; $R_{15}$ is hydroxy; X, Y, and Z are carbon; m= n=1)

The title compound was prepared by hydrolysis of compound **134a** using the standard hydrolysis conditions described in Example 2. Compound **134a** had $R_f$=0.31 (50% ether in hexane); mp 190-192°C; $^1$H NMR (400 MHz, $CDCl_3$) $\delta$ 7.05 (s, 1H, ArH), 6.98 (s, 1H, ArH), 5.72 (s, 1H, olefinic), 2.71 (m, 3H, benzylic), 2.20 (m, 2H, allylic), 2.18 (s, 3H, $CH_3$), 1.82 (m, 2H, $CH_2$), 1.75-1.59 (m, 6H, $3CH_2$), 1.66 (s, 4H, $2CH_2$), 1.26 (s, 6H, $2CH_3$), 1.25 (s, 6H, $2CH_3$).

## EXAMPLE 51

Preparation of compound **135a** according to Scheme IX

**Ethyl (2E,4E)-3-methyl-6-[(E)-2,3,6,7,8,9-hexahydro-6,6,9,9-tetramethyl-1H-cyclopenta[a]naphthalen-3-ylidene]hexa-2,4-dienoate** (structure **35**, where $R_1$, $R_2$, $R_3$, $R_4$, and $R_{19}$ are methyl; $R_9$ and $R_{10}$ are hydrogen; $R_{15}$ is ethoxy; X and Y are carbon; m = 1, and n = 0).

The title compound was prepared from 2,3,6,7,8,9-hexahydro-6,6,9,9-tetramethyl-1H-cyclopenta[*a*]naphthalen-3-one [a by-product isolated from the preparation of 2,3,5,6,7,8-hexahydro-5,5,8,8-tetramethyl-cyclopenta[*b*]naphthalen-1-one in Example 3] in a manner similar to that described for compound **51a** using the procedure described in Example 1. Compound **135a** had $^1$H NMR (400 MHz, $CDCl_3$) δ 7.36 (d, J=8.27 Hz, 1H, ArH), 7.26 (d, J=8.27 Hz, 1H, ArH), 6.90 (dd, J=11.2, 15.1 Hz, 1H, olefinic), 6.59 (d, J=11.2 Hz, 1H, olefinic), 6.31 (d, J=15.1 Hz, 1H, olefinic), 5.78 (s, CH, olefinic), 4.18 (q, J=7.02 Hz, 2H, $OCH_2$), 3.23 (br m, 2H, benzylic), 2.92 (br m, 2H, allylic), 2.37 (s, 3H, $CH_3$), 1.69 (br s, 4H, $2CH_2$), 1.36 (s, 6H, $2CH_3$), 1.32 (s, 6H, $2CH_3$), 1.31 (t, J=7.0 Hz, 3H, $CH_3$).

## EXAMPLE 52

Preparation of compound **135b** according to Scheme VIII

**(2E,4E)-3-Methyl-6-[(E)-2,3,6,7,8,9-hexahydro-6,6,9,9-tetramethyl-1H-cyclopenta[a]naphthalen-3-ylidene]hexa-2,4-dienoic acid** (structure **35**, where $R_1$, $R_2$, $R_3$, $R_4$, and $R_{19}$ are methyl; $R_9$ and $R_{10}$ are hydrogen; $R_{15}$ is hydroxy; X and Y are carbon; m = 1, and n = 0).

The title compound was prepared from compound **135a** using the standard hydrolysis condition described in Example 2. Acid **135b** had mp 195-197°C; $^1$H NMR (400 MHz, $CDCl_3$) δ 7.30 (d, J=8.2 Hz, 1H, ArH), 7.21 (d, J=8.2 Hz, 1H, ArH), 6.88 (dd, J=11.3, 15.1 Hz, 1H, olefinic), 6.54 (d, J=11.3 Hz, 1H, olefinic), 6.27 (d, J=15.1 Hz, 1H, olefinic), 5.74 (s, 1H, olefinic), 3.17 (br m, 2H, $CH_2$, benzylic), 2.86 (m, 2H, $CH_2$, allylic), 2.32 (s, 3H, $CH_3$), 1.62 (br s, 4H, $2CH_2$), 1.28 (s, 6H, $2CH_3$), 1.24 (s, 6H, $2CH_3$).

## EXAMPLE 53

Preparation of compound **136a** according to Scheme IX

**Ethyl (2E,4E)-3-methyl-6-(2,3,6,7,8,9-hexahydro-6,6,9,9-tetramethyl-1H-cyclopenta[a]naphthalen-3-yl]hexa-2,4-dienoate** (structure **36**, where $R_1$, $R_2$, $R_3$, $R_4$, and $R_{19}$ are methyl; $R_9$ and $R_{10}$ are hydrogen; $R_{15}$ is ethoxy; X and Y are carbon; m = 1, and n = 0).

The title compound was prepared from (2,3,6,7,8,9-hexahydro-6,6,9,9-tetramethyl-1H-cyclopenta[a]naphthalen-3-ylidene)ethanitrile, which was prepared from the condensation of 2,3,6,7,8,9-hexahydro-6,6,9,9-tetramethyl-1H-cyclopenta[a]naphthalen-3-one (Example 51) with cyanomethylphosphonate using NaH as a base in a manner similar to that described for compound **124a** as detailed in Example 37. Compound **136a** had $^1$H NMR(400 MHz, $CDCl_3$) δ 7.20 (d, J=8.0 Hz, 1H, ArH), 7.03 (d, J=8.0 Hz, 1H, ArH), 6.18 (m, 2H, 2 x olefinic), 5.71 (s, 1H, olefinic), 4.17 (q, J=7.6 Hz, 2H, $OCH_2$), 3.09 (m, 2H, $CH_2$, benzylic), 2.97 (m, 1H, benzylic), 2.67 (m, 1H, allylic), 2.86 (s, 3H, $CH_3$), 2.19 (m, 1H, allylic), 1.66 (m, 6H, $3CH_2$), 1.34 (s, 6H, $2CH_3$), 1.33 (s, 6H, $2CH_3$), 1.24 (t, J=7.0 Hz, 3H, $CH_3$).

## EXAMPLE 54

Preparation of compound **136b** according to Scheme IX

**(2E,4E)-3-Methyl-6-(2,3,6,7,8,9-hexahydro-6,6,9,9-tetramethyl-1H-cyclopenta[a]naphthalen-3-yl)hexa-2,4-dienoic acid** (structure **36**, where $R_1$, $R_2$, $R_3$, $R_4$, and $R_{19}$ are methyl; $R_9$ and $R_{10}$ are hydrogen; $R_{15}$ is hydroxy; X and Y are carbon; m = 1, and n = 0)

The title compound was prepared by hydrolysis of compound **136a** using the standard hydrolysis conditions described in Example 2. Compound **136b** had mp 208-210°C; $^1$H NMR (400 MHz, $CDCl_3$) δ 7.22 (d, J=8.0 Hz, 1H, ArH), 7.03 (d, J=8.0 Hz, 1H, ArH), 6.22 (m, 2H, 2 x olefinic), 5.75 (s, 1H, olefinic), 3.10 (m, 2H, benzylic), 2.95 (m, 1H, benzylic), 2.70 (m, 1H, allylic), 2.30 (s, 3H, $CH_3$), 2.20 (m, 1H, allylic), 1.66 (m, 6H, $3CH_2$), 1.32 (s, 6H, $2CH_3$), 1.28 (s,

6H, $2CH_3$).

**EXAMPLE 55**

Preparation of compound **137a** according to Scheme IX

**Ethyl (2E,4E)-3-methyl-6-[(Z)-1,2,3,4,7,8,9-heptahydro-7,7,9,9-tetramethyl-cyclopenta[f]naphthalen-4-ylidene]hexa-2,4-dienoate** (structure **35**, where $R_1$, $R_2$, $R_3$, $R_4$, and $R_{19}$ are methyl; $R_9$ and $R_{10}$ are hydrogen; $R_{15}$ is ethoxy; X and Y are carbon; m = 0, and n = 1)

The title compound was prepared from 1,2,3,4,7,8,9-heptahydro-7,7,9,9-tetramethyl-cyclopenta[*f*]naphthalen-4-one (isolated as a by-product for the preparation of 1,2,3,6,7,8-hexahydro-1,1,3,3-tetramethyl-cyclopenta[*b*]naphthalen-5-one in Example 5) using the procedure described in example 51. Compound **137a** had $R_f$=0.50 (15% ether in hexane); $^1H$ NMR (400 MHz, $CDCl_3$) δ: 7.25 (d, J=7.9 Hz, 1H, ArH), 7.15 (dd, J=15.2, 11.4 Hz, 1H, olefinic), 7.00 (d, J=7.9 Hz, 1H, ArH), 6.30 (d, J=15.2 Hz, 1H, olefinic), 6.14 (d, J=11.4 Hz, 1H, olefinic), 5.76 (s, 1H, olefinic), 4.16 (q, 2H, $OCH_2$), 2.88 (t, J=6.4 Hz, 2H, benzylic), 2.51 (t, J=6.5 Hz, 2H, allylic), 2.29 (s, 3H, $CH_3$), 1.91 (m, 4H, $2CH_2$), 1.42 (s, 6H, $2CH_3$), 1.29 (m, 9H, $3CH_3$).

**EXAMPLE 56**

Preparation of compound **137b** according to Scheme IX

**(2E,4E)-3-Methyl-6-[(Z)-1,2,3,4,7,8,9-heptahydro-7,7,9,9-tetramethyl-cyclopenta[f]naphthalen-4-ylidene]hexa-2,4-dienoic acid** (structure **35**, where $R_1$, $R_2$, $R_3$, $R_4$, and $R_{19}$ are methyl; $R_9$ and $R_{10}$ are hydrogen; $R_{15}$ is hydroxy; X and Y are carbon; m = 0, and n = 1)

The title compound was prepared from compound **137a** using the standard hydrolysis conditions described in Example 2. Compound **137b** had $R_f$=0.45 (50% ether in hexane), mp 176-178°C; $^1H$ NMR (400 MHz, $CDCl_3$) δ 7.24 (d, J=7.8 Hz, 1H, ArH), 7.19 (dd, J=15.2, 11.3 Hz, 1H, olefinic), 7.00 ( d, J=7.9 Hz, 1H, ArH), 6.33 (d, J=15.2 Hz, 1H, olefinic), 6.16 (d, J=11.3 Hz, 1H, olefinic), 5.79 (s, 1H, olefinic), 2.88 (t, J=6.4 Hz, 2H, benzylic), 2.53 (t, J=6.4 Hz, 2H, allylic), 2.30 (s, 3H, $CH_3$), 1.93 (m, 4H, $2CH_2$), 1.43 (s, 6H, $2CH_3$), 1.31 (m, 6H, $2CH_3$).

**EXAMPLE 57**

Preparation of compound **138a** according to Scheme X

**Ethyl (2E,4E)-3-methyl-6-(7,7,10,10-tetramethyl-2,3,4,5,7,8,9,10-octahydronaphtho[2,3-6]-azepinyl)hexa-2,4-dienoate** (structure **40**, where $R_1$, $R_2$, $R_3$, $R_4$, and $R_{19}$ are methyl; $R_5$, $R_6$, $R_7$, $R_8$, $R_9$, and $R_{10}$ are hydrogen; $R_{15}$ is ethoxy; X, Y, and Z are carbon; m = n = 1).

To a solution of 3,4,5,6,7,8-hexahydro-6,6,9,9-tetramethyl-2H-anthracen-1-one (256 mg, 1 mmol, from Example 1) in EtOH (10 ml) and pyridine (3 drops) was added $H_2NOH$-HCl(140 mg, 2 mmol) at 25°C, and resulting mixture was heated at reflux for 5 h. The mixture was cooled to room temperature and the filtered through filter paper and the solid was washed with hexane (50 ml). The solid was dried under vacuum to give pure *trans*-(3,4,6,7,8-hexahydro-6,6,9,9-tetramethyl-2H-anthracene-1-yl)-oxime (219 mg, 84%): $^1H$ NMR(400 MHz, $CDCl_3$) δ 7.85 (s, 1H, ArH), 7.05 (s, 1H, ArH), 2.76 (t, J=6.1 Hz, 2H, benzylic), 2.69 (t, J=6.0 Hz, 2H, $CH_2$), 1.85 (m, 2H, $CH_2$), 1.65 (s, 4H, $2CH_2$), 1.32 (s, 6H, $2CH_3$), 1.30 (s, 6H, $2CH_3$).

The above oxime (271 mg, 1 mmol) in THF(10 ml) was treated with $LiAlH_4$ (3 ml, 1 M, 3 mM) at room temperature, and the resulting mixture was heated at reflux (80°C) for 8 h. The reaction mixture was cooled to 0°C, quenched with sodium potassium tartrate (20 ml), extracted with EtOAc(100 ml), dried over $MgSO_4$ and concentrated under reduced pressure. The crude product was chromatographed (20% ether in hexane) to afford pure 5,6,7,8-tetrahydro-5,5,8,8-tetramethyl-(2,3)-naphthyl-[*b*]azepine (218 mg, 85%): $^1H$ NMR(400 MHz, $CDCl_3$) δ 6.95 (s, 1H, ArH), 6.62 (s, 1H, ArH), 3.01 (t, J=6.2 Hz, 2H, benzylic), 2.69 (br m, 2H, $CH_2$), 1.73 (m, 2H, $CH_2$), 1.62 (m, 2H, $CH_2$), 1.61 (s, 4H, $2CH_2$), 1.25 (s, 12H, $4CH_3$).

To a solution of the above azepine (257 mg, 1 mmol) in THF (5 ml) was added NaH (80 mg, 60 % in oil, 2 mmol) at 0°C, and the resulting mixture was stirred at 0°C for 30 min. To this solution was added ethyl (2E,4E)-3-methyl-6-bromohexa-2,4-dienoate (233 mg, 1 mmol) [prepared by $NaBH_4$ reduction of ethyl (2E,4E)-(3-methyl-5-formyl)penta-2,4-dienoate in Example 14, followed by bromination using $PBr_3$ in ether at 0°C to afford the necessary bromo compound]

in THF (5 ml) at room temperature, and the resulting mixture was stirred for 8 h. Standard work-up procedure as described in Example 1, followed by chromatographic purification (10% ether in hexane) gave the title ester (294 mg, 72%): $R_f$=0.78(20 % ether in hexane); $^1$H NMR(400 MHz, $CDCl_3$) δ 6.97 (s, 1H, ArH), 6.75 (s, 1H, ArH), 6.32 (d, J=15.8 Hz, 1H, olefinic), 6.22 (dt, J=15.8, 5.6 Hz, 1H, olefinic), 5.72 (s, 1H, olefinic), 4.15 (q, J=7.0 Hz, 2H, $OCH_2$), 3.85 (br d, J=5.6 Hz, 2H, allylic), 2.86 (br t, 2H, $NCH_2$), 2.73 (m, 2H, benzylic), 2.25 (s, 3H, $CH_3$), 1.66 (m, 4H, $CH_2$), 1.61 (s, 4H, $2CH_2$), 1.58 (m, 2H, $CH_2$), 1.25 (t, J=7.0 Hz, 3H, $CH_3$), 1.21 (s, 6H, $2CH_3$), 1.20 (s, 6H, $2CH_3$).

**EXAMPLE 58**

Preparation of compound **138b** according to Scheme X

**(2E,4E)-3-Methyl-6-[7,7,10,10-tetramethyl-2,3,4,5,7,8,9,10-octahydronaphtho[2,3-6]azepinyl)hexa-2,4-dienoic acid** (structure **42**, where $R_1$, $R_2$, $R_3$, $R_4$, and $R_{19}$ are methyl; $R_5$, $R_6$, $R_7$, $R_8$, $R_9$, and $R_{10}$ are hydrogen; $R_{15}$ is hydroxy; X, Y, and Z are carbon; m = n = 1).

The title compound was prepared from compound **138a** using the standard hydrolysis conditions described in Example 2. Compound **138b** as an amorphous solid had $R_f$=0.33 (50% ether in hexane); $^1$H NMR (400 MHz, $CDCl_3$) δ 6.99 (s, 1H, ArH), 6.75 (s, 1H, ArH), 6.36 (d, J=15.7 Hz, 1H, olefinic), 6.22 (dt, J=15.7, 5.6 Hz, 1H, olefinic), 5.78 (s, 1H, olefinic), 3.89 (d, J=5.6 Hz, 2H, allylic), 2.88 (br t, 2H, $NCH_2$), 2.73 (m, 2H, benzylic), 2.28 (s, 3H, $CH_3$), 1.69 (m, 2H, $CH_2$), 1.63 (s, 4H, $2CH_2$), 1.59 (m, 2H, $CH_2$), 1.23 (s, 6H, $2CH_3$), 1.22 (s, 6H, $2CH_3$).

**EXAMPLE 59**

Preparation of compound **139a** according to Scheme X

**Ethyl 3-methyl-6-(3,4,6,7,8,9-hexahydro-6,6,9,9-tetramethyl-2H-benzo[g]quinolin-1-yl)hexa-2,4-dienoate** (structure **40**, where $R_1$, $R_2$, $R_3$, $R_4$, and $R_{19}$ are methyl; $R_5$, $R_6$, $R_7$, $R_8$, $R_9$, $R_{10}$ are hydrogen; $R_{15}$ is ethoxy; X, Y, and Z are carbon; m = 1, and n = 0).

The title compound was prepared from 2,3,5,6,7,8-hexahydro-5,5,8,8-tetramethyl-cyclopenta[*b*]naphthalen-1-one [U.S. patent 2,815,382 (1957)] in a manner similar to that described for compound **138a** of Example 57. Ester **139a** had $^1$H NMR(400 MHz, $CDCl_3$) δ 6.88 (s, 1H, ArH), 6.51 (s, 1H, ArH), 6.29 (d, J=15.7 Hz, 1H, olefinic), 6.15 (dt, J=15.7, 5.6 Hz, 1H, olefinic), 5.72 (s, 1H, olefinic), 4.16 (q, J=7.0 Hz, 2H, $OCH_2$), 4.00 (d, J=5.6 Hz, 2H, allylic), 3.23 (br t, 2H, $NCH_2$), 2.73 ( br t, 2H, benzylic), 2.25 (s, 3H, $CH_3$), 1.96 (m, 2H, $CH_2$), 1.63( s, 4H, $2CH_2$), 1.26 (t, J=7.0 Hz, 3H, $CH_3$), 1.24 (s, 6H, $2CH_3$), 1.23 (s, 6H, $2CH_3$).

**EXAMPLE 60**

Preparation of compound **139b** according to Scheme X

**(2E,4E)-3-Methyl-6-[3,4,6,7,8,9-hexahydro-6,6,9,9-tetramethyl-2H-benzo[g]quinolin-1-yl)hexa-2,4-dienoic acid** (structure **40**, where $R_1$, $R_2$, $R_3$, $R_4$, and $R_{19}$ are methyl; $R_5$, $R_6$, $R_7$, $R_8$, $R_9$, and $R_{10}$ are hydrogen; $R_{15}$ is hydroxy; X, Y, and Z are carbon; m = 1, and n = 0).

The title compound was prepared from compound **139a** using the standard hydrolysis conditions detailed in Example 2. Compound **139b** had mp 211-213°C; $^1$H NMR (400 MHz, $CDCl_3$) δ 6.88 (s, 1H, ArH), 6.44 (s, 1H, ArH), 6.33 (d, J=15.7 Hz, 1H, olefinic), 6.20 (dt, J=15.7, 5.6 Hz, 1H, olefinic), 5.75 (s, 1H, olefinic), 3.98 (d, J=5.6 Hz, 2H, allylic), 3.23 (br t, 2H, $NCH_2$), 2.74 (br t, 2H, benzylic), 2.27 (s, 3H, $CH_3$), 1.98 (m, 2H, $CH_2$), 1.63 (s, 4H, $2CH_2$), 1.24 (s, 6H, $2CH_3$), 1.23 (s, 6H, $2CH_3$).

## EXAMPLE 61

Preparation of compound **140a** according to Scheme X

**Ethyl (2E,4E)-3-methyl-6-oxo-6-[5,6,7,8-tetrahydro-5,5,8,8-tetramethyl(2,3)naphthyl[b]piperidin-1-yl]hexa-2,4-dienoate** (structure **42**, where $R_1$, $R_2$, $R_3$, $R_4$, and $R_{19}$ are methyl; $R_5$, $R_6$, $R_7$, $R_8$, $R_9$, and $R_{10}$ are hydrogen; $R_{15}$ is ethoxy; X, Y, and Z are carbon; m=1, and n=0)

5,6,7,8-Tetrahydro-5,5,8,8-tetramethyl(2,3)naphthyl[*b*]piperidine (from Example 57) was coupled with ethyl (2E,4Z)-3-methyl-5-carboxylic acid-penta-2,4-dienoate [prepared by Jone's oxidation of ethyl (2E,4E)-(3-methyl-5-formyl)-penta-2,4-dienoate of Example 4] in the presence of DCC and DMAP and CSA as catalyst in $CH_2Cl_2$ to give the title ester: $R_f$=0.50 (50% ether in hexane); $^1H$ NMR (400 MHz, $CDCl_3$) $\delta$ 7.35 (d, J=15.3 Hz, 1H, olefinic), 7.08 (s, 1H, ArH), 6.85 (br s, 1H, ArH), 6.63 (d, J=15.3 Hz, 1H, olefinic), 6.05 (s, 1H, olefinic), 4.18 (q, 2H, $OCH_2$), 3.85 (t, J=6.5 Hz, 2H, $NCH_2$), 2.65 (t, J=6.5 Hz, 2H, benzylic), 2.17 (s, 3H, $CH_3$), 1.95 (m, 2H, $CH_2$), 1.65 (s, 4H, $2CH_2$), 1.25 (m, 9H, $3CH_3$), 1.21 (s, 6H, $2CH_3$)

## EXAMPLE 62

Preparation of compound **140b** according to Scheme X

**(2E,4E)-3-methyl-6-oxo-6-(6,6,9,9-tetramethyl-3,4,6,7,8,9-hexahydro-2H-benzo[g]quinolin-1-yl)hexa-2,4-dienoic acid** (structure **41**, where $R_1$, $R_2$, $R_3$, $R_4$, and $R_{19}$ are methyl; $R_5$, $R_6$, $R_7$, $R_8$, $R_9$, and $R_{10}$ are hydrogen; X, Y, and Z are carbon; m=1, and n=0)

The title compound was prepared from compound **140a** using the standard hydrolysis conditions described in Example 2. Compound **140b** had $R_f$=0.25 (in ether); mp 225-227°C; $^1H$ NMR (400 MHz, $CDCl_3$) $\delta$ 7.37 (d, J=15.3 Hz, 1H, olefinic), 7.08 (s, 1H, ArH), 6.87 (br s, 1H, ArH), 6.67 (d, J=15.3 Hz, 1H, olefinic), 6.08 (s, 1H, olefinic), 3.86 (t, J=6.5 Hz, 2H, $NH_2$), 2.68 (t, J=6.5 Hz, 2H, benzylic), 2.19 (s, 3H, $CH_3$), 1.96 (m, 2H, $CH_2$), 1.66 (s, 4H, $2CH_2$), 1.27 (s, 6H, $2CH_3$), 1.98 (s, 6H, $2CH_3$).

## EXAMPLE 63

Preparation of compound **141a** according to Scheme XI

**Ethyl (2E,4E)-3-methyl-6-oxo-6-[(2,3,5,6,7,8-hexahydro-5,5,8,8-tetramethyl)benzo[f]indol-1-yl]hexa-2,4-dienoate** (structure **47**, where $R_1$, $R_2$, $R_3$, $R_4$, and $R_{19}$ are methyl; $R_5$, $R_6$, $R_9$ and $R_{10}$ are hydrogen; $R_{15}$ is ethoxy; X is carbon; m=1, and n=0)

2,5-Dimethyl-2,5-dichlorohexane was coupled with oxindole in $CH_2Cl_2$ at 25°C in the presence of $AlCl_3$ to obtain 2,3,5,6,7,8-hexahydro-5,5,8,8-tetramethyl-1H-benz-[*f*]-2-oxo-indole, which was reduced with DIBAL in $CH_2Cl_2$ at reflux temperature to give 2,3,5,6,7,8-hexahydro-5,5,8,8-tetramethyl-1H-benz[f]-indole: $^1H$ NMR (400 MHz, $CDCl_3$) $\delta$ 7.08 (s, 1H, ArH), 6.61 (s, 1H, ArH), 3.51 (t, J=8.0 Hz, 2H, N-$CH_2$), 2.98 (t, J=8.1Hz, 2H, benzylic), 1.64 (s, 4H, $2CH_2$), 1.24 (s, 6H, $2CH_3$), 1.23 (s, 4H, $2CH_2$).

The above indole was then coupled with ethyl (2E,4E)-3-methyl-5-carboxylic acid-pent-2,4-dienoate (Example 61) using DCC and DMAP as reagents and CSA as a catalyst in $CH_2Cl_2$ at -78°C to give the title ester: $R_f$=0.50 (50% ether in hexane); $^1H$ NMR (400 MHz, $CDCl_3$) $\delta$ 8.32 (s, 1H, ArH), 7.44 (d, J=15.1 Hz, 1H, olefinic), 7.15 (s, 1H, ArH), 6.62 (d, J=15.1 Hz, 1H, olefinic), 6.06 (s, 1H, olefinic), 4.20 (m, $CH_2$;$OCH_2$), 3.19 (brt, J=9.3 Hz, 2H, benzylic), 2.30 (s, 3H, $CH_3$), 1.68 (s, 4H, $2CH_2$), 1.31 (m, 9H, $3CH_3$), 1.26 (s, 6H, $2CH_3$).

## EXAMPLE 64

Preparation of compound **141b** according to Scheme XI

**(2E,4E)-3-Methyl-6-oxo-6-[(2,3,5,6,7,8-hexahydro-5,5,8,8-tetramethyl)benzo-[f]-indol-1-yl]hexa-2,4-dienoic acid** (structure **46**, where $R_1$, $R_2$, $R_3$, $R_4$, and $R_{19}$ are methyl; $R_9$ and $R_{10}$ are hydrogen; X, and Y are carbon; and m=1)

The title acid was prepared from compound **141a** using the standard hydrolysis conditions described in Example 2. Compound **141b** had $R_f$=0.15 (50% ether in hexane); mp 226-228°C; $^1H$ NMR (400 MHz, $CDCl_3$) $\delta$ 8.33 (s, 1H,

ArH), 7.47 (d, J=15.1 Hz, 1H, olefinic), 7.16 (s, 1H, ArH), 6.67 (d, J=15.1 Hz, 1H, olefinic), 6.11 (s, 1H, olefinic), 4.21 (t, J=8.1 Hz, 2H, $NCH_2$), 3.20 (t, J=8.1Hz, 2H, benzylic), 2.36 (s, 3H, $CH_3$), 1.68 (s, 4H, $2CH_2$), 1.32 (s, 6H, $2CH_3$), 1.27 (s, 6H, $2CH_3$).

## EXAMPLE 65

Preparation of compound **142a** according to Scheme XI

**Ethyl (2E,4E)-3-methyl-6-(2,3,5,6,7,8-hexahydro-5,5,8,8-tetramethylbenzo-[f]-indol-1-yl]hexa-2,4-dienoate** (structure **46**, where $R_1$, $R_2$, $R_3$, $R_4$ and $R_{19}$ are methyl; $R_9$ and $R_{10}$ are hydrogen; $R_{15}$ is ethoxy; X and Y are carbon; and m=1)

2,3,5,6,7,8-Hexahydro-5,5,8,8-tetramethyl-1H-benz-[f]-indole (prepared previously in Example 63) was coupled with (2E,4E)-3-methyl-6-bromo-hexa-2,4-dienoate (prepared previously in Example 57) using NaH as base and THF as solvent to give the title compound: $R_f$=0.80 (50% ether in hexanes); $^1$H NMR (400 MHz, $CDCl_3$) δ 7.05 (s, 1H, ArH), 6.42 (s, 1H, ArH), 6.41 (d, J=16.4 Hz, 1H, olefinic), 6.19 (m, 1H, olefinic), 5.85 (s, 1H, olefinic), 4.16 (q, 2H, $OCH_2$), 3.80 (d, J=5.7 Hz, 2H, $NCH_2$, allylic), 3.30 (t, J=8.0 Hz, 2H, $NCH_2$), 2.91 (t, J=8.0 Hz, 2H, benzylic), 2.28 (s, 3H, $CH_3$), 1.64 (s, 6H, $2CH_3$), 1.27 (m, 9H, $3CH_3$).

## EXAMPLE 66

Preparation of compound **142b** according to Scheme XI

**(2E,4E)-3-Methyl-6-(2,3,5,6,7,8-hexahydro-5,5,8,8-tetramethylbenzo[f]indol-1-yl]hexa-2,4-dienoic acid** (structure **47**, where $R_1$, $R_2$, $R_3$, $R_4$, and $R_{19}$ are methyl; $R_9$ and $R_{10}$ are hydrogen; $R_{15}$ is hydroxy; X and Y are carbon; and m=1)

The title compound was prepared from compound **142a** using the standard hydrolysis conditions described in Example 2. Acid **142b** had $R_f$=0.30 (50% ether in hexane); mp 170-171°C; $^1$H NMR (400 MHz, $CDCl_3$) δ 7.06 (s, 1H, ArH), 6.41 (s, 1H, ArH), 6.39 (d, H=16.2 Hz, 1H, olefinic), 6.28 (m, 1H, olefinic), 5.79 (s, 1H, olefinic), 3.81 (d, J=5.7 Hz, 2H, N-$CH_2$, allylic), 3.30 (t, J=8.1 Hz, 2H, $NCH_2$), 2.92 (t, J=8.1 Hz, 2H, benzylic), 2.30 (s, 3H, $CH_3$), 1.64 (s, 4H, $2CH_2$), 1.23 (s, 12H, $4CH_3$).

## EXAMPLE 67

Preparation of compound **143a** according to Scheme XII

**Ethyl (2E,4E)-3-methyl-6-(7,7,10,10-tetrahydro-5,5,8,8-tetramethyl)benzo[f]quinolin-4-yl)hexa-2,4-dienoate** (structure **50**, where $R_1$, $R_2$, $R_3$, $R_4$, and $R_{19}$ are methyl; $R_9$ and $R_{10}$ are hydrogen; $R_{15}$ is ethoxy; X and Y are carbon; m=1, and n=0)

2,3,6,7,8,9-Hexahydro-6,6,9,9-tetramethyl-1H-cyclopenta[a]naphthalen-3-one (prepared previously in Example 51) was coupled with $H_2NOH$-HCl to give the oxime which, without further purification, was subjected to LAH reduction to give 5,6,7,8-tetrahydro-5,5,8,8-tetramethyl(1,2)-naphthyl-[*b*]-piperidine: $^1$H NMR (400 MHz, $CDCl_3$) δ 6.95 (d, J=8.7 Hz, 1H, ArH), 6.32 (d, J=8.7 Hz, 1H, ArH), 3.25 (t, J=6.3 Hz, 2H, $NCH_2$), 2.90 (t, J=6.0 Hz, 2H, benzylic), 1.87 (m, 2H, $CH_2$), 1.61 (m, 4H, $2CH_2$), 1.40 (s, 6H, $2CH_3$), 1.22 (s, 6H, $2CH_3$).

The above piperidine was then converted to the title compound in a manner similar to that of compound **89a** as described in Example 59. Compound **143a** had $R_f$=0.75 (20% ether in hexane); $^1$H NMR (400 MHz, $CDCl_3$) δ 7.05 (d, J=8.7 Hz, 1H, ArH), 6.46 (d, J=8.7 Hz, 1H, ArH), 6.24 (d, J=15.8 Hz, 1H, olefinic), 6.13 (m, 1H, olefinic), 5.73 (s, 1H, olefinic), 4.15 (q, J=7.2 Hz, 2H, $OCH_2$), 3.94 (d, J=4.8 Hz, 2H, N-$CH_2$-allylic), 3.17 (t, J=6.3 Hz, 2H, N-$CH_2$), 2.90 (t, J=5.9 Hz, 2H, benzylic), 2.26 (s, 3H, $CH_3$), 1.97 (m, 2H, $CH_2$), 1.62 (m, 6H, $2CH_3$), 1.27 (t, J=7.2 Hz, 3H, $CH_3$), 1.24 (s, 6H, $2CH_3$).

**EXAMPLE 68**

Preparation of compound **143b** according to Scheme XII

**(2E,4E)-3-Methyl-6-(1,2,3,4,7,8,9,10-octahydro-7,7,10,10-tetramethylbenzo[f]quinolin-4-yl)hexa-2,4-dienoic acid** (structure **50**, where $R_1$, $R_2$, $R_3$, $R_4$, and $R_{19}$ are methyl; $R_9$ and $R_{10}$ are hydrogen; $R_{15}$ is hydroxy; X and Y are carbon; m=1, and n=0)

The title compound was prepared from compound **143a** using the standard hydrolysis conditions in Example 2. Compound **143b** had $R_f$=0.50 (50% ether in hexane); amorphous solid HRMS Calc. $C_{26}H_{33}O_2N$ 367.2511, found 367.2504; $^1$H NMR (400 MHz, $CDCl_3$) δ 7.06 (d, J=8.7 Hz, 1H, ArH), 6.46 (d, J=8.7 Hz, 1H, ArH), 6.28 (d, J=15.7 Hz, 1H, olefinic), 6.22 (m, 1H, olefinic), 5.75 (s, 1H, olefinic), 3.95 (d, J=4.7 Hz, 2H, N-$CH_2$-allylic), 3.18 (t, J=6.2 Hz, 2H, N-$CH_2$), 2.90 (t, J=6.0 Hz, 2H, benzylic), 2.27 (s, 3H, $CH_3$), 1.90 (m, 2H, $CH_2$), 1.61 (m, 4H, $2CH_2$), 1.40 (s, 6H, $2CH_3$), 1.27 (s, 6H, $2CH_3$).

**EXAMPLE 69**

Preparation of compound **144b** according to Scheme II

**(2E, 4E)-3-Methyl-6-[(Z)-5,6,7,8-tetrahydro-5,5,8,8-tetramethylnaphthyl-(2,3-b-pyran-4-ylidene]hexa-2,4-dienoic acid** (structure **7**, where $R_1$, $R_2$, $R_3$, $R_4$ and $R_{19}$ are methyl; $R_5$, $R_6$, $R_7$, $R_8$, $R_9$ and $R_{10}$ are hydrogen; $R_{15}$ is hydroxy; X and Y are carbon; Z is oxygen; m=n=1).

The title compound was prepared in a manner similar to that of compounds **56a** and **56b** (Examples 10 and 11) except that 6,6,9,9-tetramethyl-6,7,8,9-tetrahydrobenzo[g]-chromene-4-one was used as the starting ketone (structure **1** in Scheme II). The synthesis of this intermediate ketone is detailed below.

A 200ml round bottom flask was flame dried under nitrogen and charged with sodium metal (3.2g, 140 mmol). A solution of 3-aceto-5,6,7,8-tetrahydro-5,5,8,8-tetramethyl-2-naphthol (15g, 61.0 mmol, from Example 7) in ethyl formate (350ml) was added dropwise over 1 h. The resulting yellow solution was stirred at 35°C for 4 h. The mixture was cooled to 25°C solution, diluted with 1N HCl (20ml) and extracted with ether. The extracts were washed with water, brine, and dried over $MgSO_4$. The extracts were concentrated under vacuum to give 2-hydroxy-6,6,9,9-tetramethyl-2,3,6,7,8,9-hexahydrobenzo[g]chromen-4-one structure **1** where $R_1$, $R_2$, $R_3$, and $R_4$ are methyl; $R_5$, $R_6$, and $R_7$ are hydrogen; $R_8$ is hydroxy; X and Y are carbon; Z is oxygen; and m=n=1)]: $^1$H NMR (400 MHz, $CDCl_3$) δ 7.85 (s, 1H), 6.9 (s, 1H), 5.85 (t, 1H), 3.32 (br s, 1H), 2.9 (dd, 2H), 1.67 (s, 4H), 1.27 (s, 12H).

To a solution of above benzochromen-4-one (19.6g, 71.5 mmol) in methanol (250ml) was added conc HCl (0.5ml) dropwise. The mixture was stirred at 60°C for 2.5 h. TLC analysis indicated the reaction was complete. The mixture was cooled to 25°C and diluted with water (200ml). A light brown solid precipitate was collected by filtration and dissolved in ether, washed with water, brine and dried over sodium sulfate. Concentration under vacuum gave 6,6,9,9-tetramethyl-6,7,8,9-tetrahydrobenzo[g]chromen-4-one (13.3g, 52.0 mmol, 73%) as a light brown solid: mp 202.1°C; $^1$H NMR (400 MHz, $CDCl_3$) δ 8.15 (s, 1H), 7.8 (d, 1H), 7.4 (s, 1H), 6.25 (d, 1H), 1.75 (s, 4H), 1.35 (s, 12H).

A solution of 6,6,9,9-tetramethyl-6,7,8,9-tetrahydrobenzo[g]chromen-4-one (4.4g, 17.2 mmol) in 1:1 EtOAc/methanol (250ml) containing 10% palladium on carbon (1:1g, 7% by weight) was stirred at room temperature under an atmosphere of hydrogen (balloon) for 14 h. TLC analysis indicated complete reaction. The mixture was filtered and concentrated to give a yellow oil. The oil was passed through a small column of silica (10% EtOAc in hexane) and concentration to give 6,6,9,9-tetramethyl-2,3,6,7,8,9-hexahydrobenzo[g]chromen-4-one (4.2g, 16.3 mmol, 95%) as a light yellow solid: $^1$H NMR (400 MHz, $CDCl_3$) δ 7.85 (s, 1H), 6.86 (s, 1H), 4.46 (t, 2H), 2.75 (t, 2H), 1.65 (s, 4H), 1.25 (s, 12H).

The above hexahydrobenzo[g]chromen-4-one was transformed to the title compound as outlined in Scheme II and Examples 10 and 11. Compound **144b** was obtained as a bright yellow solid: mp 228.5°C; $R_f$=0.26 (40% ether in hexane); $^1$H NMR (400 MHz, $CDCl_3$) δ 7.42 (dd, 1H), 7.38 (s, 1H), 6.8 (s, 1H), 6.4 (d, 1H), 6.07 (d, 1H), 5.32 (s, 1H), 4.32 (t, 2H), 2.66 (t, 2H), 2.35 (s, 3H), 1.68 (s, 4H), 1.28 (s, 6H), 1.26 (s, 6H).

## EXAMPLE 70

Preparation of compound **145b** according to Scheme II

**(2E,4E)-3-methyl-6-[(E)-3,4,5,6,7,8-hexahydro-2,2,5,5,8,8-hexamethyl-2H-anthracen-1-ylidene]hexa-2,4-dienoic acid** (structure **7**, where $R_1$, $R_2$, $R_3$, $R_4$, $R_5$, $R_6$,and $R_{19}$ are methyl; $R_7$, $R_8$, $R_9$, and $R_{10}$ are hydrogen; $R_{15}$ is hydroxy; X, Y, Z are carbon; m = n = 1)

The title compound was prepared in a manner similar to Example 10, except that 2,5,5,8,8-3,4,5,6,7,8-hexahydro-2,2,5,5,8,8-hexamethyl-2H-anthracen-1-one was used as the starting tricyclic ketone which was prepared by the following sequence.

The tricyclic ketone, 2,5,5,8,8-3,4,5,6,7,8-hexahydro-2,5,5,8,8-pentamethyl-2H-anthracen-1-one (0.640 g, 2.50 mmol) in THF (5.00 mL) was added dropwise to a prepared solution of LDA (2.75 mmol, 1.1 eq) in THF at -78°C. The resulting blue-green solution was stirred at -78°C for 45 min. Iodomethane (0.707 g, 5.00 mmol, 0.311 mL) was added dropwise and the solution was stirred for 1h at -78°C. The reaction solution was warmed to room temperature and became red in color. After stirring for 1.5h, the reaction solution was quenched with water and the aqueous layer extracted with EtOAc (3 x 10 mL). The combined organic extracts were washed with $H_2O$ and brine, dried ($MgSO_4$), filtered, and concentrated to give the crude methylated tricycle (0.687 g). Purification by radial chromatography (20:1 = Hex:THF) gave the dimethylated tricycle (0.090 g, 13%) as a white solid: $^1H$ NMR (400 MHz, $CDCl_3$) $\delta$ 8.02 (s, 1H, Ar-H), 7.13 (s, 1H, Ar-H), 2.92 (t, 2H, 2CH), 1.95 (t, 2H, 2CH), 1.67 (s, 4H, $CH_2$), 1.30 (s, 6H, $2CH_3$), 1.29 (s, 6H, $2CH_3$), 1.21 (s, 6H, $2CH_3$).

Procedures similar to those in Examples 10 and 11 afforded the compound **145b** as a yellow solid. $^1H$ NMR (400 MHz, $CDCl_3$) $\delta$ 7.22 (s, 1H, Ar-H), 7.18 (dd, J = 11.0, 15.2 Hz, 1H, olefinic), 7.07 (s, 1H, Ar-H), 6.41 (d, J = 15.2 Hz, 1H, olefinic), 6.28 (d, J = 11.0 Hz, 1H, olefinic), 5.80 (s, 1H, olefinic), 2.76 (t, J = 6.6 Hz, 2H, $CH_2$), 2.28 (s, 3H, $CH_3$), 1.69 (s, 4H, $CH_2$), 1.68 (t, J = 6.6 Hz, 2H, $CH_2$), 1.29 (s, 12H, $4CH_3$), 1.16 (s, 6H, $2CH_3$).

## EXAMPLE 71

Preparation of compound **146a** according to Scheme II

**Ethyl (2E,4E)-3-methyl-6-[(Z)-N-acetyl-3,4,5,6,7,8-hexahydro-10-amino-5,5,8,8-tetramethyl-2H-anthracen-1-ylidene]hexa-2,4-dienoate** (structure **7**, where $R_1$, $R_2$, $R_3$, $R_4$ and $R_{19}$ are methyl; $R_5$, $R_6$, $R_7$, $R_8$ and $R_{10}$ are hydrogen; $R_9$ is N-acetylamino; $R_{15}$ is ethoxy; X, Y, Z are carbon; m = n = 1)

The title compound was prepared in a manner similar to Example 10, except that 10-nitro-1,2,3,4,5,6,7,8-octahydro-5,5,8,8-tetramethylanthracen-1-one (from Example 8) was used as the starting tricyclic ketone and generation of the intermediate *cis*-olefinic ester was accomplished with catalytic TsOH in EtOH; the 10-nitrotricyclic *cis*-allylic alcohol was prepared from the ester as described in Example 10: $^1H$ NMR (400 MHz, $CDCl_3$) $\delta$ 7.32 (s, 1H, aromatic), 5.71 (t, J = 6.8 Hz, 1H, olefinic), 4.36 (collapsed dd, 2H, allylic), 2.59 (apparent t, J = 6.6 Hz, 2H, $CH_2$), 2.42 (m, 2H, $CH_2$), 1.91 (m, 2H, $CH_2$), 1.73 (m, 2H, $CH_2$), 1.68 (m, 2H, $CH_2$), 1.57 (s, 6H, $2CH_3$), 1.31 (s, 6H, $2CH_3$).

The intermediate 10-nitrotricyclic *cis*-allylic alcohol (250 mg, 0.76 mmol) in EtOH (4.5 mL) and water (1.0 mL) was treated with calcium chloride (54 mg) in water (0.1 mL) and zinc dust (1.63 g) at ambient temperature and the mixture was heated at reflux for 2 h. The hot mixture was filtered through a pad of celite and the filter pad was rinsed with hot EtOH (50 mL). The solution was concentrated *in vacuo*, diluted with water and extracted with EtOAc. The EtOAc layer was washed with water, saturated aqueous $NaHCO_3$, water, and brine. The solution was dried ($Na_2SO_4$), filtered, and concentrated to give 10-aminotricyclic *cis*-allylic alcohol, 225mg (99%) as a yellow oil: $^1H$ NMR (400 MHz, $CDCl_3$) $\delta$ 6.66 (s, 1H, aromatic), 5.56 (t, J = 6.5 Hz, 1H, olefinic), 4.46 (d, J = 6.5 Hz, 2H, allylic), 3.82 (broad s, 2H, $NH_2$), 2.51 (m, 2H, $CH_2$), 2.38 (m, 2H, $CH_2$), 2.04 (m, 2H, $CH_2$), 1.73 (m, 2H, $CH_2$), 1.64 (m, 2H, $CH_2$), 1.45 (s, 6H, $2CH_3$), 1.29 (s, 6H, $2CH_3$).

The amino alcohol (200 mg, 0.67 mmol), In methylene chloride (1 mL) and lutidine (0.2 mL) was treated with t-butyldimethylsilyl triflate (195 mg, 0.74 mmol) at ambient temperature and the solution was allowed to stir for 8 h. The reaction was quenched with saturated aqueous $NH_4Cl$ and diluted with EtOAc. The EtOAc layer was washed with water, saturated aqueous $NaHCO_3$, water, and brine. The solution was dried ($Na_2SO_4$), filtered, and concentrated to give the crude product. The product was purified by silica gel radial chromatography (20 : 1 = Hex : EtOAc) to give the 10-amino silyl protected *cis*-allylic alcohol 128 mg (46%) as a colorless oil: $^1H$ NMR (400 MHz, $CDCl_3$) $\delta$ 6.71 (s, 1H, aromatic), 5.57 (t, J = 6.2 Hz, 1H, olefinic), 4.53 (d, J = 6.2 Hz, 2H, allylic), 3.84 (broad s, 2H, $NH_2$), 2.54 (apparent t, J = 6.7 Hz, 2H, $CH_2$), 2.42 (m, 2H, $CH_2$), 2.06 (m, 2H, $CH_2$), 1.77 (m, 2H, $CH_2$), 1.68 (m, 2H, $CH_2$), 1.49 (s, 6H, $2CH_3$), 1.34 (s, 6H, $2CH_3$), 0.96 (s, 9H, $3CH_3$), 0.12 (s, 6H, $2CH_3$).

The 10-amino silyl protected *cis*-allylic alcohol (95 mg, 0.23 mmol), in methylene chloride (2 mL) and pyridine (0.3 mL) was treated with a catalytic amount of DMAP and acetic anhydride (1.15 mmol) and the solution was heated at reflux for 12 h. The reaction was quenched with the adition of water and diluted with EtOAc. The EtOAc layer was washed with water, saturated aqueous $NaHCO_3$, water, and brine. The solution was dried ($Na_2SO_4$), filtered, and concentrated to give the crude product. The product was purified by silica gel radial chromatography (1 : 1 = Hex : $Et_2O$) to give the N-acyl-10-amino silyl protected *cis*-allylic alcohol 67 mg (70%) as a colorless oil. The protected *cis*-allylic alcohol (0.16 mmol) was dissolved in THF (0.5 mL) and treated with tetrabutylammonium fluoride (1 M, 2.1 eq) at ambient temperature and the solution was allowed to stir for 8 h. The reaction was quenched with water and diluted with EtOAc. The EtOAc layer was washed with water, saturated aqueous $NaHCO_3$, water, and brine. The solution was dried ($Na_2SO_4$), filtered, and concentrated to give the crude product. The product was crystallized ($CH_2Cl_2$ / hexanes) to give the N-acyl-10-amino *cis*-allylic alcohol 44 mg (89%) as a yellow solid. The alcohol (0.13 mmol) was oxidized with $MnO_2$ (20 mg, 0.23 mmol) in methylene chloride (0.5 mL) at ambient temperature for 18 hours. The reaction was quenched with the addition of water and diluted with EtOAc. The EtOAc layer was washed with water, saturated aqueous $NaHCO_3$, water, and brine. The solution was dried ($Na_2SO_4$), filtered, and concentrated to give the N-acyl-10-amino *cis*-unsaturated aldehyde 43 mg (99%) as a yellow oil: $^1H$ NMR (400 MHz, $CDCl_3$) δ 9.78 (d, J = 7.9 Hz, 1H, CHO), 7.02 (s, 1H, aromatic), 6.02 (d, J = 7.9 Hz, 1H, olefinic), 2.69 (m, 2H, $CH_2$), 2.58 (m, 2H, $CH_2$), 2.23 (s, 3H, $CH_3$), 1.92 (m, 2H, $CH_2$), 1.69 (m, 4H, $2CH_2$), 1.40 (m, 6H, $2CH_3$), 1.27 (s, 6H, $2CH_3$).

Using the procedure described in Example 1, the above aldehyde was transformed into the title compound **146a**, which was obtained as a yellow oil: $^1H$ NMR (400 MHz, $CDCl_3$) δ 7.34 (s, 1H, aromatic), 7.13 (dd, J=15.1, 11.1 Hz, 1H, olefinic), 6.43 (d, J=15.1 Hz, 1H, olefinic), 6.15 (d, J=11.1 Hz, 1H, olefinic), 5.80 (broad s, 1H, olefinic), 4.14 (q, J=7.0 Hz, 2H, $-OCH_2$); 2.64 (m, 2H, $CH_2$), 2.48 (m, 2H, $CH_2$), 2.29 (s, 3H, $CH_3$), 2.22 (s, 3H, $CH_3$ allylic), 1.94 (m, 2H, $CH_2$), 1.87 (m, 2H, $CH_2$), 1.69 (m, 2H, $CH_2$), 1.30 (s, 12H, $4CH_3$); 1.28 (t, J=7.0 Hz, 3H, $CH_3$ ethyl).

## EXAMPLE 72

Preparation of compound **146b** according to Scheme II

**(2E,4E)-3-methyl-6-[(Z)-N-acetyl-3,4,5,6,7,8-hexahydro-10-amino-5,5,8,8-tetramethyl-2H-anthracen-1-ylidene]hexa-2,4-dienoic acid** (structure **7**, where $R_1$, $R_2$, $R_3$, $R_4$, and $R_{19}$ are methyl; $R_5$, $R_6$, $R_7$, $R_8$ and $R_{10}$ are hydrogen; $R_9$ is N-acetylamino; $R_{15}$ is hydroxy; X, Y, Z are carbon; m = n = 1)

Compound **143a** was hydrolyzed using the procedure of Example 2, to give the title compound **143b** 20.5 mg (30%) as a yellow film: $^1H$ NMR (400 MHz, $CDCl_3$) δ 7.36 (s, 1H, aromatic), 7.16 (dd, J=15.1, 11.1 Hz, 1H, olefinic), 6.73 (s, 1H, NH), 6.36 (d, J=15.1 Hz, 1H, olefinic), 6.18 (d, J=11.1 Hz, 1H, olefinic), 5.81 (broad s, olefinic), 2.64 (m, 2H, $CH_2$), 2.48 (m, 2H, $CH_2$), 2.29 (s, 3H, $CH_3$), 2.22 (s, 3H, $CH_3$), 1.94 (m, 2H, $CH_2$), 1.87 (m, 2H, $CH_2$), 1.69 (m, 2H, $CH_2$), 1.30 (s, 12H, $4CH_3$).

## EXAMPLE 73

Preparation of compound **147b** according to Scheme XIII **(2E,4E)-3-methyl-6-[(E)1-ethyl-6,6,9,9-tetramethyl-2,3,6,7,8,9-hexahydro- 1H-benzo[g] quinolin-4-ylidene]hexa-2,4-dienoic acid** (structure **7**, where $R_1$, $R_2$, $R_3$, $R_4$ and $R_{19}$ are methyl; $R_5$, $R_6$, $R_7$, $R_8$, $R_9$ and $R_{10}$ are hydrogen; $R_{15}$ is hydroxy; X, Y are carbon, Z is N-ethyl; m = n = 1)

The title compound was prepared in a manner similar to Example 1 except that 1-ethyl-6,6,9,9-tetramethyl-2,3,6,7,8,9-hexahydro-1H-benzo[g] quinolin-4-one was used as the starting tricyclic ketone, which was prepared by the following sequence.

Nitric acid (15.3g, 218mmol ) was added dropwise to 1,2,3,4-tetrahydro-1,1,4,4 tetramethylnaphthalene (13.7g, 72.9 mmol ) at 0 °C. Following this addition, the reaction mixture was allowed to warm to 25 °C over 2 hours. The reaction mixture was poured onto ice (200 g), neutralized by addition of sodium carbonate and extracted with ethyl acetate. Removal of solvent under reduced pressure yielded a light brown oil which crystallized upon standing (15.7g, 68.0 mmol, 93% ): $^1H$ NMR (400 MHz, $CDCl_3$) δ 8.17 (d, 1H, ArH), 7.95 (dd, 1H, ArH), 7.45 (d, 1H, ArH), 1.75 (br s, 4H, $CH_2$), 1.32 (s, 6H, $CH_3$), 1.28(s, 6H, $CH_3$).

A solution of 1,1,4,4-tetramethyl-6-nitro-1,2,3,4-tetrahydronaphthalene (15.7g, 68.0 mmol) and 10% palladium on carbon (1.57g ) in ethyl acetate (190ml) was stirred under an atmosphere of hydrogen at 25 °C for 24 hours. Filtration through celite and removal of solvent under reduced pressure yielded the amine as a golden brown oil (13.2g, 65.7 mmol, 96%): $^1H$ NMR (400 MHz, $CDCl_3$) δ 7.10 (d, 1H, ArH), 6.63(d, 1H, ArH), 6.50 (dd, 1H, ArH), 3.50 (br s, 2H, $NH_2$), 1.65 (br s, 4H, $CH_2$), 1.28 (s, 6H, $CH_3$), 1.26(s, 6H, $CH_3$).

A solution of 2-amino-5,5,8,8-tetramethyl-5,6,7,8-tetrahydronaphthalene (3.0g, 14.8 mmol) and acrylic acid

(1.0g, 14.8 mmol ) in toluene (60ml) was heated to reflux for 14 hours. Removal of solvent under reduced pressure yielded a viscous brown oil (3.0g, 10.9 mmol, 74%): $^1H$ NMR (400 MHz, $CDCl_3$) δ 7.16 (d, 1H, ArH), 6.62 (d, 1H, ArH), 6.54 (dd, 1H, ArH), 3.48 (t, 2H, N-$CH_2$), 2.68 (t, 2H,$CH_2$), 1.67 (br s, 4H, $CH_2$), 1.28 (s, 6H, $CH_3$), 1.26(s, 6H, $CH_3$).

2-amino-3-(5,5,8,8-tetramethyl-5,6,7,8-tetrahydronaphthalene)-propionic acid ( 3.0g, 10.9 mmol ) was dissolved into PPA (100ml) and stirred at 100 °C for 16 hours. The red reaction mixture was poured onto ice (200 g), neutralized by addition of sodium carbonate and extracted with ethyl acetate. Removal of solvent under reduced pressure yielded a yellow semisolid. Column chromatography (10% EtOAc/Hex) gave the desired product as a yellow solid (2.2g, 8.6 mmol, 79%): $^1H$ NMR (400 MHz, $CDCl_3$) δ 7.80 (s, 1H, ArH), 6.60 (s, 1H, ArH), 3.55 (t, 2H, N-$CH_2$), 2.68 (t, 2H,$CH_2$), 1.65 (br s, 4H, $CH_2$), 1.28 (s, 6H, $CH_3$), 1.26(s, 6H, $CH_3$).

A solution of 6,6,9,9-tetramethyl-2,3,6,7,8,9-tetrahydro-1H-benzo[g] quinoline-4-one (255mg, 1.0 mmol), iodoethane (2.0g, 12.4 mmol), and anhydrous potassium carbonate (1.4g, 10.0 mmol) in DMA (50ml) was stirred at 50-60 °C for 48 hours. The reaction mixture was diluted with water and extracted with ethyl acetate. Removal of solvent under reduced pressure yielded a yellow/brown oil (150mg) which partially solidified on standing. Column chromatography (10% -20% EtOAc/Hex) gave the desired product as a bright yellow/green solid (80mg, 0.28 mmol, 28%): $^1H$ NMR (400 MHz, $CDCl_3$) δ 7.85 (s, 1H, ArH), 6.63 (s, 1H, ArH), 3.40 (q, 2H, N-$CH_2$), 3.38 (t, 2H, N-$CH_2$), 2.65 (t, 2H,$CH_2$), 1.65 (br s, 4H, $CH_2$), 1.30 (s, 6H, $CH_3$), 1.28(s, 6H, $CH_3$), 1.16 (t, 3H, $CH_3$)

Procedures similar to those in Examples 1 and 2 afforded the title compound **147b** as a orange semi-solid material. The final product was purified by HPLC ( 90/10 MeOH/$H_2O$) to give a yellow solid.: $^1H$ NMR (400 MHz, $CDCl_3$) δ 7.46 (s, 1H, ArH), 7.05 (dd, 1H, CH), 6.62 (d, 1H, CH), 6.56 (s, 1H, ArH), 6.42 (d, 1H, CH), 5.83 (br s, 1H, CH), 3.38 (q, 2H, N-$CH_2$), 3.26 (t, 2H, N-$CH_2$), 2.83 (t, 2H,$CH_2$), 2.40 (s, 3H, $CH_3$), 1.67 (br s, 4H, $CH_2$), 1.28 (s, 6H, $CH_3$), 1.26(s, 6H, $CH_3$), 1.15 (t, 3H, $CH_3$).

## EXAMPLE 74

Preparation of compound **148b** according to Scheme XIII

**T-butyl-4-(5-carboxy-penta-2E-4E-dieneylidene)-6,6,9,9-tetramethyl-3,4,6,7,8,9-hexahydro-2H-benzo[g] quinolin-1-carboxylate** (structure **54**, where $R_1$, $R_2$, $R_3$, $R_4$, and $R_{19}$ are methyl; $R_5$, $R_7$, $R_8$, $R_9$, $R_{10}$, $R_{26}$, $R_{27}$, $R_{28}$, and $R_{29}$, are hydrogen; $R_{15}$ is hydroxy; X, Y are carbon, Z is N-1-carboxylic acid tert-butyl ester; m = n = 1)

The title compound was prepared as in Example 73 except for the nitrogen alkylation step which involved the use of t-butylchloroformate. Following the standard hydrolysis procedure of Example 2 gave 50 mg of the title compound obtained as a yellow orange oil. Column chromatography (40% Diethyl ether/Hexane) gave the desired product as a yellow film: $^1H$ NMR (400 MHz, $CDCl_3$) δ 7.57 (s, 1H, ArH), 7.43 (s, 1H, ArH), 6.98 (dd, 1H, CH), 6.72 (d, 1H, CH), 6.45 (d, 1H, CH), 5.80 (s, 1H, CH), 3.80 (t, 2H, N-$CH_2$), 2.90 (t, 2H, $CH_2$), 2.38(s, 3H, $CH_3$), 1.70 (br s, 4H, $CH_2$), 1.50 (s, 9H, $CH_3$), 1.30 (s, 6H, $CH_3$), 1.26(s, 6H, $CH_3$).

## EXAMPLE 75

Preparation of compound **149b** according to Scheme XVI

**(2E,4E)-3-methyl-(6,6,9,9-tetramethyl-2,3,6,9-tetrahydronaphtho[2,3-b]-[1,4]oxazin-4-yl)-hexa-2,4-dienoic acid** (structure 68, where $R_1$, $R_2$, $R_3$, $R_4$, and $R_{19}$ are methyl; $R_5$, $R_6$, $R_7$, $R_8$, $R_9$, $R_{10}$, $R_{26}$, $R_{27}$, $R_{28}$, and $R_{29}$, are hydrogen; $R_{15}$ is hydroxy; X, Y are carbon, W is nitrogen, Z is oxygen; m = n = 1)

To a solution of 6-6-9-9-tetramethyl-6,9-dihydro-4H-naphtho[2,3-b][1,4]oxazin-3-one (400 mg, 1.5 mmol) [prepared by Friedel Crafts alkylation/annulation of 2H-1,4-benzoxazin-3-(4H)-one with 2,5-dichloro-2,5-dimethyl hexane in the presence of aluminum chloride at ambient temperatue (24°C) in dichloromethane] in THF at ambient temperature was added $LiAlH_4$ (4.6 ml, 4.6 mmol). The reaction mixture was allowed to stir and then was heated to 80°C for 30min. The reaction mixture was poured over saturated sodium potassium tartrate solution (100 ml), extracted with EtOAc (100 ml), dried with anhydrous $MgSO_4$ (0.5 g), and concentrated to give 6,6,9,9-tetramethyl-3,4,6,9-tetrahydro-2H-naphtho[2,3-b][1,4]oxazine (300 mg, 70% yield of theory): $^1H$ NMR (400 MHz, $CDCl_3$) δ 6.70 (s, 1H, benzylic), 6.52 (s, 1H, benzylic), 4.23 (t, 2H, J = 2.8, 1.6 Hz, ring $CH_2$), 3.39 (t, 2H, J = 4.4, 6.8 Hz, ring $CH_2$), 1.63 (s, 4H, 2 $CH_2$), 1.22 (s, 12H, 4 $CH_3$).

To a solution of ethyl-6-bromohexa-2,4-dienoate (45 mg, 0.19 mmol) in THF, at ambient temperature was added NaH (4 mg, 0.14 mmol) under $N_2$(gas). The solution was stirred for 10 min and then added to 6,6,9,9-tetramethyl-3,4,6,9-tetrahydro-2H-naphtho[2,3-b][1,4]oxazine (24 mg, 0.1mmol) at ambient temperature. The reaction was then heated to 65°C overnight. The reaction was then quenched over saturated $NH_4Cl$ solution (100 ml) extracted with

EtOAc (100 ml), dried with anhydrous $MgSO_4$ (0.5 g), and concentrated. The product ran on prep tlc plates (20% EtOAc/ Hexane) to give ethyl-3-methyl-(6,6,9,9-tetramethyl-2,3,6,9-tetrahydronaphtho [2,3-b][1,4]oxazin-4-yl)hexa-2,4-dienoate (25 mg, 89% yield of theory): $^1$H NMR (400 MHz, $CDCl_3$) δ 6.71 (s, 1H, benzylic), 6.56 (s, 1H, benzylic), 6.31 (d, 1H, J= 15.6 Hz, olefinic), 6.17 (dt, 1H, olefinic), 5.57 (s, 1H, olefinic), 4.25 (t, 2H, J = 4, 4 Hz, N-$CH_2$), 4.17 (q, 2H, $CH_2CH_3$), 3.95 (d, 2H, J = 6.8 Hz, N-$CH_2$), 3.28 (t, 2H, J = 4 Hz, ring $CH_2$), 2.27 (s, 3H, $CH_3$), 1.63 (s, 4H, 2$CH_2$), 1.28 (t, 3H, $CH_3$), 1.22 (s, 6H, 2$CH_3$), 1.21 (s, 6H, 2$CH_3$).

Standard hydrolysis conditions, as in Example 2 were used to obtain 3-methyl-(6,6,9,9-tetramethyl-2,3,6,9-tetrahydronaphtho[2,3-b][1,4]oxazin-4-yl)hexa-2,4-dienoic acid (12.5 mg, 88% of theory): $^1$H NMR (400 MHz, $CDCl_3$) δ 6.71 (s, 1H, benzylic), 6.56 (s, 1H, benzylic), 6.31 (d, 1H, J = 15.6 Hz, olefinic), 6.17 (dt, 1H, olefinic), 5.57 (s, 1H, olefinic), 4.25 (t, 2H, J = 4,4 Hz, N-$CH_2$), 3.95 (d, 2H, J = 6.8 Hz, ring $CH_2$), 3.28 (t, 2H, J = 4 Hz, ring $CH_2$), 2.27 (s, 3H, $CH_3$), 1.63 (s, 4H, 2$CH_2$), 1.22 (s, 6H, 2$CH_3$), 1.21 (s, 6H, 2$CH_3$).

## EXAMPLE 76

Preparation of compound **150b** according to Scheme XVI

**(2E,4E)-3-methyl-6-oxo-6-(6,6,9,9-tetrahydro-2,3,6,9-tetrahydro-naphtho[2,3-b][1,4]oxazin-4-yl)hexa-2,4-dienoic acid** (structure 69, where $R_1$, $R_2$, $R_3$, $R_4$, and $R_{19}$ are methyl; $R_5$, $R_6$, $R_7$, $R_8$, $R_9$, $R_{10}$, $R_{26}$, $R_{27}$, $R_{28}$, and $R_{29}$, are hydrogen; $R_{15}$ is hydroxy; X, Y are carbon, W is nitrogen, Z is oxygen; m = n = 1).

To a solution of 6,6,9,9-tetramethyl-3,4,6,9-tetrahydro-2H-naphtho[2,3-b][1,4]oxazine (prepared in Example 75, 100 mg, 0.408 mmol) was added hexa-2,4-dienedioic acid mono ethylester (90 mg, 0.489 mmol) in dichloromethane, at ambient temperature. To this solution was added DMAP (50 mg, 0.489 mmol), CSA (47 mg, 0.204 mmol), and DCC (100 mg, 0.489 mmol). The reaction was stirred at ambient temperature for 30 min. EtOAc was added, and the reaction mixture was filtered and evaporated. Prep TLC was used (20% EtOAc / Hexane) to give ethyl-3-methyl-6-oxo-6-(6,6,9,9-tetrahydro-2,3,6,9-tetrahydro-naphtho[2,3-b][1,4]oxazin-4-yl)hexa-2,4-dienoate (30 mg, 40% yield of theory): 1H NMR (400 MHz, $CDCl_3$) δ 7.40 (d, 1H, olefinic), 6.90 (br s, 1H, benzylic), 6.86 (d, 1H, olefinic), 6.85 (s, 1H, benzylic), 6.09 (d, 1H, olefinic), 4.29 (t, 2H, ring $CH_2$), 4.21 (q, 2H, $CH_2$), 4.04 (t, 2H, ring $CH_2$) 2.25 (s, 3H, $CH_3$), 1.62(s, 4H, 2$CH_2$), 1.30 (t, 3H, $CH_3$), 1.28(s, 6H, 2$CH_3$), 1.22 (s, 6H, 2$CH_3$).

Standard hydrolysis conditions, as in Example 2, were used to obtain 3-methyl-6-oxo-6-(6,6,9,9-tetrahydro-2,3,6,9-tetrahydronaphtho[2,3-b][1,4]oxazin-4-yl)hexa-2,4-dienoic acid (18 mg, 40% yield of theory): $^1$H NMR (400 MHz, $CDCl_3$) 7.43 (d, 1H, olefinic), 6.92 (br s, 1H, benzylic), 6.90 (d, 1H, olefinic), 6.86 (s, 1H, benzylic), 6.13(s, 1H, olefinic), 4.31 (t, 2H, ring $CH_2$), 4.04 (t, 2H, ring $CH_2$), 2.27 (s, 3H, $CH_3$), 1.67 (s, 4H, 2$CH_2$), 1.26 (s, 6H, 2$CH_3$), 1.22 (s, 6H, 2$CH_3$).

## EXAMPLE 77

Preparation of compound **151a** according to Scheme XVII

**(2E,4E)-3-methyl-6-(6-ethyl-1,9,9-trimethyl-7-oxo-2,3,6,7,8,9-hexahydro-1H-pyrido[2,3-g]quinolin-4-ylidene)hexa-2,4-dienoic acid** (structure 76, where $R_1$ is ethyl; $R_2$, $R_3$, $R_4$, $R_{19}$ and $R_{24}$ are methyl; $R_5$, $R_7$, $R_8$, $R_9$, $R_{10}$ and $R_{28}$ are hydrogen; $R_{15}$ is hydroxy; X, Z are N; Y is carbon; m = n = 1)

To a solution of aniline (19.73g, 0.212mol) in $CH_2Cl_2$ (200ml) at 0 °C was added dropwise 3,3-dimethyl acryloyl chloride (25.0g, 0.212mol) and triethylamine (21.4g, 0.212mol) at equal rates. This mixture was allowed to warm to 25 °C and stirred at for 17 hours. The reaction mixture was diluted with water and extracted with ethyl acetate. Removal of solvent under reduced pressure yielded the amide as a fine white fibrous solid (28.9g, 0.165mol, 78%). $^1$H NMR (400 MHz, $CDCl_3$) δ 7.52 (br d, 2H, ArH), 7.40 (br s, 1H, NH), 7.28 (t, 2H, ArH), 7.05 (t, 1H, ArH), 5.72 (s, 1H, CH), 2.20 (s, 3H, $CH_3$), 1.86 (s, 3H, $CH_3$).

To a solution of 3-methylbut-2-enoic acid phenylamide (10.0g, 0.057mol) in $CH_2Cl_2$ (150ml) at 25 °C was added aluminum chloride (5.0g, 0.037mol). This mixture was heated to reflux and stirred for 6 hours. The reaction mixture was poured onto ice (30g) extracted with $CH_2Cl_2$ . Removal of solvent under reduced pressure yielded the lactam as an off white-solid (9.5g, 0.054mol, 95%): $^1$H NMR (400 MHz, $CDCl_3$) δ 9.10 (br s, 1H, NH), 7.32 (d, 1H, ArH), 7.18 (t, 1H, ArH), 7.05 (t, 1H, ArH), 6.85 (d, 1H, ArH), 2.25 (s, 2H, $CH_2$), 1.35 (s, 6H, $CH_3$).

4,4-dimethyl-3,4-dihydro-1H-quinolin-2-one (4.5g, 0.0257mol) was dissolved in sulfuric acid (30ml) and cooled to 10 °C. To this solution was added dropwise fuming nitric acid(2.16g, 0.031mol). This mixture was stirred at 10 °C for 30 min and at 25 °C for 2 hours. The reaction mixture was poured onto ice (50g) and the solid nitro lactam was filtered and rinsed with saturated sodium bicarbonate and water. The nitro lactam was collected as a fine brown powder (5.5g,

0.025mol, 97%). $^1H$ NMR (400 MHz, $CDCl_3$) δ 8.76 (br s, 1H, NH), 8.21 (d, 1H, ArH), 8.10 (dd, 1H, ArH), 6.90 (d, 1H, ArH), 2.57 (s, 2H, $CH_2$), 1.40 (s, 6H, $CH_3$).

A solution of 4,4-dimethyl-6-nitro-3,4-dihydro-1H-quinolin-2-one (3.0g, 0.0136mol) in THF (20ml) was added to a solution of sodium hydride (420mg, 0.017mol) in THF (30ml) at 25 °C under nitrogen. This mixture was stirred for 1.5 hours and iodomethane (12.8g, 0.082mol) was added all at once. The reaction mixture was heated to 50 °C for 20 hours, quenched by slow addition of water and extracted with ethyl acetate. Removal of solvent under reduced pressure afforded the ethyl lactam as a brown solid (4.5g). This material was not purified. $^1H$ NMR (400 MHz, $CDCl_3$) δ 8.19 (d, 1H, ArH), 8.17 (dd, 1H, ArH), 7.12 (d, 1H, ArH), 4.10 (q, 2H, $CH_2$), 2.57 (s, 2H, $CH_2$), 1.35 (s, 6H, $CH_3$), 1.27 (t, 3H, $CH_3$).

1-ethyl-4,4-dimethyl-6-nitro-3,4-dihydro-1H-quinolin-2-one (4.1 g crude) was suspended in a 75% solution of ethanol\water (20ml). To this mixture was added calcium chloride monohydrate (2.0g, 0.0136mol) and zinc dust (30g,0.461mol). The reaction mixture was heated at reflux for 4 hours and stirred at 25 °C for an additional 14 hours. The mixture was diluted with water, filtered and extracted with ethyl acetate. Removal of solvent under reduced pressure gave a brown oil (3.5g). Column chromatography (50% EtOAc/hex) afforded the amino lactam as a yellow oil (1.8g, 8.3mmol). $^1H$ NMR (400 MHz, $CDCl_3$) δ 6.84 (d, 1H, ArH), 6.66 (d, 1H, ArH), 6.57 (dd, 1H, ArH), 3.97 (q, 2H, $CH_2$), 3.57 (br s, 2H, $NH_2$), 2.43 (s, 2H, $CH_2$), 1.27 (s, 3H, $CH_3$), 1.25 (s, 3H, $CH_3$), 1.23(t, 3H, $CH_3$).

A solution of 6-amino-(1-ethyl-4,4-dimethyl-3,4-dihydro-1H-quinolin-2-one) (750mg, 3.5mmol) and acrylic acid (252mg, 3.5mmol ) in toluene (10ml) was heated to reflux for 28 hours. Removal of solvent under reduced pressure gave a brown oil (900mg). Column chromatography (5% - 10% MeOH/ $CHCl_3$) afforded the amino lactam as a yellow oil (597mg, 2.1mmol, 60%): $^1H$ NMR(400 MHz, $CDCl_3$) δ 6.90(d, 1H, ArH), 6.63(d, 1H, ArH), 6.54 (dd, 1H, ArH), 3.98(q, 2H, $CH_2$, 3.46 (t, 2H, N-$CH_2$), 2.70 (t, 2H, $CH_2$), 2.45 (s, 2H, $CH_2$), 1.27 (s, 3H, $CH_3$), 1.25 (s, 3H, $CH_3$), 1.22(t, 3H, $CH_3$).

3-(1-ethyl-4,4-dimethyl-2-oxo-1,2,3,4-tetrahydro-quinolin-6-yl-amino)propionic acid (597mg, 2.1mmol) was dissolved into PPA (5ml) and stirred at 100 °C for 16 hours. The red reaction mixture was poured onto ice (20g), neutralized by addition of sodium carbonate and extracted with ethyl acetate. Removal of solvent under reduced pressure gave the tricyclic ketone as a yellow semi-solid. Column chromatography (3% MeOH/ $CHCl_3$) afforded the desired product as a yellow oil (340mg, 1.3 mmol, 62%) $^1H$ NMR (400 MHz, $CDCl_3$) δ 7.50(s, 1H, ArH), 6.63 (s, 1H, ArH), 4.33 (br s, 1H, NH), 4.03 (q, 2H, $CH_2$), 3.60 (t, 2H, N-$CH_2$), 2.70 (t, 2H, $CH_2$), 2.43 (s, 2H, $CH_2$), 1.27 (s, 3H, $CH_3$), 1.25 (s, 3H, $CH_3$), 1.22(t, 3H, $CH_3$)

Conversion of the tricylic ketone **53** to the title compound **76** followed the procedures given in Examples 1 and 2. TLC (50% EtOAc/hex) afforded the desired 6-(6-ethyl-1,9,9-trimethyl-7-oxo-2,3,6,7,8,9-hexahydro-1H-pyrido [2,3-g] quinolin-4-ylidene)-3-methyl-hexa-2,4-dienoic acid (5mg) as a red orange film. $^1H$ NMR (400 MHz, $CDCl_3$) δ 7.00 (dd, 1H, CH), 6.84 (s, 1H, ArH), 6.63 (s, 1H, ArH), 6.57 (d, 1H, CH), 6.37(d, 1H, CH), 5.79 (s,1H, CH), 3.37 (q, 2H, $CH_2$), 3.20 (t, 2H, N-$CH_2$), 3.10 (t, $2H_2$ $CH_2$), 2.83 (s, 3H, N-$CH_3$), 2.80 (s, 3H, $CH_3$), 2.38 (s, 2H, $CH_2$), 1.35 (s, 6H, $CH_3$), 1.25 (t, 3H, $CH_3$).

## EXAMPLE 78

Preparation of compound **152b** according to Scheme XVIII

**E-4-[N'-(5,5,8,8-tetramethyl-3,4,5,6,7,8-hexahydro-2H-anthracen-1-ylidene)-hydrazino]benzoic acid** (structure 78, where $R_1$, $R_2$, $R_3$, and $R_4$ are methyl; $R_5$, $R_6$, $R_7$, $R_8$, $R_9$, $R_{10}$, $R_{26}$, $R_{27}$, $R_{28}$ and $R_{29}$ are hydrogen; $R_{15}$ is hydroxy; $R_{14}$ is 4-benzoic acid,W is N, X, Y, Z are carbon; m = n = 1)

The title compound was prepared from the tricyclic ketone **60** prepared in Example 1. A solution of 6,6,9,9-tetramethyl-1,2,6,7,8,9-hexahydro-3H-anthracen-1-one (100 mg, 0.39 mmol) and 4-hydrazinobenzoic acid (62.5 mg, 0.41 mmol) and one drop of conc. hydrochloric acid in 5 mL of ethanol was heated to reflux for 3.5h, then cooled to room temperature. The solvent was removed *in vacuo* to a solid residue that was subjected to flash chromatography (silica gel, hexanes-ethyl acetate, 70:30) which gave 40 mg (26%) of the desired product **1** as a light yellow solid. The geometry of the hydrazone was determined by NOE experiments. $^1H$ NMR (400 MHz, $CDCl_3$) δ 8.16 (s, 1H, hydrazone N-H), 8.05 (d, J = 8.8 Hz, 2H, benzoic acid-3,4-H's), 7.64 (s, 1H, 10-H), 7.18 (d, J = 8.8 Hz, 2H, benzoic acid-1,5-H's), 7.06 (s, 1H, 5-H), 2.74 (t, J = 6.0 Hz, 2H, 2-$CH_2$), 2.59 (t, J = 6.Hz, 2H, 4-$CH_2$), 2.01 (m, 2H, 3-$CH_2$), 1.70 (s, 4H, 7,8-$CH_2$'s), 1.36 (s, 6H), 1.29 (s, 6H).

**EXAMPLE 79**

Preparation of compound **153b** according to Scheme XVIII

**E-4-[N'-(6,6,9,9-tetramethyl-2,3,6,7,8,9-hexahydro-benzo[g]chromen-4-ylidene)-hydrazino]benzoic acid** (structure 78, where $R_1$, $R_2$, $R_3$, and $R_4$ are methyl; $R_5$, $R_6$, $R_9$, and $R_{10}$ are hydrogen; $R_{15}$ is hydroxy; $R_{14}$ is para-amino benzoate, G is C, W is N, X, Y, are carbon, Z is O, m = n = 1)

A solution of 1,2,3,4-tetrahydro-6,6,9,9-tetramethyl naphto (2,3) pyran-4-one (56 mg, 0.215 mmol) and 4-hydrazinobenzoic acid (50 mg, 0.323 mmol) and one drop of conc. hydrochloric acid in 2 mL of ethanol was heated to reflux for 3.5h, then cooled to rt. The solvent was removed *in vacuo* to afford a solid residue that was subjected to flash chromatography (silica gel, hexanes-ethyl acetate, 60:40) to give 1.7 mg (2%) of the desired product **4** as a light yellow solid. $^1H$ NMR (400 MHz, $CDCl_3$) $\delta$ 8.05 (d, J =7.9 Hz, 2H, benzoic acid-3,4-H's), 8.04 (s, 1H, hydrazone N-H), 7.53 (s, 1H, 5-H), 7.18 (d, J = 7.9 Hz, 2H, benzoic acid-1,5-H's), 6.84 (s, 1H, 10-H), 4.30 (t, 2H, J = 6.0 Hz, 2-$CH_2$), 2.75 (t, J = 6.0 Hz, 2H, 3-$CH_2$), 1.69 (s, 4H, 7,8-$CH_2$'s), 1.35 (s, 6H), 1.27 (s, 6H).

**EXAMPLE 80**

Preparation of compound **154b** according to Scheme XVII

**(2E,4E)-3-methyl-6-(6-ethyl-1,9,9-trimethyl-2,3,6,9-tetrahydro-1H-pyrido[2,3-g]quinolin-4-ylidene)hexa-2,4-dienoic acid** (structure 77, where $R_1$ is ethyl; $R_3$, $R_4$, $R_{19}$ and $R_{24}$ are methyl; $R_5$, $R_7$, $R_8$, $R_9$, $R_{10}$ and $R_{28}$ are hydrogen; $R_{15}$ is hydroxy; X, Z are N; Y is carbon; m = n = 1)

The title compound was prepared as a by-product from the synthesis of compound **151b** (Example 77). To a solution of 6-ethyl-(1,9,9,-trimethyl-7-oxo-2,3,6,7,8,9-hexahydro-1H-pryido [2,3-g] quinolin-4-ylidene)acetonitrile (50mg, 0.168mol), in anhydrous toluene (4ml) and anhydrous hexanes (14ml) at -78 °C was added DIBAL in hexanes (0. 5ml, 0. 5mol). This mixture was stirred at -30 °C for 3 hours, quenched by slow addition of saturated sodium potassium tatrate, 1 N HCL, and extracted with ethyl acetate. Removal of solvent under reduced pressure afforded a mixture of aldehydes as an red orange oil (50mg). $^1H$ NMR confirmed the presence of aldehyde protons as well as vinyl protons which correspond to enamine formed by partial reduction of the amide. This material was not purified and used crude in subsequent reactions.

Following the standard procedure demonstrated in Examples 1 and 2, the mixture of aldehydes was carried through to the hydrolysis of the ester products. The crude mixture of esters above was hydrolyzed under standard conditions to give a mixture of trienoic acids. TLC (50% EtOAc/hex) afforded the 6-(6-ethyl-1,9,9-trimethyl-2,3,6,9-tetrahydro-1H-pyrido [2,3-g] quinolin-4-ylidene)-3-methylhexa-2,4-dienoic acid (4mg) as a red film: $^1H$ NMR (400 MHz, $CDCl_3$) d7.10 (dd, 1H, CH), 6.84 (s, 1H, ArH), 6.72 (s, 1H, ArH), 6.57 (d, 1H, CH), 6.40(d, 1H, CH), 6.00 (d, 1H, CH), 5.82 (s,1H, CH), 4,42 (d, 1H, CH), 3.25 (q, 2H, $CH_2$), 3.23 (t, 2H, N-$CH_2$), 2.90 (t, 2H, $CH_2$), 2.37 (s, 3H, N-$CH_3$), 2.10 (s, 3H, $CH_3$), 1.38 (s, 3H, $CH_3$), 1.36 (s, 3H, $CH_3$), 1.25 (t, 3H, $CH_3$)

**Evaluation of Retinoid Receptor Subfamily Activity**

Utilizing the "cis-trans" or "co-transfection" assay described by Evans et al., Science, 240:889-95 (May 13, 1988), the disclosure of which is herein incorporated by reference, the retinoid compounds of the present invention were tested and found to have strong, specific activity as either selective RAR agonists, selective RXR agonists, or as pan-agonist activators of both RAR and RXR receptors. This assay is described in further detail in U.S. Patent Nos. 4,981,784 and 5,071,773, the disclosures of which are incorporated herein by reference.

The co-transfection assay provides a method for identifying functional agonists which mimic, or antagonists which inhibit, the effect of native hormones, and quantifying their activity for responsive IR proteins. In this regard, the co-transfection assay mimics an *in vivo* system in the laboratory. Importantly, activity in the co-transfection assay correlates very well with known *in vivo* activity, such that the co-transfection assay functions as a qualitative and quantitative predictor of a tested compounds *in vivo* pharmacology. See, e.g., T. Berger et al. 41 J. Steroid Biochem. Molec. Biol. 773 (1992), the disclosure of which is herein incorporated by reference.

In the co-transfection assay, a cloned cDNA for an IR (e.g., human RAR$\alpha$, RAR$\beta$, RXR$\gamma$) under the control of a constitutive promoter (e.g., the SV 40 promoter) is introduced by transfection (a procedure to induce cells to take up foreign genes) into a background cell substantially devoid of endogenous IRs. This introduced gene directs the recipient cells to make the IR protein of interest. A second gene is also introduced (co-transfected) into the same cells in conjunction with the IR gene. This second gene, comprising the cDNA for a reporter protein, such as firefly luciferase

(LUC), controlled by an appropriate hormone responsive promoter containing a hormone response element (HRE). This reporter plasmid functions as a reporter for the transcription-modulating activity of the target IR. Thus, the reporter acts as a surrogate for the products (mRNA then protein) normally expressed by a gene under control of the target receptor and its native hormone.

The co-transfection assay can detect small molecule agonists or antagonists of target IRs. Exposing the transfected cells to an agonist ligand compound increases reporter activity in the transfected cells. This activity can be conveniently measured, e.g., by increasing luciferase production, which reflects compound-dependent, IR-mediated increases in reporter transcription. To detect antagonists, the co-transfection assay is carried out in the presence of a constant concentration of an agonist to the target IR (e.g., all-*trans* retinoic acid for RARα) known to induce a defined reporter signal. Increasing concentrations of a suspected antagonist will decrease the reporter signal (e.g., luciferase production). The co-transfection assay is therefore useful to detect both agonists and antagonists of specific IRs. Furthermore, it determines not only whether a compound interacts with a particular IR, but whether this interaction mimics (agonizes) or blocks (antagonizes) the effects of the native regulatory molecules on target gene expression, as well as the specificity and strength of this interaction.

The activity of the retinoid compounds of the present invention were evaluated utilizing the co-transfection assay according to the following illustrative Example.

**EXAMPLE 81**

**Co-transfection assay**

CV-1 cells (African green monkey kidney fibroblasts) were cultured in the presence of Dulbecco's Modified Eagle Medium (DMEM) supplemented with 10% charcoal resin-stripped fetal bovine serum then transferred to 96-well microtiter plates one day prior to transfection.

To determine RAR and/or RXR agonist activity of the compounds of the present invention, the CV-1 cells were transiently transfected by calcium phosphate coprecipitation according to the procedure of Berger et al., 41 *J.Steroid Biochem. Mol. Biol.*, 733 (1992) with the following receptor expressing plasmids: pRShRARα: Giguere *et al.*, 330 Nature, 624 (1987); pRShRARβ and pRShRARγ, Ishikawa *et al.*, 4 Mol. Endocrin., 837 (1990); pRShRXRα, Mangelsdorf *et al.*, 345 Nature, 224 (1990); and pRSmRXRβ and pRSmRXRγ, Mangelsdorf *et al.*, 6 Genes & Devel., 329 (1992), the disclosures of which are herein incorporated by reference. Each of these receptor expressing plasmids was co-transfected at a concentration of 5 ng/well, along with a basal reporter plasmid at 100 ng/well, the internal control plasmid pRS-β-Gal at 50 ng/well and filler DNA, pGEM at 45 ng/well.

The basal reporter plasmid D-MTV-LUC (Hollenberg and Evans, 55 Cell, 899 (1988), the disclosure of which is herein incorporated by reference) containing two copies of the TRE-palindromic response element described in Umesono *et al.*, 336 Nature, 262 (1988), the disclosure of which is herein incorporated by reference, was used in transfections for the RARs, and the reporter plasmid CRBPIIFKLUC, which contains an RXRE (retinoid X receptor response element, as described in Mangelsdorf *et al.*; 66 Cell, 555 (1991), the disclosure of which is herein incorporated by reference), was used in transfections for the RXRs. Each of these reporter plasmids contains the cDNA for firefly luciferase (LUC) under constitutive promoter containing the appropriate RAR or RXR response element. As noted above, pRS-β-Gal, coding for constitutive expression of E. coli β-galactosidase (β-Gal), was included as an internal control for evaluation of transfection efficiency and compound toxicity.

Six hours after transfection, media was removed and the cells were washed with phosphate-buffered saline (PBS). Media containing compounds of the present invention in concentrations ranging from $10^{-12}$ to $10^{-5}$ M were added to the cells. Similarly, the reference compounds all-*trans* retinoic acid (ATRA)(Sigma Chemical), a known RAR selective compound, and 9-*cis* retinoic acid (9-*cis*) (synthesized as described in Heyman *et al.*, *Cell*, 68:397-406 (1992)), a compound with known activity on RXRs, were added at similar concentrations to provide a reference point for analysis of the activity of the compounds of the present invention. Retinoid purity was established as greater than 99% by reverse phase high-performance liquid chromatography. Retinoids were dissolved in dimethylsulfoxide for use in the transcriptional activation assays. Three to four replicates were used for each sample.

After 40 hours, the cells were washed with PBS, lysed with a Triton X-100-based buffer and assayed for LUC and β-Gal activities using a luminometer or spectrophotometer, respectively. For each replicate, the normalized response (NR) was calculated as:

$$\text{LUC response}/\beta\text{-Gal rate}$$

where $\beta\text{-Gal rate} = \beta\text{-Gal} \cdot 1\text{x}10^{-5}/\beta\text{-Gal incubation time}$.

The mean and standard error of the mean (SEM) of the NR were calculated. Data was plotted as the response of the compound compared to the reference compounds over the range of the dose-response curve. For the agonist

compounds of the present invention, the effective concentration that produced 50% of the maximum response ($EC_{50}$) was quantified.

The potency (nM) of selected retinoid compounds of the present invention are in Table 1 below.

Table 1

Potency (nM) of selected retinoid compounds of the present invention on RARα,β,γ and RXRα,β,γ, in comparison to the known RAR-active retinoid compound all-*trans* retinoic acid (**ATRA**) and RXR-active retinoid compound 9-*cis* retinoic acid (**9-*cis***).

| | RARα | RARβ | RARγ | RXRα | RXRβ | RXRγ |
|---|---|---|---|---|---|---|
| **Cmpd. No.** | **Pot nM** | **Pot nM** | **Pot nM** | **Pot nM** | **Pot nM** | **Pot nM** |
| **101b** | 32 | 8 | 7 | 35 | 55 | 41 |
| **104b** | na | 61 | 83 | 18 | 20 | 11 |
| **106b** | 84 | 20 | 14 | na | 52 | 127 |
| **109b** | 28 | 3 | 1 | 2022 | 64 | 2267 |
| **119b** | 20 | 1 | 1 | 1659 | 1145 | 742 |
| **124b** | 101 | 33 | 94 | 38 | 37 | 26 |
| **135b** | 1166 | 53 | 47 | 2783 | 1979 | 2531 |
| **138b** | na | na | na | 422 | 204 | 301 |
| **141b** | 863 | 32 | 12 | na | na | 2930 |
| **143b** | na | 466 | 1877 | 393 | 472 | 247 |
| **144b** | 14 | 3 | 2 | 36 | 45 | 40 |
| **147b** | 35 | 4 | 1 | na | 579 | 1470 |
| **152b** | 651 | 8 | 32 | na | na | na |
| **ATRA** | 436 | 78 | 19 | 1015 | 1211 | 961 |
| **9-*cis*** | 220 | 29 | 50 | 195 | 128 | 124 |

na = not active (potency of >10,000 and/or efficacy of ≤ 20%)

As can been seen in Table 1, Compounds **101b, 109b, 119b** and **144b** are extremely potent RAR active, with all compounds displaying activity at less than 10 nM on RARβ and RARγ. Further, Compounds **109b** and **119b** are RAR selective. In fact, these Compounds are 2 to 10 times more potent than the known RAR active compound ATRA on the RARs. In addition, Compound **104b** is a potent and selective RXR compound. Likewise, pan-agonist Compounds **101b**, **124b** and **144b** display superior potency profiles to that of the known RXR active pan-agonist compound 9-*cis* retinoic acid.

## <u>EXAMPLE 82</u>

In addition to the cotransfection data of Example 81, the binding of selected compounds of the present invention to the RAR and RXR receptors was also investigated according to the methodology described in M.F., Boehm, et al., "Synthesis and Structure-Activity Relationships of Novel Retinoid X Receptor Selective Retinoids", 37 *J. Med. Chem.*, 2930 (1994); M.F. Boehm, et al., "Synthesis of High Specific Activity [$^3$H]-9-*cis* Retinoic Acid and Its Application for Identifying Retinoids with Unusual Binding Properties", 37 *J. Med. Chem.*, 408 (1994), and E.A. Allegretto, et al., "Characterization and Comparison of Hormone-Binding and Transactivation Properties of Retinoic Acid and Retinoid X Receptors Expressed in Mammalian Cells and Yeast", 268 *J. Biol. Chem.*, 22625 (1993), the disclosures of which are herein incorporated by reference.

Non-specific binding was defined as that binding remaining in the presence of 500 nM of the appropriate unlabelled compound. At the end of the incubation period, bound from free ligand were separated. The amount of bound tritiated retinoids was determined by liquid scintillation counting of an aliquot (700 mL) of the supernatant fluid or the hydroxylapatite pellet.

After correcting for non-specific binding, $IC_{50}$ values were determined. The $IC_{50}$ value is defined as the concentration of competing ligand needed to reduce specific binding by 50%. The $IC_{50}$ value was determined graphically from a log-logit plot of the data. The $K_i$ values were determined by application of the Cheng-Prussof equation to the $IC_{50}$ values, the labeled ligand concentration and the $K_d$ of the labeled ligand.

The binding activity (Kd in nM) results of selected retinoid compounds of present invention, and the reference compounds ATRA, and 9-*cis* RA, is shown in Table 2 below.

Table 2

Binding (Kd in nM) of selected retinoid compounds of the present invention on RARα,β,γ and RXRα,β,γ proteins in comparison to the known RAR-active retinoid compound all-*trans* retinoic acid (**ATRA**) and RXR-active retinoid compound 9-*cis* retinoic acid (**9-*cis***).

| | **RARα** | **RARβ** | **RARγ** | **RXRα** | **RXRβ** | **RXRγ** |
|---|---|---|---|---|---|---|
| **Cmpd. No.** | **Binding Kd (nM)** | **Binding Kd (nM)** | **Binding Kd (nM)** | **Binding Kd (nM)** | **Binding Kd (nM)** | **Binding Kd (nM)** |
| **101b** | 225 | 299 | 541 | 34 | 30 | 48 |
| **109b** | 33 | 68 | 279 | 340 | 332 | 382 |
| **119b** | 24 | 50 | 87 | 246 | 372 | 398 |
| **138b** | >1000 | >1000 | >1000 | 136 | 276 | 508 |
| **144b** | 145 | 313 | 494 | 59 | 146 | 85 |
| **147b** | 6 | 4 | 8 | 433 | 548 | 724 |
| **ATRA** | 15 | 17 | 17 | 53 | 306 | 306 |
| **9-*cis*** | 93 | 97 | 148 | 8 | 15 | 14 |

As can be seen in Table 2, the compounds of the present invention show comparable binding to the known RAR active compound ATRA, and the known RXR active compound 9-*cis*.

**<u>EXAMPLE 83</u>**

Yet another recognized measure of the retinoid activity of the compounds of the present invention is the ornithine decarboxylase assay, as originally described by Verma and Boutwell, 37 <u>Cancer Research</u>, 2196 (1977), the disclosure of which is herein incorporated by reference. In Verma & Boutwell original work using retinoic acid, it was established that ornithine decarboxylase (ODC) activity increased in relation to polyamine biosynthesis. In turn, it had previously been established that increases in polyamine biosynthesis is correlated with cellular proliferation. Thus, if ODC activity could be inhibited, cell hyperproliferation could be modulated. Although all causes of increased OCD activity are yet unknown, it is known that 12-0-tetradecanoylphorbor-13-acetate (TPA) induces ODC activity. Importantly, retinoic acid inhibits this induction of ODC by TPA.

An ODC assay essentially following the procedures set out in 35 <u>Cancer Research</u>, 1662 (1975), the disclosure of which is herein incorporated by reference, was used to demonstrate the inhibition of TPA induction of ODC by the compounds of the present invention. The results of this assay on exemplary compounds, and the reference compounds ATRA and (E)-4-[2-(5,6,7,8-tetrahydro-5,5,8,8-tetramethyl-2-napthalenyl)-1-propenyl]benzoic acid (TTNPB), known RAR active compounds, are shown below in Table 3. All values are expressed as the concentration of the indicated compounds in nM required to inhibit the TPA induction of ODC by 80 percent, i.e., the $IC_{80}$ in nM.

Table 3

Inhibitory concentration required to inhibit 80% of the maximally observed TPA induction of ODC (**ODC $IC_{80}$**) in nM for selected Compounds of the present invention and reference compounds ATRA and TTNBP.

| **Compound** | **ODC $IC_{80}$ (nM)** |
|---|---|
| **101a** | 91.0 |

Table 3 (continued)

| Inhibitory concentration required to inhibit 80% of the maximally observed TPA induction of ODC (**ODC $IC_{80}$**) in nM for selected Compounds of the present invention and reference compounds ATRA and TTNBP. | |
|---|---|
| **Compound** | **ODC $IC_{80}$ (nM)** |
| **101b** | 0.78 |
| **109b** | 0.08 |
| **144b** | 0.60 |
| **ATRA** | 1.40 |
| **TTNPB** | 0.09 |

Compound **101b**, which is the ester of Compound **101a**, has been included to show that such ester analogs exhibit retinoid activity. While not being bound to a theory of operation, it is believed that such esters may operate as pro-drugs *in vivo*, possibly due to the cleavage of the ester to the active acid form of the compounds of the present invention.

## EXAMPLE 84

The *in vitro* affect of selected compounds of the present invention on the recognized cancer cell lines, RPMI 8226, ME 180 and AML-193, obtained from the American Type Culture Collection (ATCC, Rockville, MD), was investigated.

RPMI 8226 is a human hematopoietic cell line obtained from the peripheral blood of a patient with multiple myeloma, and as such is a recognized model for multiple myelomas and related malignancies. Y. Matsuoka, G.E. Moore, Y. Yagi and D. Pressman, "Production of free light chains of immunoglobulin by a hematopoietic cell line derived from a patient with multiple myeloma", 125 *Proc. Soc. Exp. Biol. Med.,* 1246 (1967), the disclosure of which is herein incorporated by reference. The cells resemble the lymphoblastoid cells of other human lymphocyte cell lines and secretes λ-type light chains of immunoglobulin. RPMI 8226 cells were grown in RPMI medium (Gibco) supplemented with 10% fetal bovine serum, glutamine and antibiotics. The cells were maintained as suspension cultures grown at 37 °C in a humidified atmosphere of 5% $CO_2$ in air. The cells were diluted to a concentration of 1 x $10^5$/mL twice a week.

ME 180 is a human epidermoid carcinoma cell line derived from the cervix, and as such is a recognized model for squamous cell carcinomas and related malignancies. J.A. Sykes, J. Whitescarver, P. Jernstrom, J.F. Nolan and P. Byatt, "Some properties of a new epithelial cell line of human origin", 45 *MH-Adenoviridae J. Natl. Cancer Inst.*, 107 (1970), the disclosure of which is herein incorporated by reference. The tumor was a highly invasive squamous cell carcinoma with irregular cell clusters and no significant keratinization. ME 180 cells were grown and maintained in McCoy's 5a medium (Gibco) supplemented with 10% fetal bovine serum, .glutamine and antibiotics. The cells were maintained as monolayer cultures grown at 37 °C in a humidified atmosphere of 5% $CO_2$ in air.

The AML-193 cell line was established from the blast cells of a patient with leukemia and was classified as M5 Acute Monocytic Leukemia, and as such is a recognized model for leukemias and related malignancies. G. Rovera, et al., 139 *J. Immunol.*, 3348 (1987), the disclosure of which is herein incorporated by reference. Over 75% of these cells are positive by immunofluorescence for the myelomonocytic antigen CS15. The cells were grown in Iscove's modified Dulbeccos's medium with 5 μg/mL transferring, 5 μg/mL insulin and 2 ng/mL rh GM-CSF. The cells were maintained as suspension cultures grown at 37 °C in a humidified atmosphere of 5% $CO_2$ in air. The cells were diluted to a concentration of 1 x $10^5$/mL twice a week.

Incorporation of $^3$H-Thymidine

Measurement of the level of radiolabeled thymidine incorporated into the above-identified cell lines provides a direct measurement of the antiproliferative properties of the compounds of the present invention. The method used for determination of the incorporation of radiolabeled thymidine was adapted from the procedure described by S. Shrivastav et al., "An in vitro assay procedure to test chemotherapeutic drugs on cells from human solid tumors", 40 *Cancer Res.*, 4438 (1980), the disclosure of which is herein incorporated by reference. RPMI 8226 or AML-193 cells were plated in a 96 well round bottom microtiter plate (Costar) at a density of 1,000 cells/well. To appropriate wells, retinoid test compounds were added at the final concentrations indicated for a final volume of 150 μL/well. The plates were incubated for 96 hours at 37 °C in a humidified atmosphere of 5% $CO_2$ in air. Subsequently, 1 μCi of [5'-$^3$H]-thymidine

(Amersham, U.K, 43 Ci/mmol specific activity) in 25 μL culture medium was added to each well and the cells were incubated for an additional six hours. The cultures were further processed as described below.

ME 180 cells, harvested by trypsinization were plated in a 96 well flat bottom microtiter plate (Costar) at a density of 2,000 cells/well. The cultures were treated as described above for RPMI 8226 with the following exceptions. After incubation, the supernatant was carefully removed, and the cells were washed with a 0.5 mM solution of thymidine in phosphate buffered saline. ME 180 cells were briefly treated with 50 μL of 2.5% trypsin to dislodge the cells from the plate. Both cell lines were then processed as follows: the cellular DNA was precipitated with 10% trichloroacetic acid onto glass fiber filter mats using a SKATRON multi-well cell harvester (Skatron Instruments, Sterling VA). Radioactivity incorporated into DNA, as a direct measurement of cell growth, was measured by liquid scintillation counting. The mean disintegrations per minute of incorporated thymidine from triplicate wells was determined. The $IC_{50}$ (nM concentration required to inhibit 50% of the maximally observed incorporation of thymidine) for Compounds **101b**, **104b**, **109b**, **119b** and **144b** of the present invention, and reference compounds ATRA and TTNBP are shown below in Tables 4, 5 and 6 for the cell lines RPMI 8226, ME 180 and AML-193 respectively.

Viability

Selected compounds of the present invention were also measured to determine their cytotoxicity on the above-identified cell lines. The procedure used was identical, with only slight modifications, to the assay described in T. Mosmann, "Rapid colorimetric assay for cellular growth and survival: application to proliferation and cytotoxicity assays", 65 *J. Immunol. Meth.*, 55 (1983), the disclosure of which is herein incorporated by reference. RPMI 8226 or AML-193 cells were plated in a 96 well round bottom microtiter plate (Costar) at a density of 1,000 cells/well. To appropriate wells, retinoid test compounds were added at the final concentrations indicated for a final volume of 150 μL/well. The plates were incubated for 96 hours at 37 °C in a humidified atmosphere of 5% $CO_2$ in air. Subsequently, 15 μL of a filter sterilized tetrazolium dye in phosphate buffered saline (Promega, Madison, WI) was added to each well and the cells were incubated for an additional four hours. Subsequent manipulations of the cultures were as described below.

ME 180 cells, harvested by trypsinization were plated in a 96 well flat bottom microtiter plate (Costar) at a density of 2,000 cells/well. The cultures were treated as described above for RPMI 8226.

After the four hours incubation, 100 μL of a solubilization/stop solution was added to each well (Promega, Madison, WI). The plates were allowed to stand overnight at 37 °C in the humidified atmosphere. The absorbance at 570 - 600 nm wavelength was recorded for each well using a Biomek ELISA plate reader (Beckman Instruments). The $IC_{50}$ (nM concentration required to inhibit 50% of the mitochondrial function, and ultimately, the viability of the cells) for Compounds **101b**, **104b**, **109b**, **119b** and **144b** of the present invention, and reference compounds ATRA and TTNBP are also shown below in Table 4, 5 and 6 for the cell lines RPMI 8226, ME 180 and AML-193 respectively.

Table 4

Inhibitory concentration required to inhibit 50% of the maximally observed radiolabeled thymidine (**TdR $IC_{50}$**) in nM, and inhibitory concentration required to inhibit 50% of the mitochondrial function (**MTS $IC_{50}$**) in nM, for selected Compounds of the present invention and reference compounds ATRA and TTNBP on the RPMI 8226 cell line.

| Compound | TdR $IC_{50}$ (nM) | MTS $IC_{50}$ (nM) |
|---|---|---|
| **101b** | 6 | 100 |
| **104b** | 30 | 90 |
| **109b** | 0.05 | 10 |
| **119b** | 0.5 | 2.6 |
| **144b** | 0.6 | 7.5 |
| **ATRA** | 102 | 756 |
| **TTNPB** | 0.2 | 10 |

Table 5

| Inhibitory concentration required to inhibit 50% of the maximally observed radiolabeled thymidine (**TdR $IC_{50}$**) in nM, and inhibitory concentration required to inhibit 50% of the mitochondrial function (**MTS $IC_{50}$**) in nM, for selected Compounds of the present invention and reference compounds ATRA and TTNBP on the ME 180 cell line. | | |
|---|---|---|
| **Compound** | **TdR $IC_{50}$ (nM)** | **MTS $IC_{50}$ (nM)** |
| **101b** | 8 | 75 |
| **104b** | 90 | 1000 |
| **109b** | 5 | 100 |
| **119b** | 0.6 | 2 |
| **144b** | 4 | 10 |
| **ATRA** | 253 | 890 |
| **TTNPB** | 0.4 | 187 |

Table 6

| Inhibitory concentration required to inhibit 50% of the maximally observed radiolabeled thymidine (**TdR $IC_{50}$**) in nM, and inhibitory concentration required to inhibit 50% of the mitochondrial function (**MTS $IC_{50}$**) in nM, for selected Compounds of the present invention and reference compounds ATRA and TTNBP on the AML-193 cell line. | | |
|---|---|---|
| **Compound** | **TdR $IC_{50}$ (nM)** | **MTS $IC_{50}$ (nM)** |
| **101b** | 37 | 1000 |
| **104b** | 90 | 1000 |
| **109b** | 500 | 1000 |
| **119b** | 0.6 | 1000 |
| **144b** | 0.6 | 1000 |
| **ATRA** | 197 | 1000 |
| **TTNPB** | 0.1 | 1000 |

**EXAMPLE 85**

The following examples provide illustrative pharmacological composition formulations: Hard gelatin capsules are prepared using the following ingredients:

| | **Quantity (mg/capsule)** |
|---|---|
| Compound **101b** | 140 |
| Starch, dried | 100 |
| Magnesium stearate | 10 |
| Total | 250 mg |

The above ingredients are mixed and filled into hard gelatin capsules in 250 mg quantities.

A tablet is prepared using the ingredients below:

| | Quantity (mg/tablet) |
|---|---|
| Compound **101b** | 140 |
| Cellulose, microcrystalline | 200 |
| Silicon dioxide, fumed | 10 |
| Stearic acid | 10 |
| Total | 360 mg |

The components are blended and compressed to form tablets each weighing 360 mg. Tablets, each containing 60 mg of active ingredient, are made as follows:

| | Quantity (mg/tablet) |
|---|---|
| Compound **101b** | 60 |
| Starch | 45 |
| Cellulose, microcrystalline | 35 |
| Polyvinylpyrrolidone (PVP) | |
| (as 10% solution in water) | 4 |
| Sodium carboxymethyl starch (SCMS) | 4.5 |
| Magnesium stearate | 0.5 |
| Talc | 1.0 |
| Total | 150 mg |

The active ingredient, starch, and cellulose are passed through a No. 45 mesh U.S. sieve and mixed thoroughly. The solution of PVP is mixed with the resultant powders, which are then passed through a No. 14 mesh U.S. sieve. The granules so produced are dried at 50°C and passed through a No. 18 mesh U.S. sieve. The SCMS, magnesium stearate, and talc, previously passed through a No. 60 mesh U.S. sieve, are then added to the granules which, after mixing, are compressed on a tablet machine to yield tablets each weighing 150 mg.

Suppositories, each containing 225 mg of active ingredient, may be made as follows:

| | |
|---|---|
| Compound **101b** | 225 mg |
| Saturated fatty acid glycerides | 2.000 mg |
| Total | 2,225 mg |

The active ingredient is passed through a No. 60 mesh U.S. sieve and suspended in the saturated fatty acid glycerides previously melted using the minimum heat necessary. The mixture is then poured into a suppository mold of normal 2g capacity and allowed to cool.

An intravenous formulation may be prepared as follows:

| | |
|---|---|
| Compound **101b** | 100 mg |

(continued)

| | |
|---|---|
| Isotonic saline | 1,000 ml |
| Glycerol | 100 ml |

The compound is dissolved in the glycerol and then the solution is slowly diluted with isotonic saline. The solution of the above ingredients is then administered intravenously at a rate of 1 ml per minute to a patient.

While in accordance with the patent statutes, description of the preferred embodiments and processing conditions have been provided, the scope of the invention is not to be limited thereto or thereby. Various modifications and alterations of the present invention will be apparent to those skilled in the art without departing from the scope and spirit of the present invention.

Consequently, for an understanding of the scope of the present invention, reference is made to the following claims.

## Claims

1. A tricyclic compound of the formula:

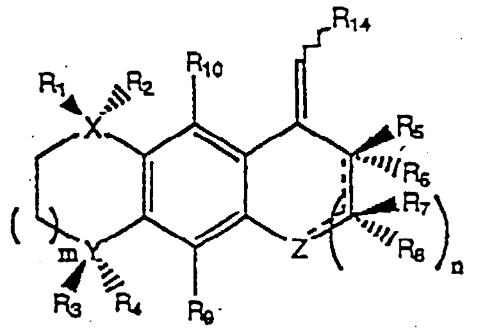

(I)

OR

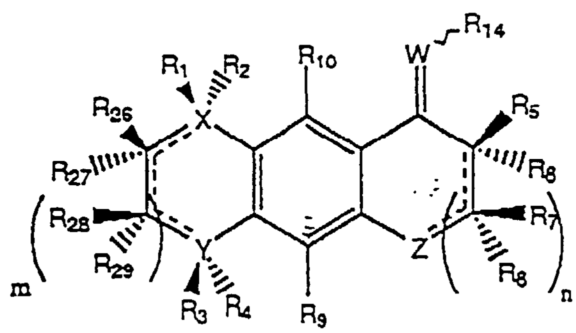

(IV)

wherein,

$R_1$ through $R_4$ each independently are hydrogen, a $C_1$ - $C_6$ alkyl, or a $C_7$ - $C_{15}$ arylalkyl:
$R_5$ through $R_8$ each independently are hydrogen, a $C_1$ - $C_6$ alkyl, or at least two of $R_5$ through $R_8$ taken together are a $C_3$ - $C_6$ cycloalkyl:
$R_9$ and $R_{10}$ each independently are hydrogen, a $C_1$-$C_6$ alklyl, F, Cl, Br, $NR_{11}R_{12}$, $NO_2$ or $OR_{13}$, where $R_{11}$ and $R_{12}$ each independently are hydrogen, a $C_1$ - $C_8$ alkyl, a $C_7$ - $C_{15}$ arylalkyl, a $C_1$ - $C_8$ acyl, provided that only one of $R_{11}$ or $R_{12}$ can be acyl, or $R_{11}$ and $R_{12}$ taken together are a $C_3$ - $C_6$ cycloalkyl, and where $R_{13}$ is hydrogen or a $C_1$ - $C_8$ alkyl or a $C_7$ - $C_{15}$ arylalkyl;

$R_{14}$ represents:

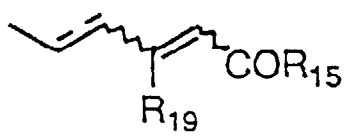

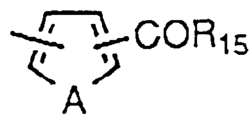

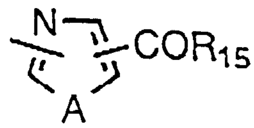

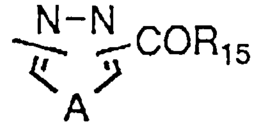

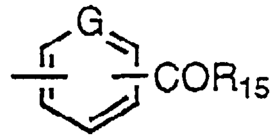

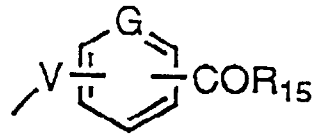

where $R_{15}$ is $OR_{16}$ or $NR_{17}R_{18}$, with $R_{16}$ being hydrogen, a $C_1$ - $C_6$ alkyl or a $C_7$ - $C_{15}$ arylalkyl, and with $R_{17}$ and $R_{18}$ each independently being hydrogen, $C_1$ - $C_6$ alkyl, a $C_7$ - $C_{15}$ arylalkyl, aryl, ortho-, meta-, or para-substituted hydroxyaryl, or taken together are a $C_3$ - $C_6$ cycloalkyl, provided that $R_{18}$ must be hydrogen when $R_{17}$ is aryl or hydroxyaryl, $R_{19}$ is a $C_1$ - $C_5$ alkyl, and A is O, S or $NR_{20}$, where $R_{20}$ is a hydrogen, $C_1$ - $C_6$ alkyl or a $C_7$ - $C_{15}$ arylalkyl;

$R_{26}$ through $R_{29}$ each independently are hydrogen or a $C_1$ - $C_6$ alkyl, or taken together then one each of $R_{26}$ and $R_{27}$ or $R_{28}$ and $R_{29}$ respectively, form a carbonyl group;

X and Y each independently represent C, O, S, N, SO or $SO_2$, provided, however, that when X or Y are O, S, SO or $SO_2$, then either $R_1$ and $R_2$ or $R_3$ and $R_4$ respectively do not exist, and further provided, that when X or Y is N, then one each of $R_1$ and $R_2$ or $R_3$ and $R_4$ respectively, do not exist;

Z is O, S, $CR_{22}R_{23}$ or $NR_{24}$, where $R_{22}$ through $R_{24}$ each independently are hydrogen or a $C_1$ - $C_6$ alkyl or $R_{22}$ and $R_{23}$ taken together are a $C_3$ - $C_6$ cycloalkyl or $R_{24}$ is carboxylic acid tert-butyl ester;

W is N or $CR_{25}$, where $R_{25}$ is hydrogen or a $C_1$ - $C_6$ alkyl;

V is C or N, provided, however, that when V is N, then no double bond exists adjacent to V;

G is C or N, provided G cannot be C when W is C;

m is 0 or 1 carbon atoms;

n is 0, 1 or 2 carbon atoms;

the dashed lines in the structures represent optional double bonds, provided, however that the double bonds cannot be contiguous, and further provided that when such optional double bonds exist then one each of $R_5$ and $R_6$ or $R_7$ and $R_8$ respectively do not exist; and

the wavy lines represent olefin bonds that are either in the *cis* (Z) or *trans* (E) configuration.

**2.** A compound according to claim 1, selected from the group consisting of ethyl (2E,4E)-3-methyl-6-[(Z)-3,4,5,6,7,8-hexahydro-5,5,8,8-tetramethyl-2H-anthracen-1-ylidene]hexa-2,4-dienoate; (2E,4E)-3-methyl-6-[(Z)-3,4,5,6,7,8-hexahydro-5,5,8,8-tetramethyl-2H-anthracen-1-ylidene]hexa-2,4-dienoic acid; ethyl (2E,4E)-3-methyl-6-[(Z)-2,3-5,6,7,8-hexahydro-5,5,8,8-tetramethylcyclopenta[b]napthalen-1-ylidene]hexa-2,4-dienoate; (2E,4E)-3-methyl-6-[(Z)-2,3-5,6,7,8-hexahydro-5,5,8,8,-tetramethylcyclopenta[b]naphthalen-1-ylidene]hexa-2,4-dienoic acid; ethyl (2E,4E)-3-methyl-6-[(Z)-1,2,3,6,7,8-hexahydro-1,1,3,3-tetramethylcycopenta[b]naphthalen-5-ylidene]hexa-2,4-dienoate; (2E,4E)-3-methyl-6-[(Z)-1,2,3,6,7,8-hexahydro-1,1,3,3-tetramethylcyclopenta[b]naphthalen-5-ylidene]hexa-2,4-dienoic acid; (2E,4E)-3-methyl-6-(2,3,6,7,8,9-hexahydro-2,2,6,6,9,9-hexamethylbenzo[b]-chromen-4-ylidene)hexa-2,4-dienoic acid; ethyl (2E,4E)-3-methyl-6-[(Z)-3,4,5,6,7,8-hexahydro-10-nitro-5,5,8,8-tetramethyl-2H-anthracen-1-ylidene]hexa-2,4-dienoate; (2E,4E)-3-methyl-6-[(Z)-3,4,5,6,7,8-hexahydro-10-nitro-5,5,8,8-tetramethyl-2H-anthracen-1-ylidene]hexa-2,4-dienoic acid; ethyl (2E,4E)-3-methyl-6-[(Z)-3,4,5,6,7,8-hexahydro-4,4,5,5,8,8-hexamethyl-2H-anthracen-1-ylidene]hexa-2,4-dienoate; (2E,4E)-3-methyl-6-[(Z)-3,4,5,6,7,8-hexahydro-4,4,5,5,8,8-hexamethyl-2H-anthracen-1-ylidene]hexa-2,4-dienoic acid; (2E,4E)-3-methyl-6-[(Z)-2,3,5,6,7,8-hexahydro-3,5,5,8,8-pentamethyl-cyclopenta[b]naphthalen-1-ylidene]hexa-2,4,-dienoic acid; (2E,4E)-3-methyl-6-[(Z)-3,5,6,7-tetrahydro-5,5,7,7-tetramethyl-2H-5-indacen-1-ylidene]hexa-2,4-dienoic acid; ethyl (2E,4E)-3-methyl-6-[(E)-3,4,5,6,7,8-hexahydro-5,5,8,8-tetramethyl-2H-anthracen-1-ylidene]hexa-2,4-dienoate; (2E,4E)-3-methyl-6-[(E)-3,4,5,6,7,8-hexahydro-5,5,8,8-tetramethyl-2H-anthracen-1-ylidene]hexa-2,4-dienoic acid; ethyl (2E,4E)-3-methyl-6-[(E)-2,3,5,6,7,8-hexahydro-5,5,8,8-tetramethylcyclopenta[b]naphthalen-1-ylidene]hexa-2,4-dienoate; (2E,4E)-3-methyl-6-[(E)-2,3,5,6,7,8-hexahydro-5,5,8,8-tetramethylcyclopenta[b]naphthalen-1-ylidene]hexa-2,4-dienoic acid; ethyl (2E,4E)-3-methyl-6-[(E)-1,2,3,6,7,8-hexahydro-1,1,3,3-tetramethylcyclopenta[b]naphthalen-5-ylidene]hexa-2,4-dienoate; (2E,4E)-3-methyl-6-[(E)-1,2,3,6,7,8-hexahydro-1,1,3,3-tetramethylcyclopenta[b]naphthalen-5-ylidene]hexa-2,4-dienoic acid; ethyl (2Z,4E)-3-methyl-6-[(E)-1,2,3,6,7,8-hexahydro-1,1,3,3-tetramethylcyclopenta[b]naphthalen-5-ylidene]hexa-2,4- dienoate; (2Z,4E)-3-methyl-6-[(E)-1,2,3,6,7,8-hexahydro-1,1,3,3-tetramethylcyclopenta[b]naphthalen-5-ylidene]hexa-2,4-dienoic acid; (2E,4E)-3-methyl-6-[(E)-3,4,5,6,7,8-hexahydro-4,4,5,5,8,8-hexamethyl-2H-anthracen-1-ylidene]hexa-2,4-dienoic acid; ethyl (2E,4E,6E)-3-methyl-6-[1,2,3,4,7,8,9,10-octahydro-1,1,4,4-tetramethyl-6H-naphthocycloheptene-10-yl]hexa-2,4,6-trienoate; (2E,4E,6E)-3-methyl-6-[1,2,3,4,7,8,9,10-octahydro-1,1,4,4-tetramethyl-6H-naphthocycloheptene-10-yl]hexa-2,4,6-trienoic acid; ethyl (2Z,4E,6E)-3-methyl-6-[1,2,3,4,7,8,9,10-octahydro-1,1,4,4-tetramethyl-6H-naphthocycloheptene-10-yl]hexa-2,4,6-trienoate; (2Z, 4E, 6E)-3-methyl-6-[1,2,3,4,7,8,9,10-octahydro-1,1,4,4-tetramethyl-6H-naphthocycloheptene-10-yl]hexa-2,4,6-trienoic acid; (2E,4E)-3-methyl-6-[(E)-3,5,6,7-tetrahydro-5,5,7,7-tetramethyl-2H-5-indacen-1-ylidene]hexa-2,4-dienoic acid; ethyl (2E,4E)-3-methyl-6-[(E)-3,4,5,6,7,8-hexahydro-10-nitro-5,5,8,8-tetramethyl-2H-anthracen-1-ylidene]hexa-2,4-dienoate; (2E,4E)-3-methyl-6-[(E)-3,4,5,6,7,8-hexahydro-10-nitro-5,5,8,8-tetramethyl-2H-anthracen-1-ylidene]hexa-2,4-dienoic acid; (2Z,4E)-3-methyl-6-[(E)-3,4,5,6,7,8-hexahydro-10-nitro-5,5,8,8-tetramethyl-2H-anthracen-1-ylidene]hexa-2,4-dienoic acid; (2E,4E)-3-methyl-6-[(E) 2,3,6,7,8,9-hexanydro-6,6,9,9-tetramethylbenzo[g]chromen-4-ylidene]hexa-2,4-dienoic acid; (2Z,4E)-3-methyl-6-[(E)-2,3,6,7,8,9-hexahydro-6,6,9,9-tetramethylbenzo[g]chromen-4-ylidene]hexa-2,4-dienoic acid; (2E,4E)-3-methyl-6-[(E)-2,3,6,7,8,9-hexahydro-2,2 6,6,9,9-hexamethylbenzo[g]chromen-4-ylidene]hexa-2,4-dienoic acid; (2E, 4E)-3-methyl-6-[(Z)-5,6,7,8-tetrahydro-5,5,8,8-tetramethylnaphthyl(2,3-b-pyran-4-ylidene]hexa-2,4-dienoic acid; (2E,4E)-3-methyl-6-[(E)-3,4,5,6,7,8-hexahydro-2,2,5,5,8,8-hexamethyl-2H-anthracen-1-ylidene]hexa-2,4-dienoic acid; Ethyl-(2E,4E)-3-methyl-6-[(Z)-N-acetyl-3,4,5,6,7,8-hexahydro-10-amino-5,5,8,8-tetramethyl-2H-anthracen-1-ylidene]hexa-2,4-dienoate; (2E,4E)-3 methyl-6-[(Z)-N-acetyl-3,4,5,6,7,8-hexahydro-10-amino-5,5,8,8-tetramethyl-2H-anthracen-1-ylidene]hexa-2,4-dienoic acid; (2E,4E)-3-methyl-6-[(E)1-ethyl-6,6,9,9-tetramethyl-2,3,6,7,8,9-hexahydro-1H-benzo[g] quinolin-4-ylidene]hexa-2,4-dienoic acid; T-butyl-4-(5-carboxy-penta-2E-4E-dieneylidene)-6,6,9,9-tetramethyl-3,4,6,7,8,9-hexahydro-2H-benzo[g]quinolin-1-carboxylate; (2E,4E)-3-methyl-6-(6-ethyl-1,9,9-trimethyl-7-oxo-2,3,6,7,8,9-hexahydro-1H-pyrido[2,3-g]quinolin-4-ylidene)hexa-2,4-dienoic acid; E-4-[N'-(5,5,8,8-tetramethyl-3,4,5,6,7,8-hexahydro-2H-anthracen-1-ylidene)-hydrazino]benzoic acid; E-4-[N'-(6,6,9,9-tetramethyl-2,3,6,7,8,9-hexahydro-benzo[g]chromen-4-ylidene)-hydrazino]benzoic acid and (2E,4E)-3-methyl-6-(6-ethyl-1,9,9-trimethyl-2,3,6,9-tetrahydro-1H-pyrido[2,3-g]quinolin-4-ylidene)hexa-2,4-dienoic acid.

**3.** A compound according to claim 1, wherein the compound is a retinoid compound.

**4.** A pharmaceutical composition comprising a compound of claim 1 and a pharmaceutically acceptable carrier.

**5.** A pharmaceutical composition according to claim 4, wherein the composition is formulated for oral, topical, intravenous, suppository or parental administration.

**6.** A pharmaceutical composition according to claim 4, wherein the compound is administered to a patient as a dosage unit at from about 1μg/kg of body weight to about 500 mg/kg of body weight.

**7.** A pharmaceutical composition according to claim 4, wherein the compound is administered to a patient as a dosage unit at from about 10μg/kg of body weight to about 250 mg/kg of body weight.

**8.** A pharmaceutical composition according to claim 4, wherein the compound is administered to a patient as a dosage unit at from about 20μg/kg of body weight to about 100 mg/kg of body weight.

**9.** Use of a compound according to claim 3 for the manufacture of a medicament for affecting RAR and/or RXR activity by *in vivo* administration of the compound.

**10.** Use of a compound according to claim 3 for the manufacture of a medicament for modulating processes mediated by RAR and/or RXR receptors by administering to a patient an amount of the compound, said compound being effective to modulate one or more processes mediated by RAR and/or RXR receptors.

**11.** Use of a compound according to claim 3 for the manufacture of a medicament for the treatment of a patient requiring retinoid therapy by administering to the patient a pharmaceutically effective amount of the compound.

**12.** Use according to claim 11, wherein the compound is effective in treating skin-related diseases and conditions, cancerous and pre-cancerous conditions, diseases of the eye, cardiovascular diseases, inflammatory diseases, neurodegenerative diseases, diseases involving modulation of apoptosis, diseases of the immune system improper pituitary function, diseases involving human papilloma virus, wound healing or restoration of hair growth.

**13.** Use of a pharmaceutical composition according to claim 4 for the manufacture of a medicament for treating a patient requiring retinoid therapy by administering to the patient a pharmaceutically effective amount of the composition.

**14.** Use according to claim 13, wherein the composition is effective in treating skin-related diseases and conditions, cancerous and pre-cancerous conditions, diseases of the eye, cardiovascular diseases, inflammatory diseases, neurodegenerative diseases, diseases involving modulation of apoptosis, diseases of the immune system, improper pituitary function, diseases involving human papilloma virus, wound healing or restoration of hair growth.

**15.** A method for determining the presence of one or more RAR and/or RXR receptors in a sample comprising combining a compound according to claim 1 with the sample containing one or more unknown retinoid receptors, and determining whether said compound binds to a receptor in the sample.

**16.** A ligand-retinoid receptor complex formed by the binding of a compound according to claim 1 to a RAR and/or RXR receptor.

**17.** A method of purifying retinoid receptors comprising combining a compound according to claim 1 with a sample containing RAR and/or RXR receptors, allowing said compound to bind said receptors, and separating out the bound combination of said compound and said RAR and/or RXR receptors.

European Patent Office

# EUROPEAN SEARCH REPORT

Application Number
EP 99 10 5682

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,X, Y | KAGECHIKA H ET AL: "Retinobenzoic acids. 3. Structure-activity relationships of retinoidal azobenzene-4-carboxylic acids and stilbene-4-carboxylic acids" JOURNAL OF MEDICINAL CHEMISTRY, vol. 32, no. 5, May 1989, pages 1098-108, XP002075257 * the whole document, particularly compounds 35-37 * --- | 1-17 | C07C57/50 C07D311/92 C07C205/56 C07C69/618 C07C233/54 C07D471/04 C07C251/84 A61K31/19 A61K31/215 A61K31/35 G01N33/53 //C07D223/14, A61K31/55, C07D221/08, A61K31/435, C07D209/60, A61K31/40, C07D221/10, |
| D,X, Y | KAGECHIKA H ET AL: "Retinobenzoic acids. 4. Conformation of aromatic amides with retinoidal activity. Importance of trans-amide structure for the activity" JOURNAL OF MEDICINAL CHEMISTRY, vol. 32, no. 10, October 1989, pages 2292-6, XP002104613 * the whole document, particularly compounds 10-13 * --- | 1-17 | |
| Y | LOELIGER P ET AL: "Arotinoids, a new class of highly active retinoids" EUROPEAN JOURNAL OF MEDICINAL CHEMISTRY - CHIMICA THERAPEUTICA, vol. 15, no. 1, 1980, pages 9-15, XP002004638 * the whole document * --- | 1-17 | TECHNICAL FIELDS SEARCHED (Int.Cl.6) C07C C07D A61K G01N |
| D,Y | US 5 124 473 A (SHROOT B ET AL) 23 June 1992 * the whole document * --- | 1-17 | |
| Y | WO 94 15902 A (LIGAND PHARMACEUTICALS INC.) 21 July 1994 * the whole document * ----- | 1-17 | |

The present search report has been drawn up for all claims

| Place of search | Date of completion of the search | Examiner |
|---|---|---|
| THE HAGUE | 2 June 1999 | Allard, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 99 10 5682

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int.Cl.6) |
| | | | C07D265/34, A61K31/535 |
| | | | TECHNICAL FIELDS SEARCHED (Int.Cl.6) |
| | | | |

The present search report has been drawn up for all claims

| Place of search | Date of completion of the search | Examiner |
|---|---|---|
| THE HAGUE | 2 June 1999 | Allard, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 99 10 5682

This annex lists the patent family members relating to the patent documents cited in the above-mentioned European search report.
The members are as contained in the European Patent Office EDP file on
The European Patent Office is in no way liable for these particulars which are merely given for the purpose of information.

02-06-1999

| Patent document cited in search report | Publication date | Patent family member(s) | Publication date |
|---|---|---|---|
| US 5124473 A | 23-06-1992 | LU 86022 A | 04-02-1987 |
| | | AT 76864 T | 15-06-1992 |
| | | AU 596109 B | 26-04-1990 |
| | | AU 6049986 A | 29-01-1987 |
| | | CA 1269371 A | 22-05-1990 |
| | | CA 1271422 A | 10-07-1990 |
| | | CA 1297127 A | 10-03-1992 |
| | | DE 3685528 A | 09-07-1992 |
| | | DK 65591 A | 11-04-1991 |
| | | DK 352486 A | 26-01-1987 |
| | | EP 0210929 A | 04-02-1987 |
| | | FI 863036 A,B, | 26-01-1987 |
| | | GR 861950 A | 25-11-1986 |
| | | JP 2614606 B | 28-05-1997 |
| | | JP 62036337 A | 17-02-1987 |
| | | PT 83056 A,B | 01-08-1986 |
| | | US 5602104 A | 11-02-1997 |
| | | US 5686586 A | 11-11-1997 |
| | | US 5434180 A | 18-07-1995 |
| WO 9415902 A | 21-07-1994 | AU 691477 B | 21-05-1998 |
| | | AU 5586894 A | 15-08-1994 |
| | | BR 9307784 A | 14-11-1995 |
| | | EP 0678086 A | 25-10-1995 |
| | | JP 8505852 T | 25-06-1996 |
| | | AT 177733 T | 15-04-1999 |
| | | AU 675430 B | 06-02-1997 |
| | | AU 4118893 A | 18-11-1993 |
| | | AU 5664294 A | 28-02-1995 |
| | | AU 6225894 A | 15-08-1994 |
| | | BR 9306284 A | 13-01-1998 |
| | | CA 2133587 A | 28-10-1993 |
| | | CA 2153235 A | 21-07-1994 |
| | | CA 2153236 A | 09-02-1995 |
| | | DE 69324043 D | 22-04-1999 |
| | | EP 0637297 A | 08-02-1995 |
| | | EP 0678087 A | 25-10-1995 |
| | | JP 8511027 T | 19-11-1996 |
| | | JP 8505359 T | 11-06-1996 |
| | | MX 9306627 A | 29-07-1994 |
| | | MX 9306629 A | 29-07-1994 |
| | | NO 943943 A | 21-12-1994 |
| | | WO 9321146 A | 28-10-1993 |
| | | WO 9415901 A | 21-07-1994 |
| | | WO 9504036 A | 09-02-1995 |
| | | US 5780676 A | 14-07-1998 |

EPO FORM P0459

For more details about this annex : see Official Journal of the European Patent Office, No. 12/82